# FASCICULE DE BREVET EUROPEEN

(11) **EP 1 135 417 B1**
(45) Date de publication et mention de la délivrance du brevet: **25.06.2003**
(21) Numéro de dépôt: 99957354.6
(22) Date de dépôt: 02.12.1999
(51) Int. Cl.: C08B 37/00, C08B 3/08, C08B 31/04, C08B 37/16, A61K 47/48, G01N 30/48

(54) **ESTERS CHIRAUX DE POLYSACCHARIDES, L'UN DE LEURS PROCEDES DE PREPARATION ET LEURS APPLICATIONS DANS L'OBTENTION D'ACIDES OPTIQUEMENT ENRICHIS OU LA CHROMATOGRAPHIE CHIRALE**
CHIRALE ESTER VON POLYSACCHARIDEN, EIN VERFAHREN ZU DEREN HERSTELLUNG UND VERWENDUNGEN ZUR HERSTELLUNG VON OPTISCH ANGEREICHERTEN SÄUREN ODER IN DER CHIRALEN CHROMATOGRAPHIE
POLYSACCHARIDE CHIRAL ESTERS, ONE OF THE METHODS FOR PREPARING THEM AND THEIR USES FOR OBTAINING OPTICALLY ENRICHED ACIDS OR CHIRAL CHROMATOGRAPHY

(30) Priorité: 03.12.1998 FR 9815452
(43) Date de publication de la demande: 26.09.2001
(73) Titulaire: Universite De Droit, D'Economie et des Sciences D'Aix-Marseille, 13620 Aix en Provence Cedex 1 (FR)
(72) Inventeur: ROUSSEL, Christian, F-13400 Aubagne (FR); BONNET, Brice, F-13100 Aix en Provence (FR); DE RIGGI, Innocenzo, F-13001 Marseille (FR); SUTEU, Cristina, F-67400 Illkirch (FR)
(74) Mandataire: Touati, Catherine
(86) Numéro de dépôt international: FR9902988
(87) Numéro de publication internationale: WO00032638

(56) Documents cités:
- EP-A- 0 436 722
- FR-A- 1 604 123
- FR-A- 2 714 671
- DATABASE WPI Week 7722 Derwent Publications Ltd., London, GB; AN 77-39182Y XP002112494 "mENTHOL RICH IN l:MENTHOL - PREPD. BY REACTING FATTY ACID AND RACEMIC MENTHOL WITH AN ESTERASE AND HYDROLYZING THE RESULTING MENTHYL ESTER." & JP 52 051095 A (TAKASAGO PERFUMERY CO LTD), 23 avril 1977 (1977-04-23)

## Description

Le domaine de la présente invention est celui de la séparation chirale et plus particulièrement des composés utiles à cette fin. Ces composés, possédant des propriétés de résolution chirale, constituent les phases stationnaires chirales (PSC) utilisées dans les techniques chromatographiques de séparation d'énantiomères, par exemple la chromatographie liquide haute performance. Les performances des PSC en reconnaissance et en rétention chirale sélective sont au coeur de ces techniques de séparation.

La présente invention concerne de nouveaux esters de composés polyhydroxylés, de préférence de polysaccharides, susceptibles d'être utilisés comme phases stationnaires chirales (PSC), mais également comme moyens d'enrichissement chiral et comme systèmes médicamenteux à libération prolongée et contrôlée de principes actifs chiraux enrichis.

L'invention a également pour objet la préparation de ces nouveaux esters de polysaccharides chiraux.

La présente invention vise, par ailleurs, *per se* :
- de nouveaux moyens d'enrichissement chiral,
- de nouveaux systèmes médicamenteux de libération prolongée et contrôlée de principes actifs,
- et de nouvelles phases stationnaires chirales ;
comprenant les esters polysaccharidiques chiraux selon l'invention.

Depuis quelques années, les composés optiquement actifs font l'objet d'un vif intérêt dans de nombreux domaines dont ceux relatifs aux produits pharmaceutiques, aux produits naturels, aux produits agrochimiques, aux cristaux liquides ferro-électriques, etc.. Cette tendance est allée de pair avec le développement des techniques de préparation, de purification et d'analyse des énantiomères.
S'agissant plus spécialement du domaine pharmaceutique, le fait que les systèmes vivants soient constitués de molécules et macromolécules chirales, telles que les protéines, les acides nucléiques et les polysaccharides, entraîne une sensibilité variable de ces organismes vivants vis-à-vis de médicaments chiraux selon que le principe actif est un énantiomère pur et/ou un racémique. C'est ainsi que sont apparus les médicaments chiraux comprenant un seul isomère optique à titre de principe(s) actif(s).
Ces médicaments chiraux sont souvent plus efficaces sous forme énantiomériquement pure que sous forme racémique. L'investigation détaillée de la pharmacocinétique, de la physiologie, de la toxicologie et de l'activité métabolique des médicaments chiraux, a donc eu pour corrolaire la mise au point de moyens de purification, d'analyse et de séparation chirale.
Cette supériorité, sur le plan des performances, de l'énantiomère pur par rapport au mélange racémique, qui est la forme la plus courante à l'issue d'une synthèse classique s'observe, non seulement dans le domaine pharmaceutique, mais également dans les autres domaines d'application des isomères optiques purs.

On connaît au moins deux méthodes d'obtention d'énantiomères sous forme pure, à savoir : la synthèse asymétrique et la résolution optique de racémiques, en particulier par chromatographie liquide haute performance (CLHP). Il se trouve que la CLHP est devenue une technique essentielle pour la recherche et le développement de médicaments chiraux. Il existe ainsi au moins 100 phases stationnaires chirales commercialement disponibles.
Généralement, on distingue deux types de PSC : celles préparées à partir de petites molécules chirales et celles constituées par des polymères dotés d'un pouvoir de résolution optique. Les PSC à base de petites molécules chirales sont préparées par liaison chimique des petites molécules chirales à un support, classiquement le gel de silice. L'aptitude à la reconnaissance chirale de ces PSC est relativement prédictible, compte tenu de la chiralité des petites molécules elles-mêmes.
S'agissant des supports PSC polymères, il en va différemment quant à la prédictibilité de leur aptitude à la reconnaissance chirale, car celle-ci ne peut être déduite de celle de l'unité monomère.

Parmi les phases stationnaires chirales PSC les plus courantes, on trouve celles constituées par des esters de polysaccharides et, plus spécialement, de cellulose ou d'amylose. Ce sont, par exemple, les triacétates, les tribenzoates, les trisphénylcarbamates ou les tricinnamates de cellulose, ou bien encore les trisphénylcarbamates ou trisphényléthylcarbamates d'amylose. La marque déposée désignant les esters de cellulose est CHIRALCEL®, tandis que celle désignant les esters d'amylose est CHIRALPAK® pour ce qui concerne les produits commercialisés par la Société DAICEL.
Ces composés polyhydroxylés que sont les polysaccharides cellulose et amylose comptent parmi les polymères naturels optiquement actifs les plus accessibles. Leur dérivatisation par estérification permet d'augmenter leur aptitude à la reconnaissance chirale.

A titre d'exemples d'esters de polysaccharides connus, employés en particulier comme PSC en chromatographie, on se réfèrera à ceux divulgués dans les articles suivants :
- *E. YASHIMA & Y. OKAMOTO, Bull. Chem. Soc. Jpn. ; 68, 3289-3307 (1995),*
- *E. YASHIMA et al., SYNLETT, 1998, 344-360 "Polysaccharide-based chiral LC columns",*
- *Y. OKAMOTO & Y. KAIDA, Journal of Chromatography A, 666 (1994), 403-419.*
Les esters de cellulose ou d'amylose divulgués dans ces articles ont ceci de caractéristique que l'acide ou le dérivé d'acide mis en oeuvre pour former les esters après association avec les hydroxyles, ne comporte pas de centre stéréogénique. Les seuls centres chiraux sont ceux du polysaccharide.
Les acides ou leurs dérivés, précurseurs des esters susvisés, peuvent être du type alkyle, tel que l'acide acétique et ses dérivés, ou du type aromatique, tel que l'acide benzoïque substitué ou non ou l'acide cinnamique substitué ou non.
Aucun de ces précurseurs-acides ne possède un carbone asymétrique situé en α de la fonction réactive COX.
Outre les applications en séparation chirale, il est à noter que les acétates de cellulose sont utilisés sous diverses formes dans l'industrie des emballages ou de la photographie.

La demande de brevet EP 0 316 270 décrit des esters de cellulose et d'acide aromatique ou d'acide aromatique et aliphatique, se présentant sous forme de particules sphériques en partie cristallines, avec un diamètre moyen de 1 à 200 µm et une surface spécifique comprise entre 10 et 300 m²/g. Ces esters cellulosiques sont destinés à être mis en oeuvre comme phase stationnaire dans un procédé de chromatographie plus spécialement adapté à la séparation de racémates. Les acides précurseurs des esters selon cette demande de brevet européen répondent à la formule :

―R―X―COOH

X pouvant être une liaison directe, un alkylène en C₁-C₄, un alcénylène en C₂-C₄, un alkylidène ou un alcylnilène et R un reste mono, bi ou tri cyclique, de préférence monocyclique et, en particulier, un aryle en C₆-C₁₄ ou un hétéroaryle.

Cette demande de brevet européen n'enseigne pas de nouveaux esters ou polysaccharides susceptibles d'avoir des propriétés intéressantes en matière de séparation et d'enrichissement chiraux.

La demande de brevet EP 436722 décrit, par des exemples détaillés, la synthèse de dérivés carbamates de polysaccharides et propose, sans citer aucun exemple, une méthode de préparation de dérivés esters de polysaccharides, via une technique, à l'époque, bien connue de l'homme de l'art. Lesdits esters de polyssaccharides décrits par les auteurs comme utiles pour la séparation d'isomères optiques, sont obtenus en faisant réagir un polysaccharide avec un composé de formule -COR où R représente notamment le sec-butyle ou le paramenthyle.

Dans cet état de la technique, l'un des objectifs essentiels de la présente invention est de fournir de nouveaux esters d'oligomères et/ou de polymères polyhydroxylés - et éventuellement polyamines - qui soient dotés de propriétés chirales améliorées.

Un autre objectif essentiel de la présente invention est de proposer de nouveaux esters polyhydroxylés, de préférence de polysaccharides, qui soient particulièrement appropriés comme moyen d'enrichissement chiral différent des techniques de séparation chromatographique et/ou comme support de chromatographie chirale pour la séparation d'énantiomères.

Un autre objectif essentiel de la présente invention est de fournir des esters de composés polyhydroxylés (oligomères et /ou polymères), performants en matière de séparation chirale et pouvant être obtenus de manière simple et économique.

Un autre objectif essentiel de l'invention est de fournir un procédé de préparation d'esters de composés polyhydroxylés, de préférence de polysaccharides, qui soient efficaces dans la séparation et/ou l'enrichissement chiral ; un tel procédé se devant d'être industriel donc peu onéreux à mettre en oeuvre.

Un autre objectif essentiel de la présente invention est de fournir un nouveau moyen d'enrichissement chiral pour la production d'énantiomères purs.

Un autre objectif essentiel de l'invention est de fournir un nouveau système médicamenteux à libération prolongée et contrôlée de principes actifs et, en particulier, des principes actifs se présentant sous forme d'isomères optiquement purs ; un tel système se devant d'être économique et simple à mettre en oeuvre.

Un autre objectif essentiel de la présente invention est de fournir de nouveaux supports ou phases stationnaires chirales, à faible coût de revient et performants, pour chromatographie et, en particulier, pour chromatographie liquide haute performance

S'étant fixés ces objectifs, parmi d'autres, les inventeurs ont eu le mérite de préparer et de caractériser de nouveaux esters de composés polyhydroxylés (monomères, oligomères, co-oligomères, polymères, copolymères), ces composés à multiples OH étant, de préférence, des polysaccharides. Ces nouveaux esters ont pour caractéristique d'être chiraux et d'être obtenus à partir d'un percurseur-acide comprenant au moins un centre stéréogénique (carbone asymétrique), situé de préférence en α de la fonction réactive, plus particulièrement carboxylique.

D'où il résulte que la présente invention concerne, tout d'abord, des esters de composés polyhydroxylés - éventuellement polyaminés - de préférence de polysaccharides, caractérisés en ce qu'ils sont chiraux et en ce qu'ils sont obtenus :ö
■ par réaction [en milieu basique] d'au moins un composé polyhydroxylé et d'au moins un précurseur-acide ou dérivé de formule (**I**) : 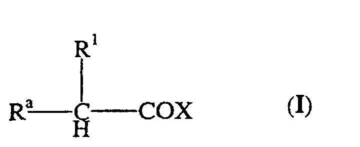
dans laquelle :
- X correspond à un hydroxyle ou à un halogène, de préférence le chlore,
- R^{a} représente un aryle, un aralkyle, un aralcényle, un alkylaryle, un alcénylaryle,
- et R¹ représente un alkyle linéaire ou ramifié et/ou cyclique en C₁-C₂₀, avantageusement en C₁-C₆ ; et en ce que
   - ils présentent au moins un centre stéréogénique introduit par le précurseur-acide ou dérivé (I), et
   - les motifs acyle fixés sur ledit composé polyhydroxylé, de préférence le polysaccharide n'étant pas tous de chiralité identique.

Les acides résultant de l'hydrolyse des dits esters ne sont en effet pas tous de même chiralité comme cela peut être observé par l'hydrolyse des esters à l'aide d'une base, de préférence une base hydroxylée tel que l'hydroxyde de lithium.

Les esters concernés selon l'invention sont ceux résultant de réactions entre des fonctions COX et OH.

Il est à noter que, dans le cas où les composés polyhydroxylés comprennent également des fonctions réactives de type aminé, les précurseurs-acides ou dérivés (**I**) peuvent réagir avec ces fonctions amines (―COX + ―NHR) pour former des liaisons amides.

Il en résulte que, dans tout le présent exposé, le terme "esters chiraux" désignera aussi bien les esters chiraux stricto sensu que les esters chiraux comprenant, en outre, des parties acyles des composés (**I**) liés au(x) composé(s) polyhydroxylé(s) par des liaisons amides.

Au sens de l'invention, les composés polyhydroxylés sont des produits chiraux, (par exemple des polymères) comprenant au moins deux motifs hydroxyles et éventuellement au moins deux motifs aminés. Ce terme "composé polyhydroxylé" englobe donc à la fois les composés ne comportant que des OH et les composés comprenant des OH et des fonctions amines.

Dans un mode de réalisation de l'invention, lesdits motifs acyle fixés sur ledit composé polyhydroxylé sont majoritairement de configuration R.

Plus particulièrement la proportion desdits motifs acyle de configuration R est comprise entre 50 et 70 %.

Dans un autre mode de réalisation, lesdits motifs acyles fixés sur ledit composé poly hydroxylé sont majoritairement de configuration S.

Plus particulièrement, la proportion desdits motifs acyles de configuration S est comprise entre 50 et 60 %.
La formule de l'ester de composé polyhydroxylé selon la présente invention est donc obtenue en remplaçant un atome d'hydrogène d'au moins un groupe hydroxyle ou aminé dudit composé polyhydroxylé par un reste 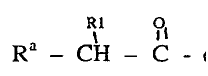 dont le centre stéréogénique peut être de chiralité différente de celui du réactif chlorure d'acide de formule (1) de départ.
Les esters selon l'invention sont obtenues par passage du précurseur-acide ou dérivé (I) sous une (des forme(s) réactive(s) intermédiaire(s) en équilibre avec l'espèce prochirale de type cétène présentent au moins un centre stéréogénique introduit par la partie acyle du précurseur-acide ou dérivé (I), dont la chiralité ne dépend pas de la chiralité du précurseur acide de départ. Les motifs acyles chiraux fixés, le sont selon une organisation spécifique qui dépend de la sensibilité des différents sites d'accrochage du composé polyhydroxydé au regard de l'espèce réactive-intermédiaire prochirale de type cétène dérivée du précurseur-acide (I). En d'autres termes, la chiralité de chaque motif acyle fixé sur les alcools primaires et secondaires du composé polyhydroxylé, de préférence du polysaccharide, ne dépend pas de la configuration initiale du précurseur-acide ou dérivé (I) de départ, mais est contrôlée essentiellement par le composé polyhydroxylé et par la nature de la (ou les) base(s) utilisée(s), et dépend également de la nature des espèce réactives intermédiaires dérivées de l'acide-précurseur ou dérivé (I), en équilibre avec l'espèce prochirale de type cétène.
Ces nouveaux esters présentent les avantages suivants :
- Ils ouvrent d'intéressantes perspectives en matière d'applications de type enrichissement chiral, de système médicamenteux de libération prolongée et contrôlée de principe actif lorsque celui-ci constitue le précurseur-acide ou dérivé (**I**) composant les motifs acyles ou comme phase stationnaire chirale de chromatographie pour la résolution de racémates.
- Les esters ainsi formés ont une structure tridimensionnelle relaxée, c'est à dire une structure dont les énergies de contraintes sont minimisées, en particulier du point de vue des interactions entre les différents centres stéréogéniques présents sur le polymère polyhydroxylé et les motifs acyles d'esters, voire d'amides, introduits.
   Cette relaxation permet d'améliorer les propriétés de ces esters en matière d'application de type enrichissement chiral, de système médicamenteux de libération prolongée et contrôlée de principe actif, lorsque celui-ci constitue le précurseur-acide ou dérivé (**I**) composant les motifs acyles, ou comme phase stationnaire chirale de chromatographie pour la résolution de racémates.
- Il n'est pas nécessaire d'utiliser un précurseur-acide ou dérivé énantiomériquement pur, onéreux,. En effet, un mélange racémique ou scalémique (c'est à dire un mélange des deux énantiomères dans des proportions quelconques), moins cher, suffit parfaitement.

S'agissant du précurseur―acide ou dérivé (**I**), on préfère, conformément à l'invention, ceux de formule (**I**) dans laquelle :
- R^{a} correspond à un substituant comprenant au moins un phényle et/ou au moins un naphtyle,
- et R¹ correspond à un méthyle, un éthyle ou un propyle.
Les précurseurs-acides ou dérivés (**I**) les plus réactifs sont des halogénures d'acide, les chlorures d'acide étant particulièrement adaptés. Ainsi, X correspond de préférence à Cl dans la formule (**I**) du précurseur-acide ou dérivé.

A titre d'exemples de précurseurs-acides ou dérivés (**I**), de préférence de chlorures d'acide, on peut faire référence à des exemples d'acides originels conduisant aux précurseurs (**I**). Ces acides originels répondent e.g. à la formule générale (**II**) : 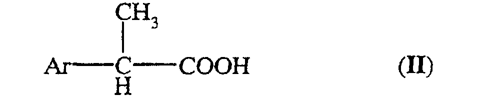 dans laquelle Ar représente un radical aromatique.
Peuvent être considérés répondant à cette formule (**II**) les composés suivants : acide α-phénylpropionique, acide α-(p-chlorophényle)propionique, acide 2-[3-benzoyl-phényl] propionique (kétoprofen), acide α-(3,5-diméthylphényl)propionique, acide α-(2-thiophényl)-propionique), acide α-(p-méthylphényl)proprionique, acide α-méthyl-4-[isobutyl]phénylacétique (ibuprofen), acide 6-méthoxy-α-méthyl-2-naphtalène-acétique (naproxen), un acide anti-inflammatoire non stéoridien.
L'ibuprofen, le kétoprofen et le naproxen sont des anti-inflammatoires non stéroïdiens bien connus. Ce sont des médicaments chiraux de nature carboxylique, parmi une série d'autres qui pourraient former des esters avec des composés polyhydroxylés, conformément à l'invention. Ces principes actifs chiraux (**II**) illustrent les possibilités d'application de l'invention dans l'industrie pharmaceutique chirale (purification d'énantiomères, enrichissement chiral, séparation d'isomères optiques purs).
Ces acides originels (**II**), médicamenteux ou non, à partir desquels peuvent être obtenus les précurseurs (**I**) sont disponibles commercialement mais peuvent être préparés par des voies de synthèse bien connues de l'homme de l'art. A titre d'exemple, on mentionnera une voie de synthèse classique qui fait intervenir trois étapes successives :
- carboxyéthylation d'un arylacétonitrile [RABJOHN, *Organic Synthesis*, Collective Vol. J. WILEY and Sons, 461-463, (1967)] :
- méthylation immédiatement suivie d'une carboxylation [M. BERCOT-VATTERONI et al., *Bull. Soc. Chim. Fr.,* 1820-1821, (1961)],
- hydrolyse du nitrile [B. S. FURNISS et al., *Wogel's Dexbetook of Practice Organic Chemistry,* 5 Ed., 1062-1063, (1993)].

Sans vouloir être lié par la théorie, il peut être précisé que l'un des intermédiaires réactionnels importants pour parvenir aux esters selon l'invention est une forme réactive dérivée des composés (**II**) et (**I**). En l'occurrence, il s'agit d'un cétène ArR₁C=C=O. Cette forme réactive intermédiaire prochirale de type cétène est transitoire.
Le cétène est, préférablement, obtenu par l'intermédiaire d'un halogénure d'acide (e. g. un chlorure), mais il n'est pas exclu de former ce cétène en agissant sur l'acide carboxylique de départ (acide originael **II**), par exemple non limitatif, à l'aide de réactifs tels que la dicyclohexylcarbodiimide, le TsCl, ou l'iodure de 1-méthyl-2-chloropyridinium (réactif de Mukaiyama).

Les fonctions réactives, susceptibles de réagir avec les précurseurs-acides ou dérivés (**I**) (de préférence les chlorures d'acide), sont des fonctions hydroxyles portées par des composés qui peuvent être des monomères et/ou des (co)oligomères et/ou des (co)polymères. Les mono-, oligo- et polysaccharides sont les composés polyhydroxylés préférés conformément à l'invention. Ces mono et/ou oligo et/ou polysaccharides sont hydrogénés ou non.

En pratique, les composés polyhydroxylés sont des polysaccharides sélectionnés parmi les celluloses et/ou leurs dérivés et/ou les amidons et leurs dérivés. Les unités monomères qui composent ces polysaccharides et qui sont plus spécialement sélectionnées, sont des unités "glucose" reliées par des liaisons β₁₋₄ dans le cas de la cellulose et des liaisons α₁₋₄ dans le cas de l'amylose et éventuellement α₁₋₆ dans le cas de l'amylopectine, pour ce qui concerne l'amidon.
Comme autres exemples de polysaccharides susceptibles de convenir pour former les esters selon l'invention, on peut citer les xylanes, les mannanes, les pullulanes, les curdlanes, les chitosanes, les dextranes, les inulines...
Comme déjà indiqué ci-dessus, les hydroxyles ne sont pas les seules fonctions réactives avec lesquelles les précurseurs-acides ou dérivés sont suceptibles de réagir. Ainsi, des fonctions aminés présentes sur les composés polyhydroxylés peuvent être à la base du greffage de motifs chiraux issus de (**I**) par formation de liaisons amides.
Il en est ainsi dans les polymères du type chitosanes.
Le fait que les polysaccharides soient préférés, n'exclut pas pour autant les esters formés à partir d'oligosaccharide de type cello-oligosaccharide ou malto-oligosaccharide. Il peut également s'agir d'oligosaccharides cycliques, tels que les α, les β et les γ cyclodextrines.

Dans le cas où le composé polyhydroxylé est un polysaccharide constitué d'unités monomères monosaccharidiques en C₆, les esters de l'invention se caractérisent par le fait que la chiralité résultante de chaque ester formé est différente pour chacune des trois fonctions alcool de chaque monomère, à savoir : la fonction alcool primaire du C₆ et les deux fonctions alcool secondaires, respectivement des C₂ et C₃.

Les esters selon l'invention peuvent être également définis par le degré de substitution (DS) des fonctions alcool des unités monomères qui constituent le composé polyhydroxylé. Ainsi, au sens de l'invention, le degré de substitution DS correspond au nombre d'équivalents de motifs acyles introduits sur le support (c'est à dire le polymère) par unité monomère.

On cite en particulier les esters dans lequel le degré de subtitution DS de chaque unité monomérique dudit composé polyhydroxylé est compris entre 0,5 et 3.

C'est ainsi que, dans le cas des polysaccharides, on peut avoir des esters dont le DS est voisin de 1 et dans lesquels la fonction alcool ayant réagi est principalement la fonction alcool primaire. Pour les esters de DS voisin de 2, ce sont principalement les fonctions alcool primaire et alcool secondaire en position en position 2 de l'unité monosaccharidique à 5 ou 6 carbones, qui réagissent avec le précurseur-acide ou dérivé (**I**). Enfin, dans les esters de DS voisin de 3, toutes les fonctions alcool du monomère saccharidique sont impliquées dans l'estérification.
Les DS, évalués par spectroscopie infrarouge à transformé de Fourier en phase solide, sont mesurés par microanalyse des éléments et par résonance magnétique du proton (200 MHz) lorsque la solubilité des esters obtenus est suffisante.

Comme cela résulte de la définition des esters selon l'invention donnée ci-dessus, ainsi que de la formule des précurseurs-acides ou dérivés (**I**) compris dans cette définition, la nature des motifs acyles desdits esters peut être différente d'une fonction alcool estérifiée à une autre sur le composé polyhydroxylé. De tels esters sont dénommés "esters mixtes" selon la nomenclature utilisée dans le présent exposé. Les esters mixtes sont ceux comprenant au moins un motif acyle provenant d'un précurseur-acide ou dérivé (**I**) asymétrique, les autres motifs acyles pouvant provenir de précurseurs-acides ou dérivés (**I**) asymétriques ou non asymétriques et différents. Ce dernier mode de réalisation de l'invention correspond au cas où les esters sont caractérisés en ce qu'ils sont obtenus en faisant réagir au moins un autre précurseur-acide ou dérivé (**I'**) différent du (ou des) précurseurs-acide(s) ou du (ou des) dérivé(s) (**I**), avec le ou les composé(s) polyhydroxylé(s) ;
cet autre réactif (**I'**) étant choisi parmi les composés chiraux utilisés dans la présente invention ou parmi des composés chiraux ou non dans la liste suivante non limitative : acides acétique, propionique, benzoïque, carbamique et dérivés.
Avantageusement, les esters mixtes comportant différents substituants acides asymétriques ou non peuvent être obtenus en introduisant, de façon séquentielle, les différents précurseurs-acides ou dérivés (**I**), de préférence les différents chlorures d'acide.
Les degrés de substitution DS de ces esters mixtes peuvent être contrôlés par les séquences d'introduction et par d'autres dispositions méthodologiques propres au procédé d'estérification. Les additions séquentielles des différents précurseurs peuvent être effectuées dans le même réacteur ou, et on préfère procéder de la sorte, par purification et caractérisation entre chaque addition.

Parmi les esters selon l'invention, on cite plus particulièrement les esters suivants comportant majoritairement desdits motifs acyles de configuration R, de préférence comportant entre 50 et 70 % de motifs de configuration R :
- l'α-phényl-propionate de cellulose
- l'α-chlorophényl-propionate d'amylose
- l'α-chlorophényl-propionate de cellulose
- l'α-chlorophényl-propionate d'amylose (vous l'avez cité 2 fois)
- l'α(3,5-diméthylphényl) propionate de cellulose
- l'α(paraméthyl-phényl) propionate de cellulose
- l'α-(2-thiophényl) propionate de cellulose
- un ester mixte de cellulose d'acide α phényl propionique et de kétoprofen
- le kétoprofénate de cellulose
- le kétoprofénate d'amylose
- l'ibuprofénate de cellulose
- le naproxénate de cellulose
- le naproxénate d'amylose
- le naproxénate de betacyclodextrine
- un ester de cellulose ou d'amylose et d'acide anti-inflammatoire non stéoridien
On cite aussi les esters comportant majoritairement desdits motifs acyles de configuration S, de préférence comportant entre 50 et 60 % de motifs de configuration S
- l'α-phényl propionate de cellulose
- l'α-chlorophényl-propionate d'amylose
- l'α-chlorophényl-propionate de cellulose
- l'α-chlorophényl-propionate d'amylose (vous l'avez cité 2 fois)
- l'α(3,5-diméthylphényl) propionate de cellulose
- l'α(paraméthyl-phényl) propionate de cellulose
- l'α-(2-thiophényl) propionate de cellulose
- un ester mixte de cellulose d'acide α phényl propionique et de kétoprofen
- le kétoprofénate de cellulose
- le kétoprofénate d'amylose
- l'ibuprofénate de cellulose
- le naproxénate de cellulose
- le naproxénate d'amylose
- le naproxénate de betacyclodextrine
- un ester de cellulose ou d'amylose et d'acide anti-inflammatoire non stéoridien

Selon un autre de ses aspects, la présente invention concerne un procédé de préparation d'esters de composés polyhydroxylés, de préférence de polysaccharides, notamment du type de ceux décrits ci-dessus. Ce procédé est caractérisé en ce qu'il consiste, essentiellement, à faire réagir au moins un composé polyhydroxylé avec au moins un acide ou dérivé de formule (**I**) en milieu basique.
Selon une disposition préférée de l'invention, on met en oeuvre au moins un chlorure d'acide à titre de réactif (**I**) et l'on récupère les esters formés en procédant à au moins une séquence précipitation/dissolution.
En pratique, ces esters sont obtenus selon l'invention par réaction, par exemple, d'un chlorure d'acide (obtenu par des réactions bien connues à partir de l'acide carboxylique correspondant cf. supra), sur un composé polyhydroxylé, de préférence un polysaccharide préalablement séché, en présence d'une base, avantageusement d'une base azotée tertiaire (par exemple la pyridine), seule ou en mélange avec une autre base ou un cosolvant (ex : pyridine, pyridine/triéthylamine, pyridine/toluène...).
La préparation des chlorures d'acide est décrite, par exemple, dans : H. Van. G. ELDEREN et al., *Chirality*, **6**, 11-16, (1994).
La réaction d'estérification peut être éventuellement catalysée à l'aide d'un catalyseur approprié, selon la nature du précurseur du type chlorure d'acide impliqué et selon le degré de substitution souhaité. Le catalyseur peut être, par exemple, la 4-diméthylamino-pyridine (DMAP).
Le milieu réactionnel est agité à une température comprise entre 25 et 120 °C, pendant une durée comprise entre 1 et 48 h. Les esters selon l'invention sont récupérés par précipitation dans un alcool, tel que le méthanol et sont ensuite purifiés par un jeu répété de précipitations dans l'alcool (méthanol) et dissolution dans un solvant organique, tel que le dichlorométhane.
Ce mode préféré de préparation des esters selon l'invention, conformément à un procédé relevant également de l'invention, s'applique, bien entendu, aux esters mixtes définis supra, en prévoyant l'étape d'addition séquentielle du précurseur-acide ou dérivé (**I**) (chlorure d'acide).
Cela correspond à une variante du procédé de l'invention selon laquelle on utilise, dans l'estérification, au moins un autre acide ou dérivé (**I'**) différent de l'(ou des) acide(s) ou du (ou des) dérivé(s) (**I**), ce réactif supplémentaire étant de préférence choisi parmi les composés utilisés dans la présente invention ou encore dans la liste suivante non exhaustive : acides acétique, propionique, benzoïque, carbamique et dérivés...

Selon une autre variante, les esters selon l'invention peuvent être obtenus sans transiter par les halogénures (de préférence chlorures) d'acide de formule (**I**), mais en faisant réagir directement un ou plusieurs réactifs appropriés avec le précurseur-acide (**I**) sous forme d'acide carboxylique.
A titre d'exemples de tels réactifs, on peut citer la dicyclohexylcarbodiimide, le TsCl, ou l'iodure de 1-méthyl-2-chloropyridinium (réactif de Mukaiyama).
Ces deux voies réactionnelles conduisent toutes deux à un intermédiaire réactionnel de type cétène : 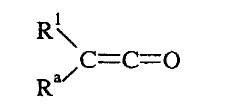 R₁, R^{a} étant tels que définis dans la formule (**I**).

La présente invention concerne, également, l'enrichissement chiral de racémates. Cette application des esters chiraux, de préférence polysaccharidiques, découle de leur caractéristique avantageuse selon laquelle la chiralité résultante de chaque motif acyle fixé sur les alcools primaire, secondaire du polysaccharide ne dépend pas de la configuration initiale de l'acide originel (**II**) mais est contrôlée essentiellement par le composé polyhydroxylé et par la nature de la (ou les) base(s) utilisée(s), et dépend également de la nature prochirale de l'espèce réactive intermédiaire de type cétène mise en jeu, A titre d'exemple démonstratif, l'analyse chromatographique des acides issus de l'hydrolyse des phases synthétisées à partir du naxopren S (+) et du naxopren racémique montre des enrichissements voisins.

La chiralité résultante de chaque ester formé a ceci de particulier qu'elle est différente pour chacune des trois fonctions alcool du monomère saccharidique, dans le cas où le polymère considéré est un polysaccharide constitué de monomères saccharidiques en C₆. Cela signifie que, à titre d'exemple illustratif et non limitatif, et de façon tout à fait surprenante et inattendue, les hydrolyses d'esters, du type naproxénate de cellulose formés dans la pyridine (avec ou sans DMAPcatalytique), font apparaître que les restes acides obtenus après hydrolyse sont majoritairement de configuration R pour les esters dont les motifs acyles sont fixés sur l'alcool primaire et l'alcool secondaire en positions 6 et 2, respectivement de l'unité glucose, tandis que les restes acides liés, avant hydrolyse, à l'alcool secondaire en position 3 de l'unité glucose sont très majoritairement de configuration S. Dans le cas où les esters sont des α-phénylpropionates de cellulose mono-, di- et trisubstitués, on remarque que l'alcool primaire oriente vers une configuration R du motif acyle fixé et que s'opère une compensation des restes issus de l'hydrolyse des esters propres aux deux fonctions alcool secondaires, pour aboutir, *in fine*, à un rapport final en faveur de la configuration R.
Ainsi, en jouant :
- sur les différences d'affinité de chacune des formes réactives issues du précurseur acide ou dérivé (**I**) vis à vis des divers sites (primaire ou secondaire) hydroxyles du composé polyhydroxylé (de préférence du polysaccharide)
- sur la nature de la (les) base(s) utilisée(s)
- et sur les différences de sensibilité des divers sites estérifiés vis à vis de l'hydrolyse, il est possible d'isoler l'un ou l'autre des deux énantiomères de façon optiquement pure, ou d'obtenir un mélange d'énantiomères enrichi en l'une des deux formes.

D'où il s'ensuit que la présente invention a également pour objet un procédé d'enrichissement chiral de racémates, caractérisé en ce qu'il consiste essentiellement :
□ **1**□- à utiliser des acides ou dérivés sous forme racémique ou énantiomériquement pure, comme produits de départ, pour préparer les esters tels que définis supra ou, les esters tels qu'obtenus par le procédé lui aussi présenté ci-avant,
□ **2**□―et à hydrolyser les esters ainsi formés, de préférence à l'aide d'une base et, plus préférentiellement encore, à l'aide d'une base hydroxylée notamment l'hydroxyde de lithium.

Ce procédé selon l'invention permet ainsi, de manière tout à fait avantageuse, d'atteindre, par exemple, un enrichissement global de 36%, correspondant à un rapport 68/32, après estérification de cellulose, dans la pyridine et à température ambiante, à l'aide de chlorure d'acide α-phénylpropionique racémique, puis hydrolyse de l'ester α-phénylpropionate de cellulose formé à l'aide d'une base forte, de préférence choisie parmi les hydroxydes de métaux alcalins, tels que, par exemple, l'hydroxyde de lithium.
En pratique, les hydrolyses sont, en effet, réalisées à l'aide d'hydroxyde de lithium monohydrate, de façon non racémisante. La composition énantiomérique de l'acide récupéré après hydrolyse est mesurée par CLHP chirale sur des phases stationnaires, de type de celles commercialisées par la Société DAICEL.

L'enrichissement est possible, du fait des différences d'affinité de chacune des formes réactives issues du précurseur acide ou dérivé (**I**) vis à vis de chaque type (primaire ou secondaire) de sites hydroxylés, du fait de la nature de la (les) base(s) utilisée(s) et du fait des différences de sensibilité vis à vis de l'hydrolyse de ces différents sites estérifiés.

Suivant une variante du procédé d'enrichissement selon l'invention, on prévoit, avant l'hydrolyse **-2-** *stricto sensu*, une étape **-1bis-** de déracémisation des esters chiraux de l'invention, de préférence au moyen d'une base azotée.
Une telle déracémisation, qui a pour effet d'améliorer la relaxation du polymère, est particulièrement intéressante dans l'optique de générer, préférentiellement, une configuration donnée du centre stéréogénique introduit, de façon à pouvoir récupérer, après hydrolyse, des mélanges d'acides optiquement enrichis.
Selon cette variante, le procédé d'enrichissement comprend les étapes essentielles suivantes :
-**1**- utilisation d'acides ou dérivés sous forme racémique ou énantiométriquement pure, comme produits de départ, pour préparer les esters tels que définis supra ou les esters tels qu'obtenus par le procédé lui aussi présenté ci-avant ;
**-1bis-** déracémisation, de préférence à l'aide d'une ou plusieurs bases azotées prises dans la liste non limitative suivante : la triéthylamine, la pyridine, les picolines, la DMAP, le DABCO, la quinoléine -;
   cette déracémisation permettant, d'une part, la relaxation du composé polyhydroxylé estérifié et permettant, d'autre part, à chaque fontion ester d'adopter la configuration absolue énergiquement la plus stable ;
**-2-** hydrolyse totale ou partielle des motifs esters, de préférence à l'aide d'une base forte, plus préférentiellement encore à l'aide d'une base forte sélectionnée parmi les hydroxydes de métaux alcalins, (LiOH étant tout spécialement sélectionné), de façon à libérer majoritairement l'un des deux énantiomères du précurseur-acide ou dérivé (**II**) de départ.

Outre ce procédé d'enrichissement chiral, la présente invention vise également, en tant que tel, un moyen d'enrichissement chiral pour la mise en oeuvre du susdit procédé, ce moyen étant caractérisé en ce qu'il comprend les esters tels que définis supra et/ou les esters obtenus par le procédé.

Pour compléter le registre des applications des esters chiraux de polysaccharides selon l'invention, on rappellera qu'ils peuvent être utilisés comme support de chromatographie chirale pour la séparation des énantiomères. D'où il s'en suit que la présente invention concerne un support énantiosélectif de chromatographie chirale caractérisé en ce qu'il comprend les esters, tels que décrits supra, et/ou les esters obtenus par le procédé lui aussi décrit supra.

Les exemples qui suivent font apparaître les résultats avantageux obtenus à l'aide des esters selon l'invention, dans le cadre de leur évaluation chromatographique à titre de phase stationnaire chirale.
Il ressort notamment de ces exemples que les phases stationnaires chirales selon l'invention peuvent avoir des ordres d'élution inverses à ceux de phases stationnaires chirales commerciales.

Dans la mesure où les esters selon l'invention peuvent être constitués, en ce qui concerne leurs acides originels (**II**) donnant naissance aux précurseurs, de principes actifs médicamenteux chiraux, et compte tenu, par ailleurs, du procédé d'enrichissement selon l'invention qui permet par hydrolyse des esters, d'obtenir des acides optiquement purs, il est apparu tout à fait judicieux de réaliser des systèmes médicamenteux à libération prolongée et contrôlée de principes actifs, comprenant les esters selon l'invention.

Ainsi, la présente invention vise également un médicament caractérisé :
- en ce qu'il comprend des esters, tels que définis supra, et/ou des esters obtenus par le procédé lui aussi défini supra, l'acide ou les acides précurseur(s) des esters constituant le ou les principes actifs,
- et en ce qu'il est apte à libérer, de manière prolongée et contrôlée, par hydrolyse, le ou les principes actifs.
En effet, l'hydrolyse selon des cinétiques différentes des esters de l'invention, permet de délivrer, de façon contrôlée et prolongée *in vivo* et *in vitro*, des principes actifs acides chiraux (par exemple de type anti-inflammatoire non stéroïdien) ayant une configuration chirale préférentielle. Or, on sait que les médicaments chiraux sont d'autant plus actifs qu'ils se présentent sous forme optiquement purifiée, d'où l'intérêt non contestable du médicament selon l'invention. Ce dernier a également l'avantage d'être particulièrement bien toléré puisque l'on sait que les composés polyhydroxylés (de préférence les polysaccharides) qui constituent un support inerte pour les principes actifs chiraux, sont biocompatibles, biodégradables et non toxiques.

La présente invention sera mieux comprise à la lumière des exemples qui suivent, qui font également ressortir tous ses avantages et ses variantes de réalisation en termes de produits, de procédés et d'applications.

### EXEMPLES

Ces exemples comprennent, dans le chapitre **A** la synthèse des chlorures d'acide ou, autrement dit, des précurseurs-acides ou dérivés (**I**).
Le chapitre **B** est consacré aux synthèses des esters (exemples 1 à 34).
Le chapitre **C** est consacré au procédé d'enrichissement selon l'invention par hydrolyse des esters des exemples 1 à 34, les exemples 42, 43, et 63 à 67 étant plus particulièrement relatifs à la variante comprenant l'étape de déracémisation du procédé d'enrichissement (exemples 35 à 62).
Le chapitre **D** concerne l'évaluation chromatographique des esters synthétisés et utilisés à titre de phase stationnaire chirale. Cette évaluation chromatographique d'esters de cellulose conformes à l'invention est réalisée par la méthode "microbatch", dans un éluant hexane/2-propanol 99/1, effectuée sur 4 racémates (Benzoïne, Base de Tröger, Trans Stilbène Oxyde, Alcool de Pirkle) de la série du Professeur OKAMOTO.

### PREAMBULE

Le schéma réactionnel général est le suivant :

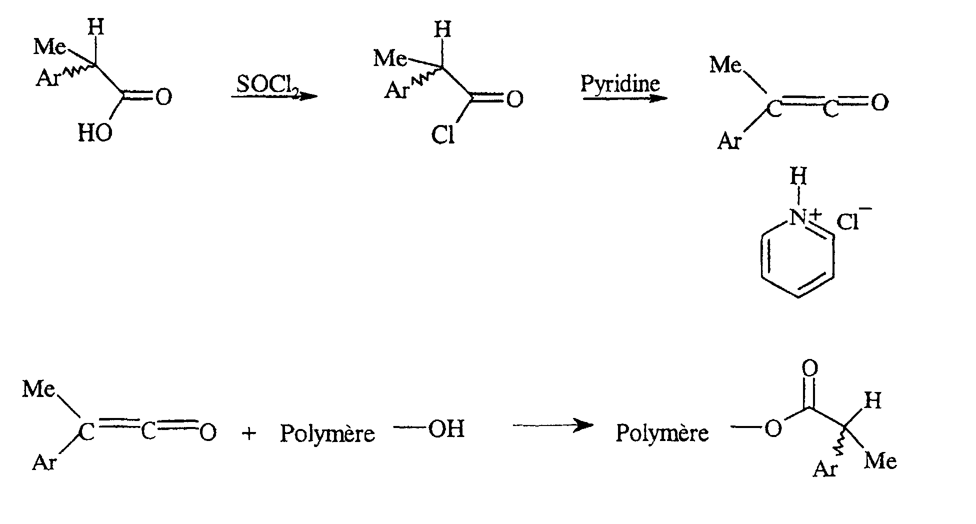

### A - SYNTHESES DES CHLORURES D'ACIDES : PRECURSEURS - ACIDES OU DERIVES (I)

### SYNTHESE DU CHLORURE DE L'ACIDE α-PHENYL PROPIONIQUE:

Dans un ballon bicol de 250 ml, muni d'un réfrigérant surmonté d'une garde à chlorure de calcium et d'une ampoule à addition, sont introduits : 3,50 g (M = 150 g ; 24 mmol) d'acide α-phényl propionique et 100 ml de CH₂Cl₂. Le mélange est agité à température ambiante. On additionne alors goutte à goutte 17,5 ml (10 éq.) de chlorure de thionyle (M = 119 g) fraîchement distillé. Après addition, le mélange est porté au reflux du CH₂Cl₂ pendant 2 h. Après évaporation du mélange réactionnel sous pression réduite, 3,91 g (M = 168,5g ; 23,3 mmol) de chlorure d'acide α-phényl propionique sont obtenus sous forme d'un solide jaune clair, soit un rendement de 96 %.
RMN ¹H (CDCl₃, 200 MHz) δ (ppm) : 1,48 (d, 3H, J = 7,0 Hz, α-CH₃) ; 4,00 (q, 1H, J = 7,0 Hz, α-CH) ; 7,13-7,30 (m, 5H aromatiques).

### SYNTHESE DU CHLORURE DE L'ACIDE α-(P-CHLOROPHENYL) PROPIONIQUE :

Le mode opératoire est identique à celui de la synthèse du chlorure de l'acide α-phénylpropionique. En partant de 4,54g (M = 184,5g, 24,6 mmol) d'acide α-(*p*-chlorophényl) propionique et 17,9 ml (10 éq.) de chlorure de thionyle, on obient 5,00g (M = 203 g, 24,6 mmol) de chlorure d'acide α-(*p*-chlorophényl) propionique, soit un rendement de 100 %.
RMN ¹H (CDCl₃, 200 MHz) δ (ppm) : 1,58 (d, 3H, J = 7,0 Hz, α-CH₃) ; 4,10 (q, 1H, J =7,0 Hz, a-CH) ; 7,21 (dd, 2H aromatiques, J=8,8 Hz et J=2,2 Hz) ; 7,36 (dd, 2H aromatiques, J = 8,6 Hz et J = 2,0 Hz).

### SYNTHESE DU CHLORURE DE KÉTOPROFEN (ACIDE (2-[3-BENZOYLPHÉNYL] PROPIONIQUE) :

Le mode opératoire est identique à celui de la synthèse du chlorure de l'acide α-phényl propionique. En partant de 4,69g (M = 254,3g, 18,4 mmol) de kétoprofen et 13,4 ml (10 éq.) de chlorure de thionyle, on obient 5,26g (M = 272,8g, 18,2 mmol) de chlorure de kétoprofen, soit un rendement de 99 %.
RMN ¹H (CDCl₃, 200 MHz) δ (ppm) : 1,62 (d, 3H, J = 7,0 Hz, a-CH₃) ; 4,20 (q, 1H, J = 7,0 Hz, α-CH) ; 7,43-7,64 (m, 5H aromatiques) ; 7,71-7,83 (m, 4H aromatiques).

### SYNTHESE DU CHLORURE DE L'ACIDE α-(3,5-DIMETHYLPHENYL) PROPIONIQUE :

Le mode opératoire est identique à celui de la synthèse du chlorure de l'acide α-phényl propionique. En partant de 2,32g (M = 178 g, 13 mmol) d'acide α-(3,5-diméthylphényl) propionique et 9,5 ml (10 éq.) de chlorure de thionyle, on obient 2,53g (M = 196,5g, 12,9 mmol) de chlorure d'acide α-(3,5-diméthylphényl) propionique, soit un rendement de 99 %.
RMN ¹H (CDCl₃, 200 MHz) δ (ppm).

### SYNTHESE DU CHLORURE DE L'ACIDE α-(P-METHYLPHENYL) PROPIONIQUE :

Le mode opératoire est identique à celui de la synthèse du chlorure de l'acide α-phényl propionique. En partant de 1,725g (M = 164g, 10,5 mmol) d'acide α-(*p*-méthylphényl) propionique et 7,7 ml (10 éq.) de chlorure de thionyle, on obient 1,92g (M = 182,5g, 10,5 mmol) de chlorure d'acide α-(*p*-méthylphényl) propionique, soit un rendement de 100 %.
RMN ¹H (CDCl₃, 200 MHz) δ (ppm) : 1,49 (d, 3H, J = 7,0 Hz, a-CH₃) ; 2,26 (s, 3H, p-CH₃) ; 4,00 (q, 1H, J = 7,0 Hz, a-CH) ; 7,10 (s, 4H aromatiques).

### SYNTHESE DU CHLORURE DE L'ACIDE α-(2-THIOPHENYL) PROPIONIQUE :

Le mode opératoire est identique à celui de la synthèse du chlorure de l'acide α-phényl propionique. En partant de 1,515g (M = 156g, 9,7 mmol) d'acide α-thiényl propionique et 7,1 ml (10 éq.) de chlorure de thionyle, on obient 1,6g (M = 174,5g, 9,2 mmol) de chlorure d'acide α-thiényl propionique, soit un rendement de 95 %.
RMN ¹H (CDCl₃, 200 MHz) δ (ppm) : 1,68 (d, 3H, J = 7,2 Hz, α-CH₃) ; 4,38 (q, 1H, J = 7,2 Hz, α-CH) ; 7,26-7,30 (m, 2H aromatiques) ; 7,52 (dd, 1H aromatique, J = 4,9 Hz et J =1,5 Hz).

### SYNTHESE DU CHLORURE D'IBUPROFEN (ACIDE α-METHYL-4-[2-METHYLPROPYL]PHENYL ACETIQUE) :

Le mode opératoire est identique à celui de la synthèse du chlorure de l'acide α-phényl propionique. En partant de 5 g (M = 206 g, 24 mmol) d'ibuprofen (acide α-méthyl-4-[2-méthylpropyl]phényl acétique) et 17,5 ml (10 éq.) de chlorure de thionyle, on obient 5,2g (M = 224,5g, 23,2 mmol) de chlorure d'ibuprofen, soit un rendement de 97 %.
RMN ¹H (CDCl₃, 200 MHz) δ (ppm) : 0,90 (d, 6H, J = 6,6 Hz, 2xCH₃) ; 1,57 (d, 3H, J = 7,0 Hz, α-CH₃) ; 1,85 (mhept, 1H, J = 6,6 Hz, 1H isopropylique) ; 2,47 (d, 2H, J = 7,2 Hz, -CH₂Ar) ; 4,09 (q, 1H, J = 7,0 Hz, -CHAr) ; 7,12-7,20 (m, 4H aromatiques).

### SYNTHÈSE DU CHLORURE DE NAPROXEN (ACIDE 6-METHOXY-α-METHYL-2-NAPHTALENE ACETIQUE) :

Le mode opératoire est identique à celui de la synthèse du chlorure de l'acide α-phényl propionique. En partant de 8,50 g (M = 230g ; 37 mmol) de S-naproxen ((+)-6-méthoxy-α-méthyl-2-naphtalène acetic acid) et 27 ml (10 éq.) de chlorure de thionyle, on obient 9,18 g (M = 248,5g, ) de chlorure de naproxen, soit un rendement de 100 %.
RMN ¹H (CDCl₃, 200 MHz) δ (ppm) : 1,67 (d, 3H, J = 7,0 Hz, α-CH₃) ; 3,92 (s, 3H O-CH₃) ; 4,25 (q, 1H, J = 7,0 Hz, α-CH) ; 7,13 (d, 1H aromatique, J = 2,5 Hz, ) ; 7,17 (dd, 1H aromatique, J = 8,8 Hz et J = 2,5 Hz) ; 7,35 (dd, 1H aromatique, J = 8,5 Hz et J = 1,9 Hz) ; 7,68 (d, 1H aromatique, J = 1,5 Hz) ; 7,73 (d, 1H aromatique, J = 8,8 Hz) ; 7,75 (d, 1H aromatique, J = 8,5 Hz).

### B - SYNTHESE DES ESTERS

### EXEMPLE 1 : SYNTHESE DE L'α-PHENYL PROPIONATE DE CELLULOSE DANS LA PYRYDINE (48 H DE REACTION) :

Dans un ballon tricol de 100 ml muni d'un réfrigérant surmonté d'une garde à chlorure de calcium, d'une ampoule à addition et d'un thermomètre, on place 0,4g (2,5 mmol) de cellulose microcristalline Avicel (Merck 2331, M = [(162)n]g sur la base du monomère glucose), préalablement séchée pendant toute une nuit à 120 °C à l'étuve à vide, et 40 ml de pyridine, préalablement distillée et mise sur KOH. On agite et on chauffe pendant une heure à 80 °C. On laisse le mélange revenir à température ambiante et on ajoute goutte à goutte 3,5g (21 mmol, 8,5 équivalents) de chlorure d'acide α-phényl propionique racémique fraîchement préparé. La réaction est portée à 115 °C pendant 48 h.
Après refroidissement le mélange réactionnel est versé dans 100 ml de méthanol sous agitation. Le précipité formé est récupéré par filtration puis dissous dans 30 ml de dichlorométhane. La solution est filtrée et concentrée sous pression réduite. Le résidu est versé dans 100 ml de méthanol sous agitation et on récupère par filtration le polymère greffé sous forme d'une poudre de couleur sombre. Ces opérations de dissolution-précipitation sont effectuées une deuxième fois. Le produit obtenu est séché à l'étuve à vide pendant deux heures à 50 °C. On obtient 1.23g (rdt = 89 % sur la base d'une trisubstitution) d'un polymère chiral α-phényl propionate de cellulose avec un degré de substitution (DS) de 2.94 déterminé par analyse élémentaire.

### ANALYSES :

- IR (KBr) νₘₐₓ (cm⁻¹): 3100-2800 (-CH₃), 1760 (C=O), 1610/1510/710 (C=C aromatiques en para), 1480 (δas-CH₃), 1170 et 1090 (C=O/C-O).
- ¹H-RMN CDCl₃, 200 MHz) δ (ppm) : 1-1.5 (m, α-CH₃). 2.2-5,1 (m, H cellulosiques + H benzyliques), 6,7-7,5 (m, H aromatiques).
- ¹³C-RMN (CDCl₃, 200 MHz) δ (ppm) : 19,2 (s. α-CH₃), 45,36 (s, CH₂ alcool primaire du glucose), 62,3 (s, α-CH), 72,17 (m, 4 CH glucose), 99,43 (s, CH glucose), 127,84 128,9 129,46 (s, CH aromatiques), 140,11 (s, C_{IV} aromatiques). 173.8 (s, C = 0 ester).
- Analyse Elémentaire :
   - % calculé : C 70,96 %, H 6,09 % (pour une trisubstitution).
   - % obtenu : C 70,81 %, H 6,08 %.
- Pouvoir rotatoire : [α]²⁰ = -104,0° (λ = 436 nm, c = 0,8 g/dm³, CHCl₃).
   La répartition des motifs acyles fixés R et S est respectivement de 62 % et 38 % (répartition déterminée par hydrolyse, cf. exemple 35).

### EXEMPLE 1A : SYNTHESE DE L'α-PHENYL PROPIONATE DE CELLULOSE DANS LA PYRIDINE (48 H DE REACTION, à 25 °C) :

On opère comme dans l'exemple 1, mais en réalisant la réaction à 25 °C au lieu de 115 °C et avec 7 équivalents (au lieu de 8,5) de chlorure d'acide α-phénylpropionique racémique. On obtient un polymère chiral d'α-phénylpropionate de cellulose avec un rendement de 61 % (sur la base d'une trisubstitution) et un DS=2.97 déterminé par analyse élémentaire. Les analyses IR et RMN sont identiques à celles décrites dans l'exemple 1.
- Analyse élémentaire :
   % calculé : C 70,96 %, H 6,09 % pour une trisubstitution
   % obtenu : C 70,86 %, H 6,12 %
   La répartition des motifs acyles fixés R et S est respectivement de 68 % et 32 % (répartition déterminée par hydrolyse, cf. exemple 35A).

### EXEMPLE 1B : SYNTHESE DE L'α-PHENYL PROPIONATE DE CELLULOSE DANS UN MELANGE PYRIDINE/TRIETHYLAMINE/DMAP (48 H DE REACTION, à 120 °C) :

On opère comme dans l'exemple 1, mais en présence d'un mélange de pyridine/triéthylamine/DMAP (4-(N,N-diméthylamino)pyridine), la pyridine et la triéthylamine étant en proportion 2/1 (v/v) et la DMAP en quantité catalystique. La réaction est réalisée avec 7 équivalents (au lieu de 8,5) de chlorure dacide α-phénylpropionique racémique. On obtient un polymère chiral d'α-phénylpropionate de cellulose avec un rendement de 70 % (sur la base d'une trisubstitution) et un DS=2,96 déterminé par analyse élémentaire. Les analyses IR et RMN sont identiques à celles décrites dans l'exemple 1.
- Analyse élémentaire :
   % calculé : C 70,96 %, H 6,09 % (pour une trisubstitution).
   % obtenu : C 70,88 %, H 6,12 %.
   La répartition des motifs esters fixés R et S est respectivement de 47 % et 53 % (répartition déterminée par hydrolyse, cf. exemple 35B).

### EXEMPLE 1C : SYNTHESE DE L'α-PHENYL PROPIONATE DE CELLULOSE DANS UN MELANGE PYRIDINE/DABCO/DMAP (24 H DE REACTION, à 90 °C) :

On opère comme dans l'exemple 1, mais en présence d'un mélange de pyridine/DABCO/DMAP, la pyridine et le DABCO (1,4-diazabicyclo[2.2.2]octane) étant en proportion 48ml et 9,6g et la DMAP en quantité catalytique. La réaction est réalisée avec 7 équivalents (au lieu de 8,5) de chlorure d'acide α-phénylpropionique racémique. On obtient un polymère chiral d'α-phénylpropionate de cellulose avec un rendement de 31 % (sur la base d'une trisubstitution) et un DS=2,00 déterminé par analyse élémentaire. Les analyses IR et RMN sont identiques à celles décrites dans l'exemple 1.
- Analyse élémentaire :
   % calculé : C 70,96 %, H 6,09 % (pour une trisubstitutton).
   % obtenu : C 67,76 %, H 5,97 %.
La répartition des motifs esters fixés R et S est respectivement de 46 % et 54 % (répartition déterminée par hydrolyse, cf. exemple 35C).

### EXEMPLE 1D: SYNTHESE DE L'α-PHENYL PROPIONATE DE CELLULOSE DANS LA 2-PICOLINE (48 H DE REACTION, à 115 °C) :

On opère comme dans l'exemple 1, mais en réalisant la réaction dans la 2-picoline. On obtient un polymère chiral d'α-phénylpropionate de cellulose avec un rendement de 52 % (sur la base d'une trisubstitution) et un DS=1.87 déterminé par analyse élémentaire.
Les analyses IR et RMN sont identiques à celles décrites dans l'exempie 1.
- Analyse élémentaire :
   % calculé : C 70,96 %, H 6,09 % (pour une trisubstitution).
   % obtenu : C 67,24 %, H 6,02 %.
La répartition des motifs esters fixés R et S est respectivement de 43 % et 57 % (répartition déterminée par hydrolyse, cf. exemple 35D).

### EXEMPLE 1E : SYNTHESE DE L'α-PHENYL PROPIONATE DE CELLULOSE DANS LA 4-PICOLINE (48 H DE REACTION, à 115 °C) :

On opère comme dans l'exemple 1, mais en réalisant la réaction dans la 4-picoline. On obtient un polymère chiral d'α-phénylpropionate de cellulose avec un rendement de 26 % (sur la base d'une trisubstitution) et un DS=1.13 déterminé par analyse élémentaire.
Les analyses IR et RMN sont identiques à celles décrites dans l'exemple 1.
- Analyse élémentaire :
   % calculé : C 70,96 %, H 6,09 % (pour une trisubstitution).
   % obtenu : C 62,77 %, H 6,34 %.
La répartition des motifs esters fixés R et S est respectivement de 54 % et 46 % (répartition déterminée par hydrolyse, cf. exemple 35E).

### EXEMPLE 1F : SYNTHESE DE L'α-PHENYL PROPIONATE D'AMYLOPECTINE DANS LA PYRIDINE (24 H DE REACTION, à 100 °C):

Dans un ballon tricol de 100 ml, muni d'un réfrigérant surmonté d'une garde à chlorure de calcium, d'un septum et d'une ampoule à addition, sont introduits : 0,90g (5,56 mmole) de glucidex préalablement séché toute une nuit sous vide à 120 °C et 60 ml de pyridine préalablement distillée et séchée sur KOH.
Le mélange est porté à 80°C, puis est refroidi. Puis, 8,42g (9 éq.) de chlorure d'acide α-phénylpropionique sont alors ajoutés à la seringue et à température ambiante au milieu réactionnel, pendant 10 min. Le mélange est ensuite porté au reflux à 100 °C pendant 24h.
Le mélange préalablement refroidi est versé sur 300 ml de MeOH. Un précipité d'ester d'amylopectine se forme immédiatement. Ce dernier est filtré et séché, puis dissous dans un minimum de CH₂Cl₂. A nouveau, la solution dans le CH₂Cl₂ est versée goutte à goutte sur 50 ml de MeOH sous agitation pour obtenir un précipité lavé de ses impuretés. Le précipité, une fois filtré et séché 4h sous vide à 25 °C, est obtenu sous forme d'une poudre très fine : 2,09g (Rdt = 67 % sur la base d'une trisubstitution). DS = 2,92 déterminé par analyse élémentaire.

### ANALYSES :

- IR (KBr) νₘₐₓ(cm⁻¹) :3100-2800 (-CH₃), 1770 (C=O), 1600/1510/700 (C=C aromatiques en para),1460 (δₐₛ CH₃), 1170 et 1090 (C=O/C-O).
- RMN ¹H (CDCl₃, 200 MHz) δ (ppm) : 0,8-1,6 (m, α-CH₃) : 2,5-5,5 (m, O-CH₃ + H amylopectine + H benzyliques) ; 6,5-7,5 (m, H aromatiques).
- Analyse élémentaire :
   % calculé : C 70,96 %, H 6,09 % pour une trisubstitution
   % obtenu : C 70,86 %, H 6,12 %
La répartition des motifs esters fixés R et S est respectivement de 49 % et 51 % (répartition déterminée par hydrolyse, cf. exemple 35F).

### EXEMPLE 2 : SYNTHESE DE L'α-PHENYL PROPIONATE DE CELLULOSE DANS LA TRIETHYLAMINE :

On opère comme dans l'exemple 1, mais en remplaçant la pyridine par de la triéthylamine ; avec 0,42g de cellulose, 3,6g (9 équivalents) de chlorure d'acide α-phényl propionique racémique, 40 ml de triéthylamine et un reflux à 115 °C. On obtient alors la cellulose initiale non estérifiée (polymère totalement insoluble dans le dichlorométhane, IR identique à la cellulose initiale).

### EXEMPLE 3 : SYNTHESE DE L'α-PHENYL PROPIONATE DE CELLULOSE DANS LA PYRIDINE (24H DE REACTION) :

On opère comme dans l'exemple 1, mais avec un temps de réaction de 24 h au lieu de 48 h ; avec 0,4g de cellulose, 3,7g (9 équivalents) de chlorure d'acide α-phényl propionique racémique, 40 ml de pyridine et un reflux à 115 °C. On obtient alors 0.63g (rdt = 44.2 % sur la base d'une trisubstitution) d'un polymère chiral α-phényl propionate de cellulose avec un degré de substitution, de 2,5 détermine par analyse élémentaire.

### ANALYSES :

- IR (KBr) νₘₐₓ (cm⁻¹) : 3500 (OH), 3100-2850 (-CH₃), 1760 (C=O), 1610/1510/710 (C=C aromatiques en para), 1480 (δas-CH₃), 1170 et 1090 (C=O/C-O),
- ¹H-RMN (CDCl₃, 200 MHz) δ (ppm) : 0,8-1,5 (m, α-CH₃), 2,2-5,2 (m, H cellulosiques + H benzyliques), 6,7-7,4 (m, H aromatiques).
- Analyse Elémentaire :
   % calculé : C 70,96 %, H 6,09 % (pour une trisubstitution).
   -% obtenu : C 69,64 %, H 6,08 %.
- Pouvoir rotatoire : [α]²⁰ = -70,0° (λ = 436 nm, c = 0.7 g/dm³, CHCl₃).
La répartition des motifs acyles fixés R et S est respectivement de 58 % et 42 % (répartition déterminée par hydrolyse, cf. exemple 36).

### EXEMPLE 4: SYNTHESE DE L' α-PHENYL PROPIONATE DE CELLULOSE DANS UN MELANGE PYRIDINE/TOLUENE :

Dans un ballon tricol de 100 ml muni d'un réfrigérant surmonté d'une garde à chlorure de calcium, d'une ampoule à addition et d'un thermomètre, on place 0,4g (2,5 mmol) de cellulose microcristalline Avicel (Merck 2331, [(162)n]g sur la base du monomère glucose), préalablement séchée pendant toute une nuit à 120 °C à l'étuve à vide, et 60 ml de toluène, préalablement distillé et séché sur tamis moléculaire. On agite et on chauffe pendant une heure à 80 °C. On ajoute 4 ml de pyridine préalablement distillée et déposée sur KOH. On laisse le mélange revenir à température ambiante et on ajoute, goutte à goutte en 10 min, 1,3g (8 mmol, 3 équivalents) de chlorure d'acide α-phényl propionique racémique, fraîchement préparé, en mélange avec 10 ml de toluène. La réaction est portée à 125 °C pendant 5 h. On laisse le mélange revenir à température ambiante et on rajoute, goutte à goutte en 10 min, 2.1g (12 mmol, 5 équivalents) de chlorure d'acide α-phényl propionique, fraîchement préparé, en mélange avec 10 ml de toluène. La réaction est de nouveau portée à 125 °C pendant 43 h.
Après refroidissement, le mélange réactionnel est versé dans 100 ml de méthanol sous agitation. Le précipité formé est récupéré par filtration, puis dissous dans 30 ml de dichlorométhane. La solution est filtrée et concentrée sous pression réduite. Le résidu est versé dans 100 ml de méthanol sous agitation et on récupère, par filtration, le polymère greffé sous forme d'une poudre de couleur sombre. Ces opérations de dissolution-précipitation sont effectuées une deuxième fois. Le produit obtenu est séché à l'étuve à vide pendant deux heures à 50 °C. On obtient 1.13g (rdt = 80 -% sur la base d'une trisubstitution) d'un polymère chiral α-phényl propionate de cellulose avec un degré de substitution de 2,5 déterminé par analyse élémentaire.

### ANALYSES :

- IR(KBr) νₘₐₓ (cm⁻¹): 3500 (OH), 3100-2800 (-CH₃), 1760 (C=O), 1610/1510/710 (C=C aromatiques en para), 1480 (δas-CH₃), 1170 et 1090 (C=O/C-O),
- ¹H-RMN (CDCl₃, 200 MHz) δ (ppm): 0,9-1,9 (m, α-CH₃), 2,4-5,1 (m, H cellulosiques + H benzyliques), 6,7-7,5 (m, H aromatiques).
- Analyse Elémentaire :
   % calculé : C 70,96 %, H 6,09 % (pour une trisubstitution).
   -% obtenu : C 69,51 %, H 6,07 %.
- Pouvoir rotatoire : [α]²⁰ = -75.0° (λ = 436 nm, c = 0,7 g/dm³, CHCl₃).
La répartition des motifs acyles fixés R et S est respectivement de 58 % et 42 % (répartition déterminés par hydrolyse, cf. exemple 37).

### EXEMPLE 5 : SYNTHESE DE L'α-PHENYL PROPIONATE DE CELLULOSE DANS UN MELANGE DE TOLUENE ET TRIETHYLAMINE :

On opère comme dans l'exemple 3, mais en remplaçant la pyridine par de la triéthylamine ; avec 0,41g de cellulose, 60 ml de toluène, 1,3 g puis 2 g (7 équivalents) de chlorure d'acide α-phényl propionique racémique en mélange avec 10 ml de toluène, 5 ml de triéthylamine et un reflux à 120 °C. On obtient la cellulose initiale non estérifiée (polymère totalement insoluble dans le dichlorométhane. IR identique à la cellulose initiale).

### EXEMPLE 6 : SYNTHESE DU DI-α-PHENYL PROPIONATE DE CELLULOSE DANS LA PYRIDINE:

On opère comme dans l'exemple 1, mais à une température de réaction de 90 °C au lieu de 115 °C, avec trois équivalents de chlorure d'acide α-phényl propionique racémique au lieu de huit et en effectuant une agitation ultrasonique pendant 30 min au moment de la dissolution de la phase dans le dichlorométhane (0,6g de cellulose, 1,9g (trois équivalents) de chlorure d'acide α-phényl propionique racémique, 45 ml de pyridine, chauffage à 90 °C). On obtient alors 0,93g (rdt = 58,9- % sur la base d'une disubstitution) d'un polymère chiral α-phényl propionate de cellulose avec un degré de substitution de 1,99 déterminé par analyse élémentaire.

### ANALYSES :

- IR(KBr) νₘₐₓ (cm⁻¹): 3530-2900 (OH), 3100-2800 (-CH₃),1760 (C=O), 1610/1510/700 (C=C aromatiques en para), 1480 (δas-CH₃), 1170 et 1090 (C=O/C-O).
- ¹H-RMN (CDCl₃, 200 MHz) δ (ppm): 0,9-1,8 (m, α-CH₃), 2,5-5,3 (m, H cellulosiques + H benzyliques + OH résiduels), 6,7-7,4 (m, H aromatiques).
- Analyse Elémentaire :
   % calculé : C 67,6 %, H 6,1 % (pour une disubstitution).
   -% obtenu : C 67,56 %, H 6,1 %.
- Le pouvoir rotatoire n'a pas été mesuré à cause de la solubilité insuffisante du polymère dans le CHCl₃.
La répartition des motifs acyles fixés R et S est respectivement de 60 % et 40 % (répartition déterminée par hydrolyse, cf. exemple 38).

### EXEMPLE 7 : SYNTHESE DU MONO-α-PHENYL PROPIONATE DE CELLULOSE DANS LA PYRIDINE :

Dans un ballon tricol de 100 ml muni d'un réfrigérant surmonté d'une garde à chlorure de calcium, d'une ampoule à addition et d'un thermomètre, on place 0,92g (5,7 mmol) de cellulose microcristalline Avicel (Merck 2331, M = [(162)n]g sur la base du monomère glucose), préalablement séchée pendant toute une nuit à 120 °C à l'étuve à vide, et 40 ml de pyridine, préalablement distillée et mise sur KOH. On agite et on chauffe pendant une heure à 80 °C. On laisse le mélange revenir à température ambiante et on ajoute goutte à goutte 1,7 g (11 mmol, 1,8 équivalents) de chlorure d'acide α-phényl propionique racémique fraîchement préparé. La réaction est portée à 90 °C pendant 48 h.
Le mélange réactionnel est versé dans 50 ml de méthanol sous agitation. Le précipité formé est récupéré par filtration, puis agité aux ultrasons pendant 15 min dans 50 ml de dichlorométhane. La solution est filtrée sur papier et concentrée sous pression réduite. Le résidu est versé dans 50 ml de méthanol sous agitation et on récupère par filtration un polymère greffé de couleur beige. Ces opérations de dissolution-filtration-précipitation sont effectuées une deuxième fois avec une filtration intermédiaire de la solution de dichlorométhane s'effectuant sur un papier de porosité plus fine. Le produit obtenu est séché à l'étuve à vide pendant deux heures à 50 °C. On obtient 1 136g (rdt = 68 -% sur la base d'une monosubstitution) d'un polymère chiral α-phényl propionate de cellulose avec un degré de substitution de 0,91 déterminé par analyse élémentaire.

### ANALYSES :

- IR(KBr) νₘₐₓ (cm⁻¹) : 3600-3200 (OH), 3020-2800 (-CH₃), 1760 (C=O), 1610/1510/710 (C=C aromatiques en para), 1470 (δas-CH₃), 1170 et 1090 (C=O/C-O).
- ¹H-RMN (Pyridine d5, 200 MHz) δ (ppm) : 1-2 (m, α-CH₃), 3-6 (m, H cellulosiques + H benzyliques + OH résiduels), 7-8 (m, H aromatiques).
- Analyse Elémentaire :
   % calculé : C 61,2 %. H 6,12 % (pour une disubstitution).
   -% obtenu : C 60,36 %, H 6,1 %.
- Le pouvoir rotatoire n'a pas été mesuré à cause de la faible solubilité du polymère dans le CHCl₃.
La répartition des motifs acyles fixés R et S est respectivement de 58 % et 42 % (répartition déterminée par hydrolyse, cf. exemple 39).

### EXEMPLE 8 : SYNTHESE DE L'α-PHENYL PROPIONATE D'AMYLOSE DANS LA PYRIDINE :

On opère comme dans l'exemple 1, mais en remplaçant la cellulose par de l'amylose B (Nacalaï Tesque Mw = 16-000) ; avec 0,4g d'amylose, 2,9g (7 équivalents) de chlorure d'acide α-phényl propionique racémique, 40 ml de pyridine et un reflux à 115 °C pendant 50 h. On obtient alors 0,887g (rdt = 64,4 % sur la base d'une trisubstitution) d'un polymère chiral α-phényl propionate d'amylose avec un degré de substitution de 3 déterminé par analyse élémentaire.

### ANALYSES :

- IR(KBr) νₘₐₓ (cm⁻¹) : 3100-2800 (-CH₃), 1760 (C=O), 1610/1500/700 (C=C aromatiques en para), 1480 (δas-CH₃), 1170 et 1090 (C=O/C-O).
- ¹H-RMN (CDCl₃, 200 MHz) δ (ppm) : 0,7-1,8 (m, α-CH₃), 2,8-5,5 (m, H cellulosiques + H benzyliques), 6,6-7,6 (m, H aromatiques).
- Analyse Elémentaire :
   % calculé : C 70,96 %, H 6,09 % (pour une trisubstitution).
   -% obtenu : C 70,95 %, H 6,09 %.
- Pouvoir rotatoire : [α]²⁰ = 113,0° (λ = 436 nm, c = 1 g/dm³, CHCl₃).
La répartition des motifs acyles fixés R et S est respectivement de 53 % et 47 % (répartition déterminée par hydrolyse, cf. exemple 40).

### EXEMPLE 9 : SYNTHESE DE L'α-PHENYL PROPIONATE DE β-CYCLODEXTRINE DANS LA PYRIDINE :

On opère comme dans l'exemple 1, mais en remplaçant la cellulose par de la β-cyclodextrine (Société Roquette, M = [(162)n]g, lot E. 0113); avec 0,374g de β-cyclodextrine, 3,5g (9 équivalents) de chlorure d'acide α-phényl propionique racémique, 40 ml de pyridine et un reflux à 115 °C pendant 18 h. On obtient alors 0,432g (rdt = 33,5 -% sur la base d'une trisubstitution) d'un polymère chiral α-phényl propionate de β-cyclodextrine avec un degré de substitution de 2,9 déterminé par analyse élémentaire.

### ANALYSES :

- IR(KBr) νₘₐₓ (cm⁻¹): 3100-2800 (-CH₃), 1770 (C=O), 1600/1510/700 (C=C aromatiques en para),1460 (δas-CH₃), 1170 et 1090 (C=O/C-O).
- ¹H-RMN (CDCl₃, 200 MHz) δ (ppm) : 0,8-2 (m, α-CH₃), 2,7-5,4 (m, H cellulosiques + H benzyliques), 6,5-7,8 (m, H aromatiques).
- Analyse Elémentaire :
   % calculé : C 70,96 %, H 6,09 % (pour une trisubstitution).
   -% obtenu : C 70,80 %, H 6,10 %.
- Pouvoir rotatoire : [α]²⁰ = +77,8° (λ = 436 nm, c = 1,6 g/dm³, CHCl₃).

### EXEMPLE 10 : SYNTHESE DE L'α-(P-CHLOROPHENYL) PROPIONATE DE CELLULOSE DANS LA PYRIDINE :

Dans un ballon tricol de 100 ml muni d'un réfrigérant surmonté d'une garde à chlorure de calcium, d'une ampoule à addition et d'un thermomètre on place 0,4g (2,5 mmol) de cellulose microcristalline Avicel (Merck 2331, M = [(162)n]g sur la base du monomère glucose), préalablement séchée pendant toute une nuit à 120 °C à l'étuve à vide, et 40 ml de pyridine, préalablement distillée et mise sur KOH. On agite et on chauffe pendant une heure à 80 °C. On laisse le mélange revenir à température ambiante et on ajoute, goutte à goutte, 3,3g (18 mmol, 7 équivalents) de chlorure d'acide α-(*p*-chlorophényl) propionique racémique fraîchement préparé. La réaction est portée à 115 °C pendant 30h.
Après refroidissement, on ajoute au mélange réactionnel 40 ml de dichlorométhane et 20 ml d'une solution saturée de Na₂CO₃. On agite 15 min et on sépare la phase aqueuse et la phase organique par décantation. On lave la phase organique avec 10 ml d'eau, on la sèche avec du MgSO₄, puis on la concentre sous pression réduite jusqu'à obtenir une solution d'environ 5 à 10 ml. Cette solution est versée dans 50 ml de méthanol sous agitation et le précipité formé est récupéré par filtration. Le polymère est dissous dans 30 ml de dichlorométhane ; cette solution est ensuite filtrée et concentrée sous pression réduite. Le résidu est versé dans 50 ml de méthanol sous agitation et on récupère par filtration un polymère greffé de couleur sombre. Ces opérations de dissolution-précipitation sont effectuées une deuxième fois. Le produit obtenu est séché à l'étuve à vide pendant deux heures à 50 °C. On obtient 1,56g (rdt = 95,5 -% sur la base d'une trisubstitution) d'un polymère chiral α-(*p*-chlorophényl) propionate de cellulose avec un degré de substitution de 3 déterminé par analyse élémentaire.

### ANALYSES :

- IR(KBr) νₘₐₓ (cm⁻¹): 3000-2800 (-CH₃), 1760 (C=O), 1600/1500/780 (C=C aromatiques disubstitués en para), 1480 (δas-CH₃), 1170 et 1090 (C=O/C-O), 810 (C-Cl).
- ¹H-RMN (CDCl₃, 200 MHz) δ (ppm) : 0,8-1,8 (m, α-CH₃), 2,2-5,2 (m, H cellulosiques + H benzyliques), 6,6-7,6 (m, H aromatiques).
- Analyse Elémentaire :
   % calculé : C 59,86 %, H 4,68 % (pour une trisubstitution).
   -% obtenu : C 59,86 %, H 4,68 %.
- Pouvoir rotatoire : [α]²⁰ = -63,0° (λ = 436 nm, c = 2,05 g/dm³, CHCl₃).
La répartition des motifs acyles fixés R et S est respectivement de 56 % et 44 % (répartition déterminée par hydrolyse, cf. exemple 41).

### EXEMPLE 11 : SYNTHESE DU MONO-α-(P-CHLOROPHENYL) PROPIONATE DE CELLULOSE DANS LA PYRIDINE :

On opère comme dans l'exemple 7, mais en remplaçant l'acide α-phényl propionique par l'acide α-(*p*-chlorophényl) propionique ; avec 0,92g de cellulose, 1,9g (1,8 équivalents) de chlorure d'acide α-(*p*-chlorophényl) propionique racémique, 40 ml de pyridine et chauffage à 90 °C. On obtient alors 1 679 g (rdt = 90-% sur la base d'une monosubstitution) d'un polymère chiral mono α-(*p*-chlorophényl) propionate de cellulose avec un degré de substitution de 0,96 déterminé par analyse élémentaire.

### ANALYSES :

- IR(KBr) νₘₐₓ (cm⁻¹): 3800-3100 (OH), 3000-2800 (-CH₃), 1760 (C―=―O), 1600/1500/780 (C-=-C aromatiques disubstitués en para), 1480 (δas-CH₃), 1170 et 1090 (C-=-O/C-O) cm⁻¹, 850 (C-Cl).
- ¹H-RMN (Pyridine d5, 200 MHz) δ (ppm) : 1-2 (m, α-CH₃), 3-6 (m, H cellulosiques + H benzylique + OH résiduels), 7-7,9 (m, H aromatiques).
- Analyse Elémentaire :
   % calculé : C 54,79 %, H 5,17 % (pour une disubstitution).
   -% obtenu : C 54,57 %, H 5 %.
- Le pouvoir rotatoire n'a pas été mesuré à cause de la faible solubilité du polymère dans le CHCl₃.

### EXEMPLE 12 : SYNTHESE DU MONO-α-(P-CHLOROPHENYL) PROPIONATE DE CELLULOSE DANS LA PYRIDINE :

On opère comme dans l'exemple 11, avec 1g de cellulose, 1,3g (1,15 équivalents) de chlorure d'acide α-(*p*-chlorophényl) propionique racémique, 40 ml de pyridine et un chauffage à 90 °C. On obtient alors 1 603 g (rdt = 79,1 -% sur la base d'une monosubstitution) d'un polymère chiral mono α-(*p*-chlorophényl) propionate de cellulose avec un degré de substitution de 0,6 déterminé par analyse élémentaire.

### ANALYSES :

- IR(KBr) νmax (cm-1) : 3700-3100 (OH), 3000-2800 (-CH3), 1760 (C=O), 1600/1500/780 (C= aromatiques disubstitués en para), 1480 (δas-CH3), 1170 et 1090 (C=O/C-O), 840 (C-Cl).
- ¹H-RMN (Pyridine d5, 200 MHz) δ (ppm) : 1-2 (m, α-CH₃), 3-6 (m, H cellulosiques + H benzylique + OH résiduels), 7-7,9 (m, H aromatiques).
- Analyse Elémentaire :
   % calculé : C 54,79 %, H 5,17 % (pour une disubstitution).
   -% obtenu : C 52,26 %, H 4,89 %.
- Le pouvoir rotatoire n'a pas été mesuré à cause de la faible solubilité du polymère dans le CHCl₃.

### EXEMPLE 13 : SYNTHESE DE L'α-(P-CHLOROPHENYL) PROPIONATE D'AMYLOSE DANS LA PYRIDINE :

On opère comme dans l'exemple 10, mais en remplaçant la cellulose par de l'amylose B (Nacalaï Tesque Mw = 16-000) ; avec 0,5 g de cellulose, 3,3 g (cinq équivalents) de chlorure d'acide α-(*p*-chlorophényl) propionique racémique, 40 ml de pyridine, et un reflux à 115 °C, pendant 30 h. On obtient alors 1,95g (rdt = 98 -% sur la base d'une trisubstitution) d'un polymère chiral α-(*p*-chlorophényl) propionate de cellulose avec un degré de substitution de 3 déterminé par analyse élémentaire.

### ANALYSES :

- IR(KBr) νₘₐₓ (cm⁻¹) : 3000-2900 (-CH₃), 1760 (C=O), 1600/1500/760 (C=C aromatiques disubstitués en para), 1480 (δas-CH₃), 1170 et 1090 (C=O/C-O), 820 (C-Cl).
- ¹H-RMN (CDCl₃, 200 MHz) δ (ppm): 0,8-1,9 (m, α-CH₃), 2,5-5,3 (m, H cellulosiques + H benzyliques), 6,6-7,6 (m, H aromatiques).
- Analyse Elémentaire :
   % calculé : C 59,86 %, H 4,68 % (pour une trisubstitution).
   % obtenu : C 59,86 %, H 4,65 %.
- POUVOIR ROTATOIRE : [α]²⁰ = 90,0° (λ = 436 nm, c = 1,58 g/dm³, CHCl₃).
La répartition des motifs acyles fixés R et S est respectivement de 52 % et 48 % (répartition déterminée par hydrolyse, cf. exemple 44).

### EXEMPLE 14 : SYNTHESE DU KETOPROFENATE DE CELLULOSE DANS LA PYRIDINE :

Dans un ballon tricol de 100 ml muni d'un réfrigérant surmonté d'une garde à chlorure de calcium, d'une ampoule à addition et d'un thermomètre on place 0.295g (1.8 mmol) de cellulose microcristalline Avicel (Merck 2331, M = [(162)n]g sur la base du monomère glucose), préalablement séchée pendant toute une nuit à 120 °C à l'étuve à vide, et 50 ml de pyridine, préalablement distillée et mise sur KOH. On agite et on chauffe pendant une heure à 80 °C. On laisse le mélange revenir à température ambiante et on ajoute goutte à goutte 4.85g (18 mmol, 10 équivalents) de chlorure de kétoprofen racémique fraîchement préparé. La réaction est portée à 90 °C pendant 22 h puis à 120 °C pendant 40 h.
Après refroidissement le mélange réactionnel est versé dans 100 ml de méthanol sous agitation. Le précipité formé est récupéré par filtration puis dissous dans 50 ml de dichlorométhane. La solution est filtrée et concentrée sous pression réduite. Le résidu est versé dans 100 ml de méthanol sous agitation et on récupère par filtration un polymère greffé de couleur sombre. Ces opérations de dissolution-précipitation sont effectuées une deuxième fois. Le produit obtenu est séché à l'étuve à vide pendant deux heures à 50 °C. On obtient 0.68g (rdt = 43 -% sur la base d'une trisubstitution) d'un polymère chiral kétoprofénate de cellulose avec un degré de substitution de 2.35 déterminé par analyse élémentaire.

### ANALYSES :

- IR(KBr) νₘₐₓ (cm⁻¹) : 3500 (OH), 3000-2900 (-CH₃), 1760 (C=O ester), 1680 et 1300 (C=O cétone), 1610/1590/780 (C=C aromatiques ), 1480 (δas -CH₃), 1170 et 1080 (C=O/C-O).
- ¹H-RMN (CDCl₃, 200 MHz) δ (ppm): 0,8-1,5 (m, α-CH₃), 2,3-5,3 (m, H cellulosiques + H benzyliques), 7-7,8 (massif, H aromatiques).
- Analyse Elémentaire :
   - % calculé : C 74,48 %, H 5,28 % (pour une trisubstitution).
   - % obtenu : C 73,00 %, H 5,33 %
- Pouvoir rotatoire : [α]²⁰ = -64,0° (λ = 436 nm, c = 1,5 g/dm³, CHCl₃).

La répartition des motifs acyles fixés R et S est respectivement de 58 % et 42 % (répartition déterminée par hydrolyse, cf. exemple 45).

### EXEMPLE 15 : SYNTHESE DU KETOPROFENATE DE CELLULOSE DANS UN MELANGE PYRIDINE/TRIETHYLAMINE/DMAP :

Dans un ballon tricol de 100 ml muni d'un réfrigérant surmonté d'une garde à chlorure de calcium, d'une ampoule à addition et d'un thermomètre on place 0,5g (3,1 mmol) de cellulose microcristalline Avicel (Merck 2331, M = [(162)n]g sur la base du monomère glucose), préalablement séchée pendant toute une nuit à 120 °C à l'étuve à vide, et un mélange : pyridine (20 ml, préalablement distillée et mise sur KOH)/triéthylamine (10 ml, fraîchement distillée)/ DMAP (quelques mg). On agite et on chauffe pendant une heure à 80 °C. On refroidit à 0 °C et on ajoute goutte à goutte 5g (18,3 mmol, 6 équivalents) de chlorure de kétoprofen racémique fraîchement préparé. La réaction est portée à 120 °C pendant 20h.
Après refroidissement le mélange réactionnel est versé dans 100 ml de méthanol sous agitation. Le précipité formé est récupéré par filtration, puis dissous dans 50 ml de dichlorométhane. La solution est filtrée et concentrée sous pression réduite. Le résidu est versé dans 100 ml de méthanol sous agitation et on récupère par filtration un polymère greffé de couleur sombre. Ces opérations de dissolution-précipitation sont effectuées une deuxième fois. Le produit obtenu est séché à l'étuve à vide pendant deux heures à 50 °C. On obtient 0,56g (rdt = 21 -% sur la base d'une trisubstitution) d'un polymère chiral kétoprofénate de cellulose avec un degré de substitution de 2,9 déterminé par analyse élémentaire.

### ANALYSES :

- IR(KBr) νₘₐₓ (cm⁻¹): 3500 (OH), 3000-2900 (-CH₃), 1760 (C=O ester), 1680 et 1300 (C=O cétone), 1610/1590/750 (C=C aromatiques), 1480 (δas -CH₃), 1170 et 1080 (C=O/C-O).
- ¹H-RMN (CDCl₃, 200 MHz) δ (ppm): 0,9-1,9 (m, α-CH₃), 2,3-5,3 (m, H cellulosiques + H benzyliques), 6,9-8,2 (massif, H aromatiques).
- Analyse Elémentaire :
   % calculé : C 74,48 %, H 5,28 % (pour une trisubstitution).
   % obtenu : C 74,38 %, H 5,30 %
- Pouvoir rotatoire : [α]²⁰ = -80,0° (λ = 436 nm, c = 1 g/dm³, CHCl₃).
La répartition des motifs acyles fixés R et S est respectivement de 49,5 % et 50,5 % (répartition déterminée par hydrolyse, cf. exemple 46).

### EXEMPLE 16 : SYNTHESE DU KETOPROFENATE D'AMYLOSE DANS LA PYRIDINE :

On opère comme dans l'exemple 14, mais en remplaçant la cellulose par de l'amylose B (Nacalaï Tesque Mw = 16-000) ; avec 0,199g de cellulose, 2,85g (8,5 équivalents) de chlorure de kétoprofen racémique, 40 ml de pyridine et 20h de chauffage à 90 °C, puis 44h à 115 °C. On obtient alors 0,416g (rdt = 42,9 % sur la base d'une trisubstitution) d'un polymère chiral kétoprofénate d'amylose avec un degré de substitution de 2,42 déterminé par analyse élémentaire.

### ANALYSES :

- IR(KBr) νₘₐₓ (cm⁻¹) : 3500 (OH), 3050-2950 (-CH₃), 1760 (C=O ester), 1680 et 1300 (C=O cétone), 1610/1590/770 (C=C aromatiques), 1470 (δas -CH₃), 1170 et 1080 (C=O/C-O).
- ¹H-RMN (CDCl₃, 200 MHz) δ (ppm) : 0,9-1,9 (m, α-CH₃), 2,3-5,3 (m, H cellulosiques + H benzyliques), 7-8,3 (massif, H aromatiques).
- Analyse Elémentaire :
   % calculé : C 74,48 %, H 5,28 % (pour une trisubstitution).
   % obtenu : C 73,28 %, H 5,33 %.
- Pouvoir rotatoire : [α]²⁰ = +90,0° (λ = 436 nm, c = 0,5 g/dm³, CHCl₃).
La répartition des motifs acyles fixés R et S est respectivement de 51 % et 49 % (répartition déterminée par hydrolyse, cf. exemple 47).

### EXEMPLE 17 : SYNTHESE DU MONO KETOPROFENATE DE CELLULOSE DANS LA PYRIDINE :

On opère comme dans l'exemple 7, mais en remplaçant l'acide α-phényl propionique par le kétoprofen et en effectuant la réaction à température ambiante ; avec 1g de cellulose, 2,4g (1,45 équivalents) de chlorure de kétoprofen racémique, 40 ml de pyridine, à 25 °C pendant 60 h. On obtient alors 2,09g (rdt = 85,2 -% sur la base d'une monosubstitution) d'un polymère chiral mono-kétoprofénate de cellulose avec un degré de substitution de 1 déterminé par analyse élémentaire.

### ANALYSES :

- IR(KBr) νₘₐₓ (cm⁻¹) : 3500-3200 (OH), 3000-2800 (-CH₃), 1760 (C=O ester), 1680 et 1290 (C=O cétone), 1610/1590/770 (C=C aromatiques), 1480 (δas -CH₃), 1170 et 1080 (C=O/C-O).
- ¹H-RMN (Pyridine d5, 200 MHz) δ (ppm) : 1-2 (m, α-CH₃), 2,3-5,3 (m, H cellulosiques + H benzylique + OH résiduels), 7-7,8 (massif, H aromatiques).
- Analyse Elémentaire :
   % calculé : C 66,33 %, H 5,53 % (pour une monosubstitution),
   % obtenu : C 66,30 %, H 5,35 %.
- Le pouvoir rotatoire n'a pas été mesuré à cause de la faible solubilité du polymère dans le CHCl₃.
La répartition des motifs acyles fixés R et S est respectivement de 55 % et 45 % (répartition déterminée par hydrolyse, cf. exemple 48).

### EXEMPLE 18 : SYNTHESE DE L'IBUPROFENATE DE CELLULOSE DANS LA PYRIDINE :

On opère comme dans l'exemple 1, mais en remplaçant l'acide α-phényl propionique par de l'ibuprofen ; avec 0,383g de cellulose, 5g (9,5 équivalents) de chlorure d'ibuprofen racémique, 40 ml de pyridine et un reflux à 115 °C. On obtient alors 1,207g (rdt = 70,3 -% sur la base d'une trisubstitution) d'un polymère chiral d'ibuprofénate de cellulose avec un degré de substitution de 2,73 déterminé par analyse élémentaire.

### ANALYSES :

- IR(KBr) νₘₐₓ (cm⁻¹) : 3500 (OH), 3000-2800 et 1420 (-CH₃,-CH₂), 1760 (C=O ester), 1500/820 (C=C aromatiques), 1480 (δas -CH₃), 1170 et 1090 (C=O/C-O).
- ¹H-RMN (CDCl₃, 200 MHz) δ (ppm) : 0,8 (m, (CH₃)₂-CH), 1,1-1,4 (m, α-CH₃), 1,9 (m, (CH₃)₂-CH), 2,4 (m, CH₂-CH), 3,1-5 (m, H cellulosiques + H benzyliques), 6,8-7,2 (m, H aromatiques).
- Analyse Elémentaire :
   % calculé : C 74,38 %, H 7,99 % (pour une trisubstitution).
   % obtenu : C 73,73 %, H 8,10 %
- Pouvoir rotatoire : [α]²⁰ = -81,0° (λ = 436 nm, c = 1 g/dm³, CHCl₃).
La répartition des motifs acyles fixés R et S est respectivement de 63 % et 37 % (répartition déterminée par hydrolyse, cf. exemple 49).

### EXEMPLE 18A : SYNTHESE DE L'IBUPROFENATE DE CELLULOSE DANS UN MELANGE PYRIDINE/TRIETHYLAMINE/DMAP (48 H DE REACTION, A 115 °C) :

On opère comme dans l'exemple 18. mais en présence d'un mélange de pyridine/triéthylamine/DMAP, la pyridine et la triéthylamine étant en proportion 2/1 (v/v) et la DMAP en quantité catalytique. On obtient ainsi un polymère chiral d'ibuprofénate de cellulose avec un rendement de 51% (sur la base d'une trisubstitution) et un degré de substitution de 2,77 déterminé par analyse élémentaire.
Les analyses IR et RMN sont identiques à celles décrites dans l'exemple 18.
- Analyse élémentaire :
   % calculé : C 74,38 %, H 7,99 % (pour une trisubstitution).
   % obtenu : C 73,83 %, H 8,02 %.
La répartition des motifs esters fixés R et S est respectivement de 47 % et 53 % (répartition déterminée par hydrolyse, cf. exemple 49A).

### EXEMPLE 19: SYNTHESE DE L'IBUPROFENATE DE β-CYCLODEXTRINE DANS LA PYRIDINE :

On opère comme dans l'exemple 10, mais en remplaçant l'acide *α*-(*p*-chlorophényl) propionique par de l'ibuprofen et la cellulose par de la de β-cyclodextrine; avec 0,5g de β-cyclodextrine, 4g (5,8 équivalents) de chlorure d'ibuprofen racémique, 40 ml de pyridine et un reflux à 120 °C pendant 18 h. On obtient alors 0,48g (rdt = 1,5 -% sur la base d'une trisubstitution) d'un polymère chiral d'ibuprofénate de β-cyclodextrine avec un degré de substitution de 2,9 déterminé par analyse élémentaire.

### ANALYSES :

- IR(KBr) νₘₐₓ (cm⁻¹) : 3000-2800 et 1390 (-CH₃,-CH₂), 1760 (C=O ester), 1530/820 (C=C aromatiques), 1480 (δas -CH₃), 1170 et 1090 (C=O/C-O).
- ¹H-RMN (CDCl₃, 200 MHz) δ (ppm) : 0,9 (m, (CH₃)₂-CH), 1,1-1,6 (m, α-CH₃), 1,9 (m, (CH₃)₂-CH), 2,4 (m, CH₂-CH), 2,9-5,5 (m, H cellulosiques + H benzyliques), 6,5-7,3 (m, H aromatiques).
- Analyse Elémentaire :
   % calculé : C 74,38 %, H 7,99 % (pour une trisubstitution).
   % obtenu : C 73,30 %, H 8,05 %.
- Pouvoir rotatoire : [α]²⁰ = +158.2° (λ = 436 nm, c = 3,3 g/dm³, CHCl₃).

### EXEMPLE 20: SYNTHESE DE L'α-(3,5-DIMETHYLPHENYL)-PROPIONATE DE CELLULOSE DANS LA PYRIDINE :

On opère comme dans l'exemple 14, mais en remplaçant le kétoprofen par l'acide 3,5-diméthylphényl propionique ; avec 0,25g de cellulose, 2,73g (9équivalents) de chlorure d'acide 3,5-diméthylphényl propionique racémique, 40 ml de pyridine, chauffage 24h à 90 °C puis 20h à 120 °C et agitation ultrasonique lors de la dissolution de la phase dans le dichlorométhane au moment de la purification. On obtient alors 0,447g (rdt = 45,1 -% sur la base d'une trisubstitution) d'un polymère chiral 3,5-diméthylphényl propionate de cellulose avec un degré de substitution de 1,74 déterminé par analyse élémentaire.

### ANALYSES :

- IR(KBr) νmax (cm-1) : 3500-3400 (OH), 3000-2900 (-CH3), 1760 (C=O ester), 1600/800 (C=C aromatiques trisubstitués symétriques), 1470 (δas -CH3), 1170 et 1090 (C=O/C-O).
- ¹H-RMN (Pyridine d5, 200 MHz) δ (ppm) : 1,1-2 (m, α-CH₃), 2,1-2,8 (m, 2*CH₃-Ar), 3-6 (m, H cellulosiques + H benzyliques), 6,8-7,5 (m, H aromatiques).
- Analyse Elémentaire :
   % calculé : C 72,9 %, H 7,17 % (pour une trisubstitution).
   % obtenu : C 68,5 %, H 6,63 %.
- Le pouvoir rotatoire n'a pas été mesuré à cause de la faible solubilité du polymère dans le CHCl₃.
La répartition des motifs acyles fixés R et S est respectivement de 58 % et 42 % (répartition déterminée par hydrolyse, cf. exemple 50).

### EXEMPLE 21: SYNTHESE DE L'α-(3,5-DIMETHYLPHENYL)-PROPIONATE DE CELLULOSE DANS LA PYRIDINE+DMAP :

On opère comme dans l'exemple 20, mais en ajoutant quelques mg de DMAP à la pyridine ; avec 0,21g de cellulose, 2,34g (9équivalents) de chlorure d'acide 3,5-diméthylphényl propionique racémique, 40 ml de pyridine +quelques mg de DMAP, chauffage 24h à 90 °C puis 44h à 120 °C. On obtient alors 0,613g (rdt = 73,7- % sur la base d'une trisubstitution) d'un polymère chiral 3,5-diméthylphényl propionate de cellulose avec un degré de substitution de 3 déterminé par analyse élémentaire.

### ANALYSES :

- IR(KBr) νₘₐₓ (cm⁻¹) : 3000-2800 (-CH₃), 1760 (C=O ester), 1610/810 (C=C aromatiques trisubstitués symétriques), 1470 (δas -CH₃), 1170 et 1090 (C=O/C-O).
- ¹H-RMN (CDCl₃, 200 MHz) δ (ppm) : 1-1,8 (m, α-CH₃), 2,1-2,8 (m, 2*CH₃-Ar), 2,7-5,4 (m, H cellulosiques + H benzyliques), 6,4-7,2 (m, H aromatiques).
- Analyse Elémentaire :
   % calculé : C 72,90 %, H 7,17 % (pour une trisubstitution).
   % obtenu : C 72,98 %, H 7,18 %
- Pouvoir rotatoire : [α]²⁰ = -87,0° (λ = 436 nm, c = 1,5 g/dm³, CHCl₃).
La répartition des motifs acyles fixés R et S est respectivement de 63 % et 37 % (répartition déterminée par hydrolyse, cf. exemple 51).

### EXEMPLE 22 : SYNTHESE DE L'α-(P-METHYLPHENYL)-PROPIONATE DE CELLULOSE DANS LA PYRIDINE :

On opère comme dans l'exemple 20, mais en remplaçant l'acide 3,5-diméthylphényl propionique par l'acide *p*-méthylphényl propionique ; avec 0,18g de cellulose, 1,82g (9équivalents) de chlorure d'acide *p*-méthylphényl propionique racémique, 30 ml de pyridine, chauffage 24h à 90 °C puis 45h à 120 °C. On obtient alors 0,491g (rdt = 74-% sur la base d'une trisubstitution) d'un polymère chiral *p*-méthylphényl propionate de cellulose avec un degré de substitution de 3 déterminé par analyse élémentaire.

### ANALYSES :

- IR(KBr) νmax (cm-1) : 3000-2800 (-CH3), 1760 C=O ester), 1550/800 (C=C aromatiques disubstitués), 1480 (δas -CH3), 1170 et 1090 (C=O/C-O).
- ¹H-RMN: (CDCl₃, 200 MHz) δ (ppm) = 0,9-1,6 (m, α-CH₃), 2-2,5 (m, CH₃-Ar), 2,7-5,4 (m, H cellulosiques + H benzyliques), 6,6-7,2 (m, H aromatiques).
- Analyse Elémentaire :
   % calculé : C 72,00 %, H 6,67 % (pour une trisubstitution).
   % obtenu : C 72,01 %, H 6,68 %
- Pouvoir rotatoire : [α]²⁰ = -110,0° (λ = 436 nm, c = 1,5 g/dm³, CHCl₃).
La répartition des motifs acyles fixés R et S est respectivement de 62 % et 38 % (répartition déterminée par hydrolyse, cf. exemple 52).

### EXEMPLE 23 : SYNTHESE DE L'α-(2-THIOPHENYL) PROPIONATE DE CELLULOSE DANS LA PYRIDINE :

Dans un ballon tricol de 100 ml, muni d'un réfrigérant surmonté d'une garde à chlorure de calcium, d'une ampoule à addition et d'un thermomètre on place 0,158g (1 mmol) de cellulose microcristalline Avicel (Merck 2331, M = [(162)n]g sur la base du monomère glucose), préalablement séchée pendant toute une nuit à 120 °C à l'étuve à vide, et 40 ml de pyridine, préalablement distillée et mise sur KOH. On agite et on chauffe pendant une heure à 80 °C. On laisse le mélange revenir à température ambiante et on ajoute goutte à goutte 1,53g (9 mmol, 9 équivalents) de chlorure d'acide 2-thiophényl propionique racémique fraîchement préparé. Lors de l'addition, le chlorure réagit très rapidement au contact des vapeurs même de pyridine. La réaction est laissée à température ambiante pendant 22h puis portée à 90 °C pendant 48 h.
Après refroidissement le mélange réactionnel est versé dans 100 ml de méthanol sous agitation. Le précipité formé est récupéré par filtration puis dissous sous agitation ultrosonique dans 50 ml de dichlorométhane. La solution est filtrée sur fritté 4 pour éliminer les particules sous forme solide et concentrée sous pression réduite. Le résidu est versé dans 100 ml de méthanol sous agitation et on récupère par filtration un polymère greffé de couleur sombre. Ces opérations de dissolution-précipitation sont effectuées une deuxième fois. Le produit obtenu est séché à l'étuve à vide pendant deux heures à 50 °C. On obtient 0,159g (rdt = 27,2- % sur la base d'une trisubstitution) d'un polymère chiral 2-thiophényl propionate de cellulose avec un degré de substitution de 1,98 déterminé par analyse élémentaire.

### ANALYSES :

- IR(KBr) νₘₐₓ (cm⁻¹) : 3500-3400 (OH), 3000-2900 (-CH₃), 1760 (C=O ester), 1460 (δas -CH₃), 1170 et 1090 (C=O/C-O), 700 (C-S).
- ¹H-RMN (CDCl₃, 200 MHz) δ (ppm) : 1-1,9 (m, α-CH₃), 3,2-5,5 (m, H cellulosiques + H benzyliques), 6,5-7,5 (massif, H hétérocycliques).
- Analyse Elémentaire :
   % calculé : C 56,25 %, H 4,86 % (pour une trisubstitution).
   % obtenu : C 54,73 %, H 5,03 %.
- Le polymère n'est pas assez soluble dans le CHCl₃ pour pouvoir mesurer un pouvoir rotatoire.
La répartition des motifs acyles fixés R et S est respectivement de 50 % et 50 % (répartition déterminée par hydrolyse, cf. exemple 53).

### EXEMPLE 24: SYNTHESE DE L'α-(2-THIOPHENYL) PROPIONATE DE CELLULOSE DANS LA PYRIDINE :

On opère comme dans l'exemple 23, mais en laissant la réaction à température ambiante ; avec 0,185g de cellulose, 2g (9équivalents) de chlorure d'acide 2-thiophényl propionique racémique, 30 ml de pyridine, 48h à température ambiante. On obtient alors 0,324g (rdt = 47,3 -% sur la base d'une trisubstitution) d'un polymère chiral 2-thiophényl propionate de cellulose avec un degré de substitution de 2,85 déterminé par analyse élémentaire.

### ANALYSES :

- IR(KBr) νₘₐₓ (cm⁻¹) : 3000-2900 (-CH₃), 1760 (C=O ester), 1470 (δas -CH₃), 1170 et 1090 (C=O/C-O), 700 (C-S).
- ¹H-RMN(CDCl₃, 200 MHz) δ (ppm) : 1-2 (m, 9H, α-CH₃), 2,7-5,3 (m, H cellulosiques + H benzyliques), 6,3-7,3 (massif, H hétérocycliques).
- Analyse Elémentaire :
   % calculé : C 56,25 %, H 4,86 % (pour une trisubstitution).
   % obtenu : C 56,09 %, H 4,87 %
- Pouvoir rotatoire : [α]²⁰ = -78,0° (λ = 436 nm, c = 0,5 g/dm³, CHCl₃).
La répartition des motifs acyles fixés R et S est respectivement de 54 % et 46 % (répartition déterminée par hydrolyse, cf. exemple 54).

EXEMPLE 25 : SYNTHESE D'UN ESTER MIXTE DE CELLULOSE D'ACIDE α-PHENYL PROPIONIQUE ET DE KETOPROFEN (AVEC PURIFICATION ET ANALYSES INTERMEDIAIRES) :

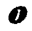 *SYNTHESE D'UN α-PHENYL PROPIONATE DE CELLULOSE DE DS=0,6 :*

On opère comme dans l'exemple 7, mais avec 1,2 équivalents de chlorure d'acide α-phényl propionique racémique au lieu de 1,8 ; avec 0,9g de cellulose, 1,1g (1,2 équivalents) de chlorure d'acide α-phényl propionique racémique, 40 ml de pyridine et chauffage 48h à 90 °C. On obtient alors 1,3g (rdt = 79,6 % sur la base d'une monosubstitution) d'un polymère chiral α-phényl propionate de cellulose avec un degré de substitution de 0,6 déterminé par analyse élémentaire.

### ANALYSES :

- IR(KBr) νₘₐₓ (cm⁻¹) : 3600-3200 (OH), 3000-2800 (-CH₃), 1760 (C=O), 1600/1500/700 (C=C aromatiques en para), 1450 (δas-CH₃), 1170 et 1090 (C=O/C-O).
- ¹H-RMN (Pyridine d5, 200 MHz) δ (ppm) : 1-2 (m, α-CH₃), 3-6 (m, H cellulosiques + H benzyliques + OH résiduels), 7-8 (m, H aromatiques).
- Analyse Elémentaire :
   % calculé : C 61,20 %, H 6,12 % (pour une disubstitution).
   % obtenu : C 56,81 %, H 5,84 %.
- Le pouvoir rotatoire n'a pas été mesuré à cause de la faible solubilité du polymère dans le CHCl₃.
La répartition des motifs acyles fixés R et S est respectivement de 55 % et 45 % pour les motifs acyles issus de l'acide α-phénylpropionique (répartition déterminée par hydrolyse, cf. exemple 55).
La répartition des motifs acyles fixés R et S est respectivement de 54 % et 46 % pour les motifs acyles issus du kétoprofen (répartition déterminée par hydrolyse, cf. exemple 55).

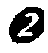 *SYNTHESE DE L'ESTER MIXTE PAR ADDITION DE 4 EQUIVALENTS DE CHLORURE DE KETOPROFEN A LA PHASE PRECEDENTE.*

Dans un ballon tricol de 100 ml, muni d'un réfrigérant surmonté d'une garde à chlorure de calcium, d'une ampoule à addition et d'un thermomètre on place 0,5g (2 mmol) de polymère chiral α-phényl propionate de cellulose de DS = 0,6 et 40 ml de pyridine, préalablement distillée et mise sur KOH. On agite et on chauffe pendant 20 min à 60 °C pour dissoudre la phase. On laisse le mélange revenir à température ambiante et on ajoute, goutte à goutte 2,2g (8 mmol, 4 équivalents) de chlorure de kétoprofen racémique fraîchement préparé. La réaction est portée à 105 °C pendant 48 h.

Après refroidissement le mélange réactionnel est versé dans 100 ml de méthanol sous agitation. Le précipité formé est récupéré par filtration puis dissous dans 30 ml de dichlorométhane. La solution est filtrée et concentrée sous pression réduite. Le résidu est versé dans 100 ml de méthanol sous agitation et on récupère par filtration un polymère greffé de couleur sombre. Ces opérations de dissolution-précipitation sont effectuées une deuxième fois. Le produit obtenu est séché à l'étuve à vide pendant deux heures à 50 °C. On obtient 1,227g (rdt =- 30 % sur la base d'un polymère de DS = 0,6 pour l'acide α-phényl propionique et de DS = 2 pour le kétoprofen) d'un polymère chiral mixte d'α-aryl propionate de cellulose, avec un degré de substitution de 0,6 pour l'acide α-phényl propionique et de 1,63 pour le kétoprofen déterminé par analyse élémentaire.

### ANALYSES :

- IR(KBr) νₘₐₓ (cm⁻¹) : 3550-3450 (OH), 3000-2800 (-CH₃), 1760 et 1300 (C=O ester), 1680 (C=O cétone), 1600/1590/720/690 (C=C aromatiques), 1450 (δas -CH₃), 1170 et 1090 (C=O/C-O).
- ¹H-RMN (CDCl₃, 200 MHz) δ (ppm) : 0,8-1,8 (m, α-CH₃), 2-5 (m, H cellulosiques + H benzyliques), 6,6-8 (massif, H aromatiques).
- Analyse Elémentaire :
   % calculé : C 73,63 %, H 5,48 % (DS = 0,6 pour l'acide α-phényl propionique et de DS = 2 pour le kétoprofen).
   % obtenu : C 71,79 %, H 5,85 % (DS = 0,6 pour l'acide α-phényl propionique et de DS = 1,63 pour le kétoprofen).
- Pouvoir rotatoire : [α]²⁰ = -73,0° (λ = 436 nm, c = 1 g/dm³, CHCl₃).

EXEMPLE 26 : SYNTHESE D'UN ESTER MIXTE DE CELLULOSE DE KETOPROFEN ET D'ACIDE α-PHENYL PROPIONIQUE (AVEC PURIFICATION ET ANALYSES INTERMEDIAIRES) :

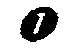 *SYNTHESE D'UN KETOPROFENATE DE CELLULOSE DE DS=1 :*

Voir exemple 17.

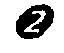 *SYNTHESE DE L'ESTER MIXTE PAR ADDITION DE 4 EQUIVALENTS DE CHLORURE D'ACIDE α-PHENYL PROPIONIQUE A LA PHASE PRECEDENTE :*

On opère comme dans la deuxième partie de l'exemple 25, mais en remplaçant l'α-phényl propionate de cellulose de DS = 0,6 par le kétoprofénate de cellulose de DS = 1 (de l'exemple 16) et l'addition de chlorure de kétoprofen par l'addition de 4 équivalents de chlorure d'acide α-phényl propionique ; avec 1,01g de polymère chiral kétoprofenate de cellulose de DS = 1, 1,9g (5 équivalents) de chlorure d'acide α-phényl propionique racémique, 30 ml de pyridine et chauffage 48h à 105 °C. On obtient 0,96g (rdt = 57 % sur la base d'un polymère de DS = 1 pour le kétoprofen et de DS = 2 pour l'acide α-phényl propionique) d'un polymère chiral mixte d'α-aryl propionate de cellulose avec un degré de substitution de 1 pour le kétoprofen et de 0,55 pour l'acide α-phényl propionique déterminé par analyse élémentaire.

### ANALYSES :

- IR(KBr) νₘₐₓ (cm⁻¹): 3500-3400 (OH), 3000-2800 (-CH₃), 1760 et 1300 (C=O ester), 1670 (C=O cétone), 1600/1500/730/710 (C=C aromatiques), 1450 (δas -CH₃), 1170 et 1090 (C=O/C-O).
- ¹H-RMN (CDCl₃, 200 MHz) δ (ppm) : 0,7-1,8 (m, α-CH₃), 2,5-5 (m, H cellulosiques + H benzyliques), 6,8-7,8 (massif, H aromatiques).
- Analyse Elémentaire :

- % calculé : C 72,52 %, H 5,74 % (DS = 1 pour le kétoprofen et de DS = 2 pour l'acide α-phényl propionique).
   % obtenu : C 68,61 %, H 6,01 % (DS = 1 pour le kétoprofen et de DS = 0,55 pour l'acide α-phényl propionique).
- Pouvoir rotatoire : [α]²⁰ = -80,0° (λ = 436 nm, c = 1 g/dm³, CHCl₃).

### EXEMPLE 27 : SYNTHESE DU NAPROXENATE DE CELLULOSE A PARTIR DU NAPROXEN S(+) (9 EQ.), DANS LE MELANGE PYRIDINE/TRIETHYLAMINE/DMAP :

Dans un ballon tricol de 100 ml, muni d'un réfrigérant surmonté d'une garde à chlorure de calcium et d'une ampoule à addition, sont introduits : 0,5g (3,09 mmole) de cellulose préalablement séchée toute une nuit sous vide à 120 °C ; 25 ml d'un mélange pyridine/triéthylamine (2/1) préalablement distillées et stockées respectivement sur KOH et tamis moléculaire 3Å ; ainsi qu'une quantité catalytique de 4-diméthylaminopyridine (DMAP).
La cellulose est mise à gonfler dans ce mélange à 80°C pendant une demi-heure. Une solution de 6,9g (9 éq.) de chlorure de naproxen S(+) dissous dans 50 rnl de CH₂Cl₂ (séché sur tamis moléculaire), est alors ajoutée à cette température au milieu réactionnel, pendant environ une heure. Le dichlorométhane est chassé par un courant d'azote. Le mélange est ensuite porté au reflux à 120 °C pendant 16h.
Le mélange préalablement refroidi est jeté sur 150 ml de MeOH. Un précipité beige se forme immédiatement. Ce dernier est filtré et lavé dans 150 ml de MeOH aux ultrasons pendant 15 min. Le précipité est à nouveau filtré sur büchner et séché : on récupère 1g (Rdt = 41 % sur la base d'une trisubstitution) d'ester cellulosique brut d'un DS = 2.7 déterminé par analyse élémentaire. Le solide est dissous dans 200 ml de CH₂Cl₂ ; le mélange obtenu est filtré sur coton pour donner une solution limpide. La solution de CH₂Cl₂ est concentrée puis versée goutte à goutte sur 50 ml de méthanol. Le précipité obtenu sous forme de poudre est filtrée sur membrane (0,45 micron) puis lavé aux ultrasons avec 20 ml d'un mélange iPrOH/Hexane (10/90). On filtre à nouveau sur membrane et on obtient, après séchage à 60 °C à l'étuve à vide, 650 mg (Rdt = 26,4 %) de naproxenate de cellulose d'un degré de substitution DS = 2,8 déterminé par analyse élémentaire.

### ANALYSES :

- IR (KBr νₘₐₓ(cm⁻¹): 2937 (δ CH), 1742 (δ C=O), 1607 et 1510 (δₐₛ et δ₅ C=C), 1268 (δₐₛ CH₃-O-Aryle), 1070-1153 (δ (C=O)-O).
- RMN ¹H (CDCl₃, 200 MHz) δ (ppm) : 0,4-1,8 (m, α-CH₃): 2,7-5,1 (m, O-CH₃ + H cellulosiques + H benzylique) ; 6,4-7,8 (m, H aromatiques).
- Analyse Elémentaire :
   % calculé : C 72,18 %, H 5,76 % pour une trisubstitution.
   % obtenu : C 71,48 %, H 5,57 % pour DS = 2,7.
   % obtenu : C 71,81 %, H 5,78 % pour DS = 2,8
- Pouvoir rotatoire : [α]²⁰ = -37,0° (λ = 436 nm, c = 1 g/dm³, CHCl₃).
La répartition des motifs acyles fixés R et S est respectivement de 43 % et 57 % (répartition déterminée par hydrolyse, cf. exemple 56).

### EXEMPLE 27A: SYNTHESE DU NAPROXENATE DE CELLULOSE A PARTIR DU NAPROXEN S(+) (1,5 EQUIVALENTS) DANS UN MELANGE PYRIDINE/TRIETHYLAMINE/DMAP (24 H DE REACTION, à 120 °C) :

On opère comme dans l'exemple 27, mais en présence de 1,5 équivalents (au lieu de 9) de chlorure de naproxen S(+) et pendant 24h. On obtient ainsi un polymère chiral de naproxénate de cellulose avec un rendement de 65% (sur la base d'une monosubstitution) et un DS=0,49 déterminé par analyse élémentaire.
Les analyses IR et RMN sont identiques à celles décrites dans l'exemple 18.
- Analyse élémentaire :
   % calculé : C 64,17 %, H 5,88 % pour une monosubstitution
   % obtenu : C 57,13 %, H 5,73 %
   La répartition des motifs esters fixés R et S est respectivement de 48 % et 52 % (répartition déterminée par hydrolyse, cf. exemple 56A).

### EXEMPLE 28 : SYNTHESE DU NAPROXENATE DE CELLULOSE A PARTIR DU NAPROXEN RACEMIQUE (6 EQ.), DANS LE MELANGE PYRIDINE/TRIETHYLAMINE/DMAP :

En opérant dans les mêmes conditions qu'avec le chlorure de naproxen S(+) (exemple 27), 1,0g (6,18 mmole) de cellulose est mis à réagir cette fois avec 9,32 g (6 éq.) de chlorure de naproxen S,R dissous dans 40 ml.
On récupère après le même traitement, 1g (Rdt = 50 % sur la base d'une trisubstitution) d'ester cellulosique brut d'un DS = 2,6 déterminé par analyse élémentaire.

### ANALYSES-:

- Les spectres IR et RMN sont identiques à ceux obtenus avec le chlorure de naproxen S(+).
- Analyse Elémentaire :
   % calculé : C 72,18 %, H 5,76 % pour une trisubstitution.
   % obtenu : C 71,43 %, H 5,80 %.
- Pouvoir rotatoire : [α]²⁰ = -30,0° (λ = 436 nm, c = 1 g/dm³, CHCl₃).
La répartition des motifs acyles fixés R et S est respectivement de 44 % et 56 % (répartition déterminée par hydrolyse, cf. exemple 57).

### EXEMPLE 28A : SYNTHESE DU NAPROXENATE DE CELLULOSE A PARTIR. DU NAPROXEN RACEMIQUE (1.5 EQUIVALENTS) DANS UN MELANGE PYRIDINE/TRIETHYLAMINE/DMAP (24 H DE REACTION, à 120 °C) :

On opère comme dans l'exemple 28, mais en présence de 1,5 équivalents (au lieu de 6) de chlorure de naproxen racémique. On obtient ainsi un polymère chiral de naproxénate de cellulose avec un rendement de 69 % (sur la base d'une monosubstitution) et un DS=0,49 déterminé par analyse élémentaire. Les analyses IR et RMN sont identiques à celles décrites dans l'exemple 27A.
- Analyse élémentaire :
   % calculé : C 64,17 %, H 5,88 % (pour une monosubstitution).
   % obtenu : C 58,19 %, H 5,78 %.
La répartition des motifs esters fixés R et S est respectivement de 44 % et 56 % (répartition déterminée par hydrolyse, cf. exemple 57A).

### EXEMPLE 29 : SYNTHESE DU NAPROXENATE DE CELLULOSE A PARTIR DU NAPROXEN RACEMIQUE (2,1 EQ.), DANS LE MELANGE PYRIDINE/DMAP :

La réaction est menée dans les mêmes conditions que dans l'exemple 27, mais cette fois sans triéthylamine, dans 30 ml de pyridine avec une quantité catalytique de DMAP. 1,0g (6,18 mmole) de cellulose est mis à réagir avec 3,22g (2,1 éq.) de chlorure de naproxen S,R dissous dans 20 ml. Le mélange est porté à 100 °C pendant 48 h.
Après le traitement habituel, on récupère 2,85g d'ester cellulosique de DS = 1,7 (Rdt-= 89 % sur la base d'une disubstitution) déterminé par analyse élémentaire.

### ANALYSES :

- IR (KBr) νₘₐₓ(cm⁻¹) : 3437 (δ O-H), 2937 (δ C-H), 1742 (δ C=-O), 1607 et 1506 (δₐₛ et δₛ C=C), 1268 (δₐₛ CH₃-O-Aryle), 1153-1070 (δ (C=O)-O).
- RMN ¹H (pyridine d5, 200 MHz) δ (ppm) : 1,2-2,4 (m, α-CH₃) ; 3,3-4,4(m, O-CH₃ + H cellulosiques + H benzyliques) ; 4,8-5,4 (m, H cellulosiques) : 6,5-8,5 (m, H aromatiques).
- Analyse Elémentaire :
- % calculé : C 69,62 %, H 5,63 % pour une disubstitution
   % obtenu : C 68,41 %, H 5,43 %.
- Le pouvoir rotatoire n'a pas été mesuré à cause de la faible solubilité du polymère dans le CHCl₃.
La répartition des motifs acyles fixés R et S est respectivement de 60 % et 40 % (répartition déterminée par hydrolyse, cf. exemple 58).

### EXEMPLE 29A : SYNTHESE DU NAPROXENATE DE CELLULOSE A PARTIR DU NAPROXEN RACEMIQUE (7 EQUIVALENTS) DANS UN MELANGE PYRIDINE/DMAP (24 H DE REACTION, à 120 °C) :

On opère comme dans l'exemple 29, mais en présence de 7 équivalents (au lieu de 2,1) de chlorure de naproxen racémique. On obtient ainsi un polymère chiral de naproxénate de cellulose avec un rendement de 83% (sur la base d'une trisubstitution) et un DS=3,00 déterminé par analyse élémentaire. Les analyses IR et RMN sont identiques à celles décrites dans l'exemple 27.
- Analyse élémentaire :
   % calculé : C 72,18 %, H 5,76 % (pour une trisubstitution).
   % obtenu : C 72,18 %, H 5,67 %.
La répartition des motifs esters fixés R et S est respectivement de 57 % et 43 % (répartition déterminée par hydrolyse, cf. exemple 58A).

### EXEMPLE 30 : SYNTHESE DU NAPROXENATE DE CELLULOSE A PARTIR DU NAPROXEN RACEMIQUE (1,5 EQ.), DANS LE MELANGE PYRIDINE/DMAP :

Dans les conditions décrites pour l'addition du chlorure de naproxen racémique (2,1 éq.) sur la cellulose (exemple 29), 0,75g (4,63 mmole) de cellulose sont mis à réagir avec 1,72g (1,5 éq.) de chlorure de naproxen S,R.
Après le traitement habituel, on récupère 1,59g d'ester cellulosique de DS_{moyen} = 0,71 (Rdt -=- 92 % sur la base d'une monosubstitution) déterminé par analyse élémentaire.

### ANALYSES :

- IR (KBr) νₘₐₓ(cm⁻¹) : 3344 (δ O-H), 2937 et 2904 (δ C-H), 1740 (δ C=O), 1607 et 1506 (δₐₛ et δₛ C=C), 1266 (δₐₛ CH₃-O-Aryle), 1162-1060 (δ (C=O)-O).
- RMN ¹H (pyridine d5, 200 MHz) δ (ppm) : 1,2-2,0 (m, α-CH₃) ; 3,3-4,4 (m, O-CH₃ + H cellulosique + H benzylique) ; 4,9-5,7 (m, H cellulosique) ; 6,9-8,3 (m, H aromatique).
- Analyse Elémentaire :
   % calculé : C 64,17 %, H 5,88 % pour une monosubstitution
   % obtenu : C 61,82 %, H 5,48 % pour un DS = 0,77
   % obtenu : C 60,30 %, H 5,30 % pour un DS = 0,65
- Le pouvoir rotatoire n'a pas été mesuré à cause de la faible solubilité du polymère dans le CHCl₃.
La répartition des motifs acyles fixés R et S est respectivement de 55 % et 45 % (répartition déterminée par hydrolyse, cf. exemple 59).

### EXEMPLE 31 : SYNTHESE DU NAPROXENATE DE CELLULOSE A PARTIR DU NAPROXEN S(+) (2,1 EQ.), DANS LE MELANGE PYRIDINE/DMAP :

Dans les conditions décrites dans l'exemple 29, 0,9g (5,6 mmole) de cellulose sont mis à réagir avec 2,82g (11,4 mmol, 2,1 éq.) de chlorure de naproxen S(+) additionné en solution dans le CH₂Cl₂ à 40 °C. La réaction est portée au reflux 48h à 90 °C.
Après le traitement habituel, on récupère 1,87g (Rdt = 57 % sur la base d'une disubstitution) d'ester cellulosique de DS = 1,5 déterminé par analyse élémentaire.

### ANALYSES :

- Le spectre IR est identique à celui obtenu avec le chlorure de naproxen S,R de l'exemple 29.
- RMN ¹H (pyridine d5, 200 MHz) δ (ppm) : 1,2-2,0 (m, α-CH₃) : 3,3-4,6 (m, O-CH₃ + H cellulosiques + H benzylique) ; 4,8-5,7 (m, H cellulosiques) : 6,8-8,3 (m, H aromatiques).
- Analyse Elémentaire :
   % calculé : C 69,62 %, H 5,63 % pour une disubstitution
   % obtenu : C 67,72 %, H 6,04 %.
- Le pouvoir rotatoire n'a pas été mesuré à cause de la faible solubilité du polymère dans le CHCl₃.
La répartition des motifs acyles fixés R et S est respectivement de 53 % et 47 % (répartition déterminée par hydrolyse, cf. exemple 60).

### EXEMPLE 32 : SYNTHESE DU NAPROXENATE DE CELLULOSE A PARTIR DU NAPROXEN S(+) (1 EQ.), DANS LE MELANGE PYRIDINE/DMAP :

Dans les conditions décrites dans l'exemple 30, 1g (6,2 mmole) de cellulose sont mis à réagir avec 1,53g (6,2 mmol, 1 éq.) de chlorure de naproxen S(+).
Après le traitement habituel, on récupère 1,75g (Rdt = 76 % sur la base d'une monosubstitution) d'ester cellulosique de DS- = 0,6 déterminé par analyse élémentaire.

### ANALYSES :

Les spectres IR et RMN sont identiques à ceux obtenus avec le chlorure de naproxen S,R de l'exemple 30.
- Analyse Elémentaire :
   % calculé : C 64,17 %, H 5,88 % pour une monosubstitution,
   % obtenu : C 59,43 %, H 6,10 %.
- Le pouvoir rotatoire n'a pas été mesuré à cause de la faible solubilité du polymère dans le CHCl₃.
La réparation des motifs acyles fixés R et S est respectivement de 53 % et 47 % (répartition déterminée par hydrolyse, cf. exemple 61).

### EXEMPLE 32A : SYNTHESE DU NAPROXENATE DE CELLULOSE A PARTIR DU NAPROXEN S(+) (7 EQUIVALENTS) DANS UN MELANGE PYRIDINE/DMAP (24 H DE REACTION, A 120 °C) :

On opère comme dans l'exemple 32, mais en présence de 7 équivalents (au lieu de 1) de chlorure de naproxen S(+) et pendant 24h. On obtient ainsi un polymère chiral de naproxénate de cellulose avec un rendement de 82 % (sur la base d'une trisubstitution) et un un DS=2,80 déterminé par analyse élémentaire. Les analyses IR et RMN sont identiques à celles décrites dans l'exemple 27.
- Analyse élémentaire :
   % calculé : C 72,18 %, H 5,76 % pour une trisubstitution
   % obtenu : C 71,84 %, H 5,75 %
La répartition des motifs esters fixés R et S est respectivement de 55 % et 45 % (répartition déterminée par hydrolyse, cf. exemple 61A).

### EXEMPLE 33 : SYNTHESE DU NAPROXENATE D'AMYLOSE A PARTIR DU NAPROXEN S(+) (6,2 EQ.), DANS LE MELANGE PYRIDINE/TRIETHYLAMINE/DMAP :

Dans les conditions décrites dans l'exemple 27, 0,2g (1,3 mmole) d'amylose sont mis à réagir avec 2,0g (8,0 mmol, 1 éq.) de chlorure de naproxen S(+).
Après le traitement habituel, on récupère 170 mg (Rdt = 16 % sur la base d'une trisubstitution) d'ester amylosique de DS = 2,3 déterminé par analyse élémentaire.

### ANALYSES :

- IR (KBr) νₘₐₓ(cm⁻¹) : 3442 (δ O-H), 2937 (δ C-H), 1737 (δ C=O), 1606 et 1506 (δₐₛ et δₛ C=
- C), 1266 (δₐₛ CH₃-O-Aryle), 1162-1032 (δ (C=O)-O).
- RMN ¹H (CDCl₃, 200 MHz) δ (ppm) : 0,3-2,0 (m, α-CH₃) ; 2,5-5,7 (m, O-CH₃ + H cellulosique + H benzylique) ; 6,4-8,0 (m, H aromatique).
- Analyse Elémentaire :
   % calculé : C 72,18 %, H 5,76 % pour une monosubstitution,
   % obtenu : C 70,32 %, H 6,49 %.
- Pouvoir rotatoire : [α]²⁰ = +29,0° (λ = 436 nm, c = 1 g/dm³, CHCl₃).

### EXEMPLE 34 : SYNTHESE DU NAPROXENATE DE β-CYCLODEXTRINE A PARTIR DU NAPROXEN RACEMIQUE (6 EQ.), DANS LA PYRIDINE :

Dans un ballon tricol de 100 ml, muni d'un réfrigérant surmonté d'une garde à chlorure de calcium et d'une ampoule à addition, sont introduits : 0,5g (3,09 mmole) de β-cyclodextrine préalablement séchée toute une nuit sous vide à 120 °C et 15 ml de pyridine préalablement distillée et séchée sur KOH. Le mélange est porté à 80°C, puis est refroidi. Une solution de 5,0g (6 éq.) de chlorure de naproxen racémique dissous dans 15 ml de CH₂Cl₂ (séché sur tamis moléculaire), est alors ajoutée à température ambiante au milieu réactionnel, pendant 5 min. Le mélange est ensuite porté à un léger reflux à 40 °C pendant 16h. Puis le dichlorométhane est chassé par un flux d'azote et le mélange réactionnel porté au reflux de la pyridine pendant 3h.
Le mélange préalablement refroidi est dilué avec 50 ml de CH₂Cl₂ et lavé avec une solution saturée de bicarbonate de sodium, puis avec une solution d'HCl 3M et enfin à l'eau. La phase organique est séchée sur MgSO₄ et concentrée. La solution obtenue est versée sur 30 ml de MeOH. Un précipité blanc d'ester de β-cyclodextrine se forme immédiatement. Ce dernier est filtré et séché, puis dissous dans 10 ml de CH₂Cl₂. A nouveau, la solution dans le CH₂Cl₂ est versée goutte à goutte sur 25 ml de MeOH sous agitation pour obtenir un précipité lavé de ses impuretés. Le dichlorométhane est alors évaporé et le précipité, une fois filtré et séché 4h sous vide à 25 °C, est obtenu sous forme d'une poudre très fine : 2,20g (Rdt = 89 % sur la base d'une trisubstitution), DS = 2,81 déterminé par analyse élémentaire.

### ANALYSES :

- IR (KBr) νₘₐₓ(cm⁻¹) : 1760 (δ C=O), 1510 (δₐₛ CH₃), 1170 et 1090 (δ (CO)-O).
- RMN ¹H (CDCl₃, 200 MHz) δ (ppm) : 0,8-1,7 (m, α-CH₃) ; 2,9-5,1 (m, O-CH₃ + H cyclodextrine + H benzyliques) ; 6,8-8,0 (m, H aromatiques).
- Analyse élémentaire :
   % calculé : C 72,18 %, H 5,76 % pour une trisubstitution
   % obtenu : C 71,81 %, H 6,02 %
- Pouvoir rotatoire : [α]²⁰=258,2° (λ=436 nm, c=2,8 g/dm³, CHCl₃).
   [α]²⁰=119,6° (λ=589 nm, c=2,8 g/dm³, CHCl₃).
La répartition des motifs esters fixés R et S est respectivement de 50 % et 50 % (répartition déterminée par hydrolyse, cf. exemple 62).

### C - ENRICHISSEMENT PAR HYDROLYSES DES PHASES

### EXEMPLE 35: HYDROLYSE DE L'α-PHENYL PROPIONATE DE CELLULOSE (DS=2,94, EXEMPLE 1):

Dans 70 ml de tétrahydrofurane (THF), on introduit 0,247g (0,45 mmol) d'α-phényl propionate de cellulose à température ambiante. On agite afin de dissoudre la phase puis on ajoute 10 ml d'eau distillée et 0,17g (0,4 mmol) de LiOH monohydrate (M = 42g).
On agite à température ambiante pendant 24h.
On ajoute 10 ml d'eau et on évapore le THF sous pression réduite. Le mélange est refroidi à 0 °C et 2 ml d'une solution de soude 2M sont ajoutés. On agite 30 min, puis on filtre pour récupérer la phase hydrolysée résiduelle. La phase aqueuse est lavée 2 fois avec 10 ml de dichlorométhane pour enlever les impuretés organiques. Après acidification avec une solution aqueuse d'acide chlorhydrique 3M, extraction avec 3 fois 20 ml d'éther, séchage au MgSO₄ et concentration à sec sous pression réduite, on obtient 0,198g (1,32 mmol) d'acide α-phényl propionique.
Le rendement de l'hydrolyse est quantitatif et l'acide récupéré est analysé par RMN.
¹H-RMN (CDCl₃, 200 MHz) δ (ppm) : 1,55 (d, 3H, α-CH₃), 3,75 (q, 1H, H benzyliques), 7,2-7,4 (m, 5H, H aromatiques), 10,2-10,9 (m, 1H, H acide).
La phase hydrolysée récupérée est analysée par IR solide (KBr) : son spectre est identique à celui de la cellulose de départ.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH: 98/1,95/0,05, 0,75 ml/min, λ = 230 nm. On obtient 2 pics à Rt = 12,27 min (62 %) et Rt = 14,55 min (38 %).
La littérature indique que le 1^{er} pic correspond à la forme R (-).

### EXEMPLE 35A : HYDROLYSE DE L'α-PHENYL PROPIONATE DE CELLULOSE (DS=2,97, EXEMPLE 1A):

On opère comme dans l'exemple 35. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 35. Les pourcentages des deux énantiomères R(-) et S(+) sont respectivement de 68 % et 32 %.

### EXEMPLE 35B : HYDROLYSE DE L'α-PHENYL PROPIONATE DE CELLULOSE (DS=2,96, EXEMPLE 1B) :

On opère comme dans l'exemple 35. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 35. Les pourcentages des deux énantiomères R(-) et S(+) sont respectivement de 47 % et 53 %.

### EXEMPLE 35C : HYDROLYSE DE L'α-PHENYL PROPIONATE DE CELLULOSE (DS=2,00, EXEMPLE 1C) :

On opère comme dans l'exemple 35. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 35. Les pourcentages des deux énantiomères R(-) et S(+) sont respectivement de 46 % et 54 %.

### EXEMPLE 35D : HYDROLYSE DE L'α-PHENYL PROPIONATE DE CELLULOSE (DS=1,87, EXEMPLE 1D) :

On opère comme dans l'exemple 35. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 35. Les pourcentages des deux énantiomères R(-) et S(+) sont respectivement de 43 % et 57 %.

### EXEMPLE 35E : HYDROLYSE DE L'α-PHENYL PROPIONATE DE CELLULOSE (DS=1,13, EXEMPLE 1E) :

On opère comme dans l'exemple 35. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomériquc de cet acide a été déterminée par HPLC chirale dans les mêmes conditions que dans l'exemple 35. Les pourcentages des deux énantiomères R(-) et S(+) sont respectivement de 54 % et 46 %.

### EXEMPLE 35F : HYDROLYSE DE L'α-PHENYL PROPIONATE D'AMYLOPECTINE (DS=2,92, EXEMPLE 1F) :

On opère comme dans l'exemple 35. Le rendement de l'hydrolyse est quantitatif et la structure de l'acide récupéré est confirmée par RMN. La phase hydrolysée récupérée est analysée par IR solide (KBr) : son spectre est identique à celui du glucidex de départ. La composition énantiomérique de cet acide a été déterminée par HPLC chirale dans les mêmes conditions que dans l'exemple 35. Les pourcentages des deux énantiomères R(-) et S(+) sont respectivement de 49 % et 51 %.

### EXEMPLE 36 : HYDROLYSE DE L'α-PHENYL PROPIONATE DE CELLULOSE (DS=2,5, EXEMPLE 3) :

On opère comme dans l'exemple 35, mais avec 72h d'agitation à température ambiante au lieu de 24h ; avec 0,098g de phase, 0,091g de LiOH monohydrate, 60 ml de THF. On obtient alors 0,081g (0,54 mmol) d'acide α-phényl propionique.
Le rendement de l'hydrolyse est quantitatif et l'acide récupéré est analysé par RMN.
¹H-RMN (CDCl₃, 200 MHz) δ (ppm): 1,55 (d, 3H, α-CH₃), 3,75 (q, 1H, H benzyliques), 7,2-7,4 (m, 5H, H aromatiques) 9,8-11,2 (m, 1H, H acide).
La phase hydrolysée récupérée est analysée par IR solide (KBr) : son spectre est identique à celui de la cellulose de départ.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 99/0,95/0,05, 1 ml/min, λ = 230 nm. On obtient 2 pics à Rt = 18,35 min (58,4 %) et Rt = 23,06 min (41,6 %).
La littérature indique que le 1^{er} pic correspond à la forme R (-).
La prédominence de l'énantiomère (-) est confirmée par le pouvoir rotatoire du mélange ainsi obtenu.

Pouvoir rotatoire : [α]²⁰ = -9,4° (λ = 589 nm, c = 16 g/dm³, CHCl₃).

### EXEMPLE 37: HYDROLYSE DE L'α-PHENYL PROPIONATE DE CELLULOSE (DS=2,5, EXEMPLE 4) :

On opère comme dans l'exemple 35, mais avec un temps d'agitation de 72h au lieu de 24h ; avec 0,12g de phase, 0,114g de LiOH monohydrate, 60 ml de THF. On obtient alors 0,085g (0,57 mmol) d'acide α-phényl propionique.
Le rendement de l'hydrolyse est quantitatif et l'acide récupéré est analysé par RMN :
¹H-RMN (CDCl₃, 200 MHz) δ (ppm): 1,55 (d, 3H, α-CH₃), 3,75 (q, 1H, H benzyliques), 7,27-7,32 (m, 5H, H aromatiques), 11-11,7 ( m, 1H, H acide).
La phase hydrolysée récupérée est analysée par IR solide (KBr) : son spectre est identique à celui de la cellulose de départ.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 99/0,95/0,05, 1 ml/min, λ = 230 nm. On obtient 2 pics à Rt = 18,10 min (58,3 %) et Rt = 22,47 min (41,7 %).
La littérature indique que le 1^{er} pic correspond à la forme R (-).
La prédominence de l'énantiomère (-) est confirmée par le pouvoir rotatoire du mélange ainsi obtenu.

Pouvoir rotatoire : [α]²⁰ = -9,0° (λ = 589 nm, c = 14 g/dm³, CHCl₃).

### EXEMPLE 38 : HYDROLYSE DE L'α-PHENYL PROPIONATE DE CELLULOSE (DS=1,99, EXEMPLE 6) :

On opère comme dans l'exemple 35, avec 0,21g de phase, 0,165g de LiOH monohydrate, 60 ml de THF et 24h d'hydrolyse. On obtient alors 0,04g (0,27 mmol) d'acide α-phényl propionique.
Le rendement de l'hydrolyse est de 27 % et l'acide récupéré est analysé par RMN.
¹H-RMN (CDCl₃, 200 MHz) δ (ppm): 1,55 (d, 3H, α-CH₃), 3,75 (q, 1H, H benzyliques), 7,27-7,32 (m, 5H, H aromatiques), 11-11,7 ( m, 1H, H acide).
La phase hydrolysée récupérée est analysée par IR solide (KBr) : son spectre est identique à celui de la cellulose de départ.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH: 98/1,95/0,05, 0,75 ml/min, λ = 230 nm. On obtient 2 pics à Rt = 12,31 min (59,6 %) et Rt = 14,59 min (40,4 %).
La littérature indique que le 1^{er} pic correspond à la forme R (-).

### EXEMPLE 39: HYDROLYSE DE L'α-PHENYL PROPIONATE DE CELLULOSE (DS=0,91, EXEMPLE 7) :

On opère comme dans l'exemple 35, avec 0,103g de phase, 0,13g de LiOH monohydrate, 50 ml de THF et 72h d'hydrolyse. On obtient alors 0,056 g (0,37 mmol) d'acide α-phényl propionique.
Le rendement de l'hydrolyse est quantitatif et l'acide récupéré est analysé par RMN.
¹H-RMN (CDCl₃, 200 MHz) δ (ppm) : 1,55 (d, 3H, α-CH₃), 3,75 (q, 1H, H benzylique), 7,27-7,32 (m, 5H, H aromatiques), 11-11,7 ( m, 1H, H acide).
La cellulose récupérée après hydrolyse est analysée par IR solide (KBr) : son spectre est identique à celui de la cellulose de départ.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 99/0,95/0,05, 1 ml/min, λ = 230 nm. On obtient 2 pics à Rt = 22,38 min (58 %) et Rt = 28,24 min (42 %).
La littérature indique que le 1^{er} pic correspond à la forme R (-).
La prédominence de l'énantiomère (-) est confirmée par le pouvoir rotatoire du mélange ainsi obtenu.

Pouvoir rotatoire :
- [α]²⁰ = -10,8° (λ = 589 nm, c = 3,6 g/dm³, CHCl₃).
- [α]²⁰ = -22,5° (λ = 436 nm, c = 3,6 g/dm³, CHCl₃).

### EXEMPLE 40 : HYDROLYSE DE L'α-PHENYL PROPIONATE D'AMYLOSE (DS=3,0, EXEMPLE 8) :

On opère comme dans l'exemple 35, en agitant le mélange réactionnel à température ambiante pendant 72h au lieu de 24h ; avec 0,103g de phase, 0,082g de LiOH monohydrate, 60 ml de THF. On obtient alors 0,077g (0,51 mmol) d'acide α-phényl propionique.
Le rendement de l'hydrolyse est quantitatif et l'acide récupéré est analysé par RMN.
¹H-RMN (CDCl₃, 200 MHz) δ (ppm) : 1,52 (d, 3H, α-CH₃), 3,7 (q, 1H, H benzyliques), 7,27-7,32 (m, 5H, H aromatiques), 11,1-11,5 ( m, 1H, H acide).
L'amylose est restée en solution et n'a pu être récupérée par filtration.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 99/0,95/0,05, 1 ml/min, λ = 230 nm. On obtient 2 pics à Rt = 18,23 min (53- %) et Rt = 22,79min (47 -%).
La littérature indique que le 1^{er} pic correspond à la forme R (-).
La prédominence de î'énantiomère (-) est confirmée par le pouvoir rotatoire du mélange ainsi obtenu.

Pouvoir rotatoire : [α]²⁰ = -4,0° (λ = 589 nm, c = 19 g/dm³, CHCl₃).

### EXEMPLE 41 : HYDROLYSE DE L'α-(P-CHLOROPHENYL) PROPIONATE DE CELLULOSE (DS=3, EXEMPLE 10):

En opérant comme dans l'exemple 35, avec 0,187g de phase, 0,18g de LiOH monohydrate, 60 ml de THF et 24h d'hydrolyse, on obtient 0,16g (0,87 mmol) d'acide α-(*p*-chlorophényl) propionique.
Le rendement de l'hydrolyse est quantitatif et l'acide récupéré est analysé par RMN.
¹H-RMN (CDCl₃, 200 MHz) δ (ppm) : 1,48 (d, 3H, α-CH₃), 3,7 (q, 1H, H benzyliques), 7,14-7,38 (m, 4H, H aromatiques), 10,8-11,2 ( m, 1H, H acide).
La phase hydrolysée récupérée est analysée par IR solide (KBr) : son spectre est identique à celui de la cellulose de départ.
La composition énantiomérique de cet acide a été déterminée par HPLC chirale sur une colonne (R-R)-Whelk-0-1 (Lichrocart 250*4 mm, 5µm, Merck) avec comme conditions d'analyse : hexane/iPrOH/CH₃COOH : 98/1,5/0,5, 0,8 ml/min, λ = 230 nm. On obtient 2 pics à Rt = 6,8 min (43,7 %) et Rt = 8,5 min (56,3 %).
Le signe du pouvoir rotatoire indique un enrichissement en énantiomère (-) qui correspond donc au 2^{e} pic.

Pouvoir rotatoire : [α]²⁰ = -8,0° (λ = 589 nm, c = 14 g/dm³, CHCl₃).

### EXEMPLE 42 : DERACEMISATION, PUIS HYDROLYSE DE L'α-(P-CHLOROPHENYL) PROPIONATE DE CELLULOSE (DS=3, EXEMPLE 10) A L'AIDE DE DIISOPROPYLAMIDURE DE LITHIUM :

Dans 50 ml de tétrahydrofurane (THF), on introduit 0,3g (0,45 mmol) d'α-(*p*-chlorophényl) propionate de cellulose à température ambiante. On agite afin de dissoudre la phase et on refroidit à -10°C. On ajoute, sous agitation et toujours à -10°C, 1,6 ml (3,3 mmol) d'une solution 2M de LDA goutte à goutte. On agite à -10°C pendant 2h, puis on additionne 0,5 ml (30 mmol) d'eau distillée. On laisse le mélange revenir à température ambiante et on agite à nouveau pendant 12h à température ambiante. On ajoute 20 ml d'eau et on évapore le THF sous pression réduite. Le mélange est refroidi à 0 °C et 1 ml d'une solution de soude 2M est ajouté. On agite 30 min, puis on filtre pour récupérer la phase hydrolysée résiduelle.
La phase aqueuse est lavée 2 fois avec 10 ml de dichlorométhane pour enlever les impuretés organiques. Après acidification avec une solution aqueuse d'acide chlorhydrique 3M, extraction avec 3 fois 20 ml d'éther, séchage au MgSO₄ et concentration à sec sous pression réduite, on obtient 0,241g (1,31 mmol) d'acide α-(*p*-chlorophényl) propionique.
Le rendement de l'hydrolyse est quantitatif et l'acide récupéré est analysé par RMN.
¹H-RMN (CDCl₃, 200 MHz) δ (ppm) : 1,5 (d, 3H, α-CH₃), 3,7 (q, 1H, H benzyliques), 7,25-7,3 (m, 4H, H aromatiques), 10-11,5 ( m, 1H, H acide).
La composition énantiomérique de cet acide a été déterminée par HPLC chirale sur une colonne (R-R)-Whelk-0-1 (Lichrocart 250*4 mm, 5µm, Merck) avec comme conditions d'analyse- : hexane/iPrOH/CH₃COOH : 98/1,5/0,5, 0,8 ml/min, λ = 230 nm. On obtient 2 pics à Rt = 7,92 min (50,6 %) et Rt = 8,9 min (49,4 %).
Le signe du pouvoir rotatoire indique un enrichissement en énantiomère (+) qui correspond donc au 1^{er} pic.
Pouvoir rotatoire : [α]²⁰ = 0,5° (λ = 589 nm, c = 2 g/dm³, CHCl₃).

### EXEMPLE 43 : DERACEMISATION , PUIS HYDROLYSE DE L'α-(P-CHLOROPHENYL) PROPIONATE DE CELLULOSE (DS=3, EXEMPLE 10) A L'AIDE DE TRIETHYLAMINE :

Dans 50 ml de dichlorométhane, on introduit 0,2g (0,3 mmol) d'α-(*p*-chlorophényl) propionate de cellulose à température ambiante et 1g (0,01 mole) de triéthylamine. On scelle le flacon et on le place dans un bain thermostaté à 30°C pendant 14 jours. On lave le mélange avec une solution d'acide chlorhydrique 3M puis à l'eau. La phase organique est séchée sur MgSO₄ puis évaporée à sec sous pression réduite. On récupère la phase déracémisée que l'on hydrolyse à l'aide d'un mélange hydroxyde de lithium/eau, suivant le mode opératoire d'hydrolyse précédent. On récupère 0,161g (0,9 mmol) d'acide α-(*p*-chlorophényl) propionique.
Le rendement de l'hydrolyse est quantitatif et la structure de l'acide récupéré est confirmé par ¹H-RMN.
La composition énantiomérique de cet acide a été déterminée par HPLC chirale sur une colonne (R-R)-Whelk-0-1 (Lichrocart 250*4 mm, 5µm, Merck) avec comme conditions d'analyse : hexane/iPrOH/CH₃COOH : 98/1,5/0,5, 0,8 ml/min, λ = 230 nm. On obtient 2 pics à Rt = 7,68 min (49,7 %) et Rt = 8,57 min (50,3 %).

### EXEMPLE 44 : HYDROLYSE DE L'α-(P-CHLOROPHENYL) PROPIONATE D'AMYLOSE (DS=3, EXEMPLE 13) :

On opère comme dans l'exemple 35, avec 0,109g de phase, 0,074g de LiOH monohydrate, 60 ml de THF et 72h d'hydrolyse. On obtient alors 0,1g (0,54 mmol) d'acide α-(*p*-chlorophényl) propionique.
Le rendement de l'hydrolyse est quantitatif et l'acide récupéré est analysé par RMN.
¹H-RMN (CDCl₃, 200 MHz) δ (ppm) : 1,48 (d, 3H, α-CH₃), 3,7 (q, 1H, H benzyliques), 7,21-7,32 (m, 4H, H aromatiques), 8,2-9,2 ( m, 1H, H acide).
L'amylose est restée en solution et n'a pu être récupérée par filtration.
La composition énantiomérique de cet acide a été déterminée par HPLC chirale sur une colonne (R-R)-Whelk-0-1 (Lichrocart 250*4 mm, 5µm, Merck) avec comme conditions d'analyse : hexane/iPrOH/CH₃COOH : 98/1,5/0,5, 1 m/min, λ = 230 nm. On obtient 2 pics à Rt = 6,28 min (47,7 %) et Rt = 7,24 min (52,3 %).
Le signe du pouvoir rotatoire indique un enrichissement en énantiomère (-) qui correspond donc au 2^{e} pic.

Pouvoir rotatoire : [α]²⁰ = -2,0° (λ = 589 nm, c = 19 g/dm³, CHCl₃).

### EXEMPLE 45 : HYDROLYSE DU KETOPROFENATE DE CELLULOSE (DS=2,35, EXEMPLE 14) :

On opère comme dans l'exemple 35, avec 0,113g de phase, 0,071g de LiOH monohydrate, 60 ml de THF et 72h d'hydrolyse. On obtient alors 0,078g (0,31 mmol) de kétoprofen.
Le rendement de l'hydrolyse est de 83 -% et l'acide récupéré est analysé par RMN.
¹H-RMN (CDCl₃, 200 MHz) δ (ppm) : 1,52 (d, 3H, α-CH₃), 3,8 (q, 1H, H benzyliques), 7,23-7,8 (m, 9H, H aromatiques), 10,2-10,6 ( m, 1H, H acide).
La phase hydrolysée récupérée est analysée par IR solide (KBr) : son spectre est identique à celui de la cellulose de départ avec une très légère bande C-=-O à 1-760cm⁻¹ et 1680 cm⁻¹.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 98/1,95/0,05, 1 ml/min, λ = 230 nm. On obtient 2 pics à Rt = 29,22 min (58,3 %) et Rt = 31,84 min (41,7 %).
La littérature indique que le 1^{er} pic correspond à la forme R(-).
La prédominence de l'énantiomère (-) est confirmée par le pouvoir rotatoire du mélange ainsi obtenu.

Pouvoir rotatoire :
- [α]²⁰ = -4,5° (λ = 589 nm, c = 4,4 g/dm³, CHCl₃).
- [α]²⁰ = -9,3° (λ = 436 nm, c = 4,4 g/dm³, CHCl₃).

### EXEMPLE 46: HYDROLYSE DU KETOPROFENATE DE CELLULOSE (DS=2,9, EXEMPLE 15) :

En opérant comme dans l'exemple 35, avec 0,099g de phase, 0,051g de LiOH monohydrate, 50 ml de THF et 48h d'hydrolyse, on obtient 0,084g (0,33 mmol) de kétoprofen.
Le rendement de l'hydrolyse est de 97 -% et l'acide récupéré est analysé par RMN.
¹H-RMN (CDCl₃, 200 MHz) δ (ppm): 1,52 (d, 3H, α-CH₃), 3,8 (q, 1H, H benzyliques), 7,23-7,8 (m, 9H, H aromatiques), 10,2-10,6 ( m, 1H, H acide).
La phase hydrolysée récupérée est analysée par IR solide (KBr) : son spectre identique à celui de la cellulose initiale présente une légère bande C-=-O à 1760 cm⁻¹ et 1680 cm⁻¹.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 99/0,95/0,05, 1 ml/min, λ = 230 nm. On obtient 2 pics à Rt = 63,79 min (49,5 %) et Rt = 70,86 min (50,5 %).
La littérature indique que le 2^{e} pic correspond à la forme S(+).
La prédominence de l'énantiomère (+) est confirmée par le pouvoir rotatoire du mélange ainsi obtenu.

Pouvoir rotatoire :
- [α]²⁰ = 0,2° (λ = 589 nm, c = 5,2 g/dm³, CHCl₃).
- [α]²⁰ = 0,4° (λ = 436 nm, c = 5,2 g/dm³, CHCl₃).

### EXEMPLE 47 : HYDROLYSE DU KETOPROFENATE D'AMYLOSE (DS=2,42, EXEMPLE 16) :

En opérant comme dans l'exemple 35, avec 0,1063g de phase, 0,068g de LiOH monohydrate, 50 ml de THF et 72h d'hydrolyse, on obtient 0,085g (0,34 mmol) de kétoprofen.
Le rendement de l'hydrolyse est quantitatif et l'acide récupéré est analysé par RMN.
¹H-RMN (CDCl₃, 200 MHz) δ (ppm) : 1,54 (d, 3H, α-CH₃), 3,8 (q, 1H, H benzyliques), 7,23-8 (m, 9H, H aromatiques), 8,2-8,8 ( m, 1H, H acide).
L'amylose est restée en solution et n'a pu être récupérée par filtration.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 99/0,95/0,05, 1 ml/min, λ = 230 nm. On obtient 2 pics à Rt = 62,46 min (50,7 %) et Rt = 69,12 min (49,3 %).
La littérature indique que le 1^{er} pic correspond à la forme R(-).
La prédominence de l'énantiomère (-) est confirmée par le pouvoir rotatoire du mélange ainsi obtenu.

Pouvoir rotatoire :
- [α]²⁰ = -0,9° (λ = 589 nm, c = 3,4 g/dm³, CHCl₃).
- [α]²⁰ = -0,9° (λ = 436 nm, c = 3,4 g/dm³, CHCl₃).

### EXEMPLE 48: HYDROLYSE DU MONO-KETOPROFENATE DE CELLULOSE (DS=1, EXEMPLE 17) :

En opérant comme dans l'exemple 35, avec 0,202g de phase, 0,13g de LiOH monohydrate, 50 ml de THF et 24h d'hydrolyse, on obtient 0,169g (0,67 mmol) de kétoprofen.
Le rendement de l'hydrolyse est de 71 -% et l'acide récupéré est analysé par RMN.
¹H-RMN (CDCl₃, 200 MHz) δ (ppm) : 1,5 (d, 3H, α-CH₃), 3,7 (q, 1H, H benzylique), 7,23-8 (m, 9H, H aromatiques), 9,2-10 ( m, 1H, H acide).
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 98/1,95/0,05, 0,8 ml/min, λ = 230 nm. On obtient 2 pics à Rt = 30,43 min (55,3 %) et Rt = 32,75 min (44,7 %).
La littérature indique que le 1^{er} pic correspond à la forme R(-).
La prédominence de l'énantiomère (-) est confirmée par le pouvoir rotatoire du mélange ainsi obtenu.

Pouvoir rotatoire : [α]²⁰ = -3,3° (λ = 436 nm, c = 2g/dm³, CHCl₃).

### EXEMPLE 49 : HYDROLYSE DE L'IBUPROFENATE DE CELLULOSE (DS=2,73, EXEMPLE 18) :

En opérant comme dans l'exemple 35, avec 0,102g de phase, 0,61g de LiOH monohydrate, 50 ml de THF et 72h d'hydrolyse, on obtient 0,035g (0,17 mmol) de kétoprofen.
Le rendement de l'hydrolyse est de 50 -% et l'acide récupéré est analysé par RMN.
¹H-RMN (CDCl₃, 200 MHz) δ (ppm) : 1,2 (d, 6H, (CH₃)₂-CH), 1,4 (d, 3H, α-CH₃), 1,8 (sept, 1H, (CH₃)₂-CH), 2,4 (d, 2H, CH₂-CH₃), 3,7 (q, 1H, H benzyliques), 7-7,25 (m, 4H, H aromatiques), 9 -10 ( m, 1H, H acide).
La phase hydrolysée récupérée est analysée par IR solide (KBr) : on observe la présence d'une forte bande OH mais également de la bande C-=-O à 1760 cm⁻¹ qui confirme que l'hydrolyse n'a été que partielle.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 99/0,95/0,05, 1 ml/min, λ = 230 nm. On obtient 2 pics à Rt = 11,66 min (63,3 %) et Rt = 13,99 min (36,7 %).
La littérature indique que le 1^{er} pic correspond à la forme R(-).
La prédominence de l'énantiomère (-) est confirmée par le pouvoir rotatoire du mélange ainsi obtenu.

Pouvoir rotatoire : [α]²⁰ = -10,1° (λ = 589 nm, c = 7 g/dm³, CHCl₃).

### EXEMPLE 49A : HYDROLYSE DE L' IBUPROFENATE DE CELLULOSE (DS=2,77, EXEMPLE 18A) :

On opère comme dans l'exemple 49 mais pendant 10 jours. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 49. Les pourcentages des deux énantiomères R(-) et S(+) sont respectivement de 48 % et 52 %.

### EXEMPLE 50: HYDROLYSE DE L'α -(3,5-DIMETHYLPHENYL) PROPIONATE DE CELLULOSE (DS = 1,74, EXEMPLE 20):

En opérant comme dans l'exemple 35, avec 0,148g de phase, 0,101g de LiOH monohydrate, 60 ml de THF et 72h d'hydrolyse, on obtient 0,117g (0,66 mmol) d'acide 3,5-diméthylphényl propionique.
Le rendement de l'hydrolyse est quantitatif et l'acide récupéré est analysé par RMN.
¹H-RMN (CDCl₃, 200 MHz) δ (ppm) : 1,48 (d, 3H, α-CH₃), 2,29 (s, 6H, CH₃-Ar), 3,67 (q, 1H, H benzyliques), 6,92 (s, 3H, H aromatiques), 9,5 -11 ( m, 1H, H acide).
La phase hydrolysée récupérée est analysée par IR solide (KBr) : son spectre est identique à celui de la cellulose de départ.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 99/0,95/0,05, 1 ml/min, λ = 230 nm. On obtient 2 pics à Rt = 16,79 min (58 %) et Rt = 28,27 min (42 %).
Le signe du pouvoir rotatoire indique un enrichissement en énantiomère (-) qui est donc le 1^{er} pic.

Pouvoir rotatoire :
- [α]²⁰ = -8,0° (λ = 589 nm, c = 4 g/dm³, CHCl₃).
- [α]²⁰ = -17.0° (λ = 436 nm, c = 4 g/dm³, CHCl₃).

### EXEMPLE 51 : HYDROLYSE DE L'α-(3,5-DIMETHYLPHENYL) PROPIONATE DE CELLULOSE (DS=3, EXEMPLE 21) :

En opérant comme dans l'exemple 35, avec 0,124g de phase, 0,84g de LiOH monohydrate, 60 ml de THF et 72h d'hydrolyse, on obtient 0,091g (0,51 mmol) d'acide 3,5-diméthylphényl propionique.
Le rendement de l'hydrolyse est de 90- % et l'acide récupéré est analysé par RMN.
¹H-RMN (CDCl₃, 200 MHz) δ (ppm) : 1,5 (d, 3H, α-CH₃), 2,29 (s, 6H, CH₃-Ar, 3,7 (q, 1H, H benzyliques), 6,92 (s, 3H, H aromatiques), 9,5 -11 ( m, 1H, H acide).
La phase hydrolysée récupérée est analysée par IR solide (KBr): son spectre est identique à celui la cellulose de départ avec une très légère bande C-=-O à 1760 cm⁻¹.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 98/1,95/0,05, 0,8 ml/min, λ = 230 nm. On obtient 2 pics à Rt = 10,11 min (63 %) et Rt = 13,84 min (37 %).
Le signe du pouvoir rotatoire indique un enrichissement en énantiomère (-) qui correspond donc au 1^{er} pic.

Pouvoir rotatoire :
- [α]²⁰ = -15,7° (λ = 589 nm, c = 22 g/dm³, CHCl₃).
- [α]²⁰ = -33,1° (λ = 436 nm, c = 22 g/dm³, CHCl₃).

### EXEMPLE 52 : HYDROLYSE DE L'α-(P-METHYLPHENYL) PROPIONATE DE CELLULOSE (DS=3, EXEMPLE 22) :

En opérant comme dans l'exemple 35 avec 0,103g de phase, 0,74g de LiOH monohydrate, 60 ml de THF et 72h d'hydrolyse, on obtient 0,077g (0,47 mmol) d'acide *p*-méthylphényl propionique.
Le rendement de l'hydrolyse est quantitatif et l'acide récupéré est analysé par RMN.
¹H-RMN (CDCl₃, 200 MHz) δ (ppm) : 1,4 (d, 3H, α-CH₃), 2,25 (s, 3H, CH₃-Ar, 3,6 (q, 1H, H benzyliques), 7,03-7,16 (s, 4H, H aromatiques), 9,5-10,5 ( m, 1H, H acide).
La phase hydrolysée récupérée est analysée par IR solide (KBr): son spectre est identique à celui de la cellulose de départ.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 98/1,95/0,05, 0,8 ml/min, λ = 230 nm. On obtient 2 pics à Rt = 12,02 min (62,4 %) et Rt = 13,53 min (37,6 %).
Le signe du pouvoir rotatoire indique un enrichissement en énantiomère (-) qui correspond donc au 1^{er} pic.

Pouvoir rotatoire :
- [α]²⁰ = -15,6° (λ = 589 nm, c = 21 g/dm³, CHCl₃).
- [α]²⁰ = -18,6° (λ = 436 nm, c = 21 g/dm³, CHCl₃).

### EXEMPLE 53 : HYDROLYSE DE L'α-(2-THIOPHENYL) PROPIONATE DE CELLULOSE (DS=1,98, EXEMPLE 23) :

En opérant comme dans l'exemple 35, avec 0,115g de phase, 0,111g de LiOH monohydrate, 50 ml de THF et 72h d'hydrolyse, on obtient 0,069g (0,45 mmol) d'acide 2-thiophényl propionique.
Le rendement de l'hydrolyse est quantitatif et l'acide récupéré est analysé par RMN.
¹H-RMN (CDCl₃, 200 MHz) δ (ppm) : 1,51 (d, 3H, α-CH₃), 3,95 (q, 1H, H benzyliques), 6,9 (m, 2H, H aromatiques), 7,15 (m, 1H, H aromatiques), 9,5 -9,8 ( m, 1H, H acide).
La phase hydrolysée récupérée est analysée par IR solide (KBr) : son spectre est identique à celui de la cellulose de départ.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH : 99/0,95/0,05, 1 ml/min, λ = 230 nm. On obtient 2 pics à Rt = 22,66 min (50,1 %) et Rt = 30,72 min (49,9 %).
La mesure du pouvoir rotatoire confirme que le mélange est racémique.

### EXEMPLE 54: HYDROLYSE DE L'α-(2-THIOPHENYL) PROPIONATE DE CELLULOSE (DS=2,85, EXEMPLE 24) :

En opérant comme dans l'exemple 35, avec 0,106g de phase, 0,083g de LiOH monohydrate, 50 ml de THF et 72h d'hydrolyse, on obtient 0,081g (0,52 mmol) d'acide 2-thiophényl propionique.
Le rendement de l'hydrolyse est quantitatif et l'acide récupéré est analysé par RMN.
¹H-RMN (CDCl₃, 200 MHz) δ (ppm) : 1.6 (d, 3H, α-CH₃), 4 (q, 1H, H benzyliques), 6,95 (m, 2H, H aromatiques), 7,2 (m, 1H, H aromatiques), 10,7 -11,3 ( m, 1H, H acide).
La phase hydrolysée récupérée est analysée par IR solide (KBr): son spectre est identique à celui de la cellulose de départ.
La composition énantiomérique de cet acide a été déterminée par HPLC chirale sur une colonne Chiralcel OD-H (250*4 mm, 5µm, DAICEL japon) avec comme conditions d'analyse: hexane/iPrOH/CF₃COOH: 99/95/0,05, 1 ml/min, λ = 230 nm. On obtient 2 pics à Rt = 21,63 min (53,4 %) et Rt = 29,54 min (46,6 %).
Le signe du pouvoir rotatoire indique un enrichissement en énantiomère (-) qui correspond donc au 1^{er} pic.

Pouvoir rotatoire ; [α]²⁰ = -1,7° (λ = 589nm, c = 17 g/dm³, CHCl₃).

### EXEMPLE 55 : HYDROLYSE DE LA PHASE MIXTE DE CELLULOSE DECRITE DANS L'EXEMPLE 25 (ACIDE α-PHENYL PROPIONIQUE DS=0,6 + KETOPROFEN DS=1,63) :

En opérant comme dans l'exemple 35, avec 0,389g de phase, 0,18g de LiOH monohydrate, 60 ml de THF et 48h d'hydrolyse, on obtient 0,31g d'acide α-phényl propionique et de kétoprofen.
Le rendement de l'hydrolyse en ces deux acides est quantitatif.
La phase hydrolysée récupérée est analysée par IR solide (KBr) : son spectre est identique à celui de la cellulose de départ.
Les compositions énantiomériques de ces deux acides ont été déterminées par HPLC chirale sur une colonne Chiralcel OD-H (250*4 mm, 5µm, DAICEL japon) avec comme conditions d'analyse : hexane/iPrOH/CF₃COOH : 98/1,95/0,05, 0,8 ml/min, λ = 230 nm. On obtient 4 pics : 2 pics correspondant à l'acide α phényl propionique à Rt = 12,58 min (55 %, R(-)) et Rt = 14,75 min (45 %, S(+)), 2 pics correspondant au kétoprofen à Rt = 30,27 min (53,7 %, R(-)) et Rt = 32,56 min (46,3 %, S(+)).

### EXEMPLE 56 : HYDROLYSE DU NAPROXENATE DE CELLULOSE (OBTENU A PARTIR DU NAPROXEN S(+), DS=2,7) DECRIT DANS L'EXEMPLE 27 :

Dans un ballon monocol de 250 ml sont introduits : 110 mg (0,15 mmol) d'ester à hydrolyser, 70 ml de THF, 10 ml d'eau et 63 mg (10 éq.) d'hydroxyde de lithium monohydrate. Le mélange est agité à température ambiante pendant 24h. Puis, le THF est évaporé à température ambiante et 10 ml d'eau sont ajoutés au résidu d'évaporation. Ce mélange est traité avec 2 ml de NaOH 2M, puis il est filtré sur coton pour éliminer la cellulose non réagie. La solution aqueuse obtenue est extraite au CH₂Cl₂ afin d'extraire les impuretés organiques et l'ester non réagi, puis elle est acidifiée jusqu'à pH = 1 avec de l'HCl 3M. La phase aqueuse est extraite à l'éther diéthylique. La phase éthérée est séchée sur MgSO₄ et le solvant est évaporé. On obtient ainsi 70 mg de naproxen (Rdt = 75 %). La composition énantiomérique de l'acide récupéré est déterminée par HPLC chirale, sur une colonne (R-R)-Whelk-0-1 (Lichrocart 250*4 mm, 5µm, Merck) dans les conditions d'analyse suivantes : hexane/iPrOH/CH₃COOH : 80/19,5/0,5 ; d = 1 ml/min ; λ1 = 230 nm. Deux pics sont obtenus à des temps Rt = 6,98 min (57 %) et Rt = 11,43 min (43 %).
La littérature indique que le 1^{er} pic correspond à la forme S(+).
L'injection de l'énantiomère S(+) seul et dans des conditions identiques confirme qu'il est le premier élué des deux énantiomères.

### EXEMPLE 56A : HYDROLYSE DU NAPROXENATE DE CELLULOSE (DS=0,49, EXEMPLE 27A) :

On opère comme dans l'exemple 56. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ.
La composition énantiomérique de cet acide a été déterminée par HPLC chirale dans les mêmes conditions que dans l'exemple 56. Les pourcentages des deux énantiomères R(-) et S(+) sont respectivement de 48 % et 52 %.

### EXEMPLE 57 : HYDROLYSE DU NAPROXENATE DE CELLULOSE (OBTENU A PARTIR DU NAPROXEN RACEMIQUE, DS=2,6) DECRIT DANS L'EXEMPLE 28 :

Dans les mêmes conditions (voir exemple 56) que pour l'hydrolyse de l'ester de naproxen S(+) (DS = 2,7, exemple 26), 300 mg (0,38 mmol) d'ester et 138 mg (8,6 éq.) d'hydroxyde de lithium sont mis à réagir. Après traitement, on obtient 250 mg de naproxen (Rdt = 98 %). La composition énantiomérique de l'acide récupéré est déterminée par HPLC chirale, sur une colonne (R-R)-Whelk-0-1 (Lichrocart 250*4 mm, 5µm, Merck) dans les conditions d'analyse suivantes : hexane/EtOH/CH₃COOH : 80/19,5/0,5 ; d = 1 ml/min ; λ -= 230 nm, Deux pics sont obtenus à des temps Rt = 5,66 min (56 %) et Rt = 8,29 min (44 %) respectivement pour les énantiomères S(+) et R(-).

### EXEMPLE 57A : HYDROLYSE DU NAPROXENATE DE CELLULOSE (DS=0,49, EXEMPLE 28A) :

On opère comme dans l'exemple 56. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 56. Les pourcentages des deux énantiomères R(-) et S(+) sont respectivement de 44 % et 56 %.

### EXEMPLE 58 : HYDROLYSE DU NAPROXENATE DE CELLULOSE (OBTENU A PARTIR DU NAPROXEN RACEMIQUE, DS=1,7) DECRIT DANS L'EXEMPLE -29:

L'hydrolyse est menée suivant la méthode habituelle (exemple 56) avec 150 mg (0,29 mmol) d'ester (DS = 1,7) et 68 mg (5,6 éq.) de LiOH. Après traitement, on obtient 110 mg de naproxen (Rdt = 98 %). La composition énantiomérique de l'acide récupéré est déterminée par HPLC chirale, sur une colonne (R-R)-Whelk-0-1 (Lichrocart 250*4 mm, 5µm, Merck) dans les conditions d'analyse suivantes : hexane/EtOH/CH₃COOH : 80/19,5/0,5 ; d = 1 ml/min ; λ = 230 nm. Deux pics sont obtenus à des temps Rt = 7,42 min (40 %) et Rt = 11,91 min (60 %) respectivement pour les énantiomères S(+) et R(-).

### EXEMPLE 58A : HYDROLYSE DU NAPROXENATE DE CELLULOSE (DS=3,00, EXEMPLE 29A) :

On opère comme dans l'exemple 56. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 56. Les pourcentages des deux énantiomères R(-) et S(+) sont respectivement de 57 % et 43 %.

### EXEMPLE 59 : HYDROLYSE DU NAPROXENATE DE CELLULOSE (OBTENU A PARTIR DU NAPROXEN RACEMIQUE, DS=0,71) DECRIT DANS L'EXEMPLE-30:

L'hydrolyse est menée suivant la méthode habituelle (exemple 56) avec 150 mg (0,48 mmol) d'ester (DS = 0,71) et 54 mg (2,7 éq.) de LiOH. Après traitement, on obtient 60 mg de naproxen (Rdt = 76 %). La composition énantiomérique de l'acide récupéré est déterminée par HPLC chirale, sur une colonne (R-R)-Whelk-0-1 (Lichrocart 250*4 mm, 5µm, Merck) dans les conditions d'analyse suivantes : hexane/EtOH/CH₃COOH : 80/19,5/0,5 ; d = 1 ml/min ; λ = 230 nm. Deux pics sont obtenus à des temps Rt = 7,34 min (45 %) et Rt = 11,74 min (55 %) respectivement pour les énantiomères S(+) et R(-).

### EXEMPLE 60: HYDROLYSE DU NAPROXENATE DE CELLULOSE (OBTENU A PARTIR DU NAPROXEN S(+), DS=1,5) DECRIT DANS L'EXEMPLE 31 :

L'hydrolyse est menée suivant la méthode habituelle (exemple 56) avec 293 mg (0,61 mmol) d'ester (DS = 1,5) et 170 mg (7 éq.) de LiOH. Après traitement, on obtient 180 mg de naproxen (Rdt = 86 %). La composition énantiomérique de l'acide récupéré est déterminée par HPLC chirale, sur une colonne (R-R)-Whelk-0-1 (Lichrocart 250*4 mm, 5µm, Merck) dans les conditions d'analyse suivantes : hexane/EtOH/CH₃COOH : 80/19,5/0,5 ; d = 1 ml/min ; λ = 230 nm. Deux pics sont obtenus à des temps Rt = 8,44 min (47 %) et Rt = 14,18 min (53 %) respectivement pour les énantiomères S(+) et R(-).

### EXEMPLE 61 : HYDROLYSE DU NAPROXENATE DE CELLULOSE (OBTENU A PARTIR DU NAPROXEN S(+), DS=0,6) DECRIT DANS L'EXEMPLE 32 :

L'hydrolyse est menée suivant la méthode habituelle (exemple 56) avec 330 mg (1,14 mmol) d'ester (DS = 0,6) et 48 mg (11,4 mmol, 10 éq.) de LiOH. Après traitement, on obtient 150 mg de naproxen (Rdt = 93 %). La composition énantiomérique de l'acide récupéré est déterminée par HPLC chirale, sur une colonne (R-R)-Whelk-0―1 (Lichrocart 250*4 mm, 5µm, Merck) dans les conditions d'analyse suivantes : hexane/iPrOH/CH₃COOH: 80/19,5/0,5 ; d = 1 ml/min ; λ = 230 nm. Deux pics sont obtenus à des temps Rt = 6,94 min (47 %) et Rt = 10,46 min (53 %) respectivement pour les énantiomères S(+) et R(-).

### EXEMPLE 61A : HYDROLYSE DU NAPROXENATE DE CELLULOSE (DS=2,80, EXEMPLE 32A) :

On opère comme dans l'exemple 56. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 56. Les pourcentages des deux énantiomères R(-) et S(+) sont respectivement de 55 % et 45 %.

### EXEMPLE 62 : HYDROLYSE DU NAPROXENATE DE β-CYCLODEXTRINE : (DS=2,81, EXEMPLE 34) :

On opère comme dans l'exemple 56. Le rendement de l'hydrolyse est quantitatif et la structure de l'acide récupéré est confirmée par RMN. La phase hydrolysée récupérée est analysée par IR solide (KBr) : son spectre est identique à celui de la β-cyclodextrine de départ. La composition énantiomérique de cet acide a été déterminée par HPLC chirale dans les mêmes conditions que dans l'exemple 56. Les pourcentages des deux énantiomères R(-) et S(+) sont respectivement de 50% et 50%.

### EXEMPLE 63: DERACEMISATION DE L'α-PHENYL PROPIONATE DE CELLULOSE (DS=2,96 DE L'EXEMPLE 1B) DANS LA PYRIDINE A 25 °C

Dans un pillulier, on place 50 mg d' α-phényl propionate de cellulose de DS=2,96 obtenu au cours de la réaction décrite dans l'exemple 1B, et 2,7 ml de pyridine. Le mélange est agité à température ambiante pendant 8 jours. Le solvant est évaporé, puis une hydrolyse est réalisée dans les conditions décrites dans l'exemple 35. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 35. Les pourcentages des deux énantiomères R(-) et S(+) sont respectivement de 46% et 54%.

### EXEMPLE 64: DERACEMISATION DE L'α-PHENYL PROPIONATE DE CELLULOSE (DS=2,96 DE L'EXEMPLE 1B) DANS LA PYRIDINE A 90 °C

On opère comme dans l'exemple 63 à la différence que le mélange est cette fois agité pendant 24h à 90 °C. Le traitement et l'hydrolyse sont réalisés comme dans l'exemple 63. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 35. Les pourcentages des deux énantiomères R(-) et S(+) sont respectivement de 49% et 51%.

### EXEMPLE 65 : DERACEMISATION DE L'α-PHENYL PROPIONATE DE CELLULOSE (DS=2,94 DE L'EXEMPLE 1) DANS UN MELANGE PYRIDINE/TRIETHYLAMINE/DMAP A 25 °C.

Dans un pillulier, on place 50 mg d' α-phényl propionate de cellulose de DS=2,94 obtenu au cours de la réaction décrite dans l'exemple 1, et 2,7 ml d'un mélange pyridine/triéthylamine dans une proportion 2/1 (v/v) et une quantité catalytique de DMAP. Le mélange est agité à température ambiante pendant 8 jours. Le traitement et l'hydrolyse sont réalisés comme dans l'exemple 63. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 35. Les pourcentages des deux énantiomères R(-) et S(+) sont respectivement de 61% et 39%.

### EXEMPLE 66: DERACEMISATION DE L'α-PHENYL PROPIONATE DE CELLULOSE (DS=2,94 DE L'EXEMPLE 1) DANS UN MELANGE PYRIDINE/TRRTHYLAMINE/DMAP A 90 °C.

On opère comme dans l'exemple 65 à la différence que le mélange est cette fois agité pendant 24h à 90 °C. Le traitement et l'hydrolyse sont réalisés comme dans l'exemple 63. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 35. Les pourcentages des deux énantiomères R(-) et S(+) sont respectivement de 56% et 44%.

### EXEMPLE 67: DERACEMISATION DE L'α-PHENYL PROPIONATE DE CELLULOSE (DS=2,94 DE L'EXEMPLE 1) DANS UN MELANGE PYRIDINE/DABCO/DMAP A 90 °C.

On opère comme dans l'exemple 66 à la différencc que le mélange est cette fois constitué de 1 ml de pyridine, 0,5g de DABCO (1,4-diazabicyclo[2,2,2]octane) et une quantité catalytique de DMAP. Après réaction, le mélange est jeté dans 10 ml de méthanol. L'ester de cellulose ainsi précipité est filtré et séché. L'hydrolyse de cet ester est réalisée comme dans l'exemple 63. Le rendement de l'hydrolyse est quantitatif et la structure de l'acide récupéré est confirmée par RMN. La phase hydrolysée récupérée est analysée par IR solide (KBr) : son spectre est identique à celui de la cellulose de départ. La composition énantiomérique de cet acide a été déterminée par HPLC chirale dans les mêmes conditions que dans l'exemple 35. Les pourcentages des deux énantiomères R(-) et S(+) sont respectivement de 46% et 54%.

### D - EVALUATION CHROMATOGRAPHIQUE DES ESTERS SYNTHETISES ET UTILISES A TITRE DE PHASES STATIONNAIRES CHIRALES

### MODE OPÉRATOIRE ET PRINCIPE :

4 mg de phase stationnaire à tester et 100 µl d'une solution du racémique test, de concentration comprise entre 0,5 et 1 mg/ml (suivant la solubilité du composé), sont introduits dans un pillulier puis gardés pendant 2h à 10°C afin de permettre l'adsorption du composé test sur la phase stationnaire. On prélève à froid 25 µl de surnageant par décantation ou filtration que l'on dilue (concentration spécifique à chaque racémique) à l'aide de l'éluant d'analyse adapté au racémate considéré. On effectue la même opération de dilution à partir de 25 µl de la solution initiale du racémique. Les solutions sont injectées en HPLC chirale dans des conditions d'analyses propre à chaque racémate. L'analyse chromatographique du surnageant permet, par comparaison au racémique, de déterminer l'enrichissement (E), l'adsorption du composé sur la phase chirale (ads) et l'énantiosélectivité (a) de la phase vis à vis du composé considéré.

### DESCRIPTION DES TABLEAUX :

TABLEAU 1 : Evaluation chromatographique des esters de cellulose et d'amylose par la méthode "microbatch" dans un éluant hexane/2-propanol 99/1. La Benzoïne, la Base de Tröger et l'Alcool de Pirkle ont été injectés sur la CHIRALPAK AS dans l'hexane/2-propanol : 90/10, le Trans Stilbène Oxyde a été injecté sur la CHIRALCEL OD-H dans l'hexane/2-propanol : 90/10.
Les définitions des termes utilisés dans ce tableau sont les suivantes :
⇒ ads : adsorption = (1- [(Atrac × A^{1,2}) ö (Arac^{1,2} × At )]) × 100
⇒ E : enrichissement = différence des concentrations des deux énantiomères du composé tests après adsorption sur la phase.
⇒ α : énantiosélectivité = ([(Arac² × At)/ (Atrac × A²)]- 1)ö([(Arac¹ × At)ö(Atrac × A¹)]-1)
Atrac : Aire du 1,3,5-tris t-butyl benzène (TTB) dans le racémique. Le TTB sert à mesurer le volume mort de la colonne.
Arac¹: Aire, dans le racémique, du pic correspondant à l'énantiomère le moins adsorbé sur la phase à tester.
Arac²: Aire, dans le racémique, du pic correspondant à l'énantiomère le plus adsorbé sur la phase à tester.
At : Aire du 1.3,5 tris t-butyl benzène (TTB) dans la solution surnageante.
¹: Aire de l'énantiomère le moins adsorbé sur la phase à tester.
A²: Aire de l'énantiomère le plus adsorbé sur la phase à tester.

TABLEAU 2 : Ordre d'élution, configuration et énantiosélectivité des 4 composés tests sur les phases commerciales de DAICEL: Cellulose tris-(4-méthyl benzoate) (CHIRALCEL OJ), Cellulose tris-(3,5-diméthyl phénylcarbamate) (CHIRALCEL OD) Amylose tris-[(S)-alpha-phénéthyl-carbamate] (CHIRALPAK AS) dans un éluant hexane/2-propanol : 90/10.
Les définitions des termes utilisés dans ce tableau sont les suivantes :
α : énantiosélectivité = (facteur de rétention de l'énantiomère le plus adsorbé) ö (facteur de rétention de l'énantiomère le moins adsorbé)

### COMMENTAIRES ET RESULTATS :

Les esters de cellulose ou de polysaccharide de la présente invention montrent de fortes adsorptions pour la benzoïne et l'alcool de Pirkle et des adsorptions moyennes pour la base de Tröger et le trans stilbène oxyde.
La comparaison entre les phases testées et les phases commerciales (tableau 2) met en évidence, pour certains racémates, des énantiosélectivités (α) comparables entre les deux séries de phases avec des adsorptions non négligeables et des inversions d'ordre d'élution. A titre d'exemple non limitatif, la comparaison de l'adsorption de ces racémates sur les phases testées et sur les phases commerciales CHIRALCEL OJ, CHIRALCEL OD et CHIRALPAK AS montre des inversions d'ordre d'élution : en effet l'énantiomère de la benzoïne majoritairement adsorbé sur les phases stationnaires testées a la configuration S(+) comme dans le cas de la CHIRALCEL OJ, mais l'ordre est inversé pour la CHIRALCEL OD et la CHIRALPAK AS. Dans le cas de l'alcool de Pirkle on constate une inversion de l'ordre d'élution entre les phases testées et les trois phases commerciales. Dans le cas du trans stilbène oxyde, bien que les adsorptions soient moins importantes que dans les deux cas précédents, on remarque un ordre identique pour la CHIRALCEL OD mais inverse par rapport aux deux autres phases commerciales. Ces inversions d'ordre d'élution sont très utiles dans les cas où il est souhaitable d'avoir un énantiomère, en particulier, qui soit élué en premier.
Certaines de ces phases présentent, également, des énantiosélectivités comparables aux phases commerciales avec des adsorptions non négligeables comme l'α-phényl-propionate de cellulose DS = 2,5 (exemple 3 tableau 1) qui présente un α = 1,3 pour la benzoïne identique à celui de la CHIRALCEL OJ dans les mêmes conditions d'analyses. On peut également citer la phase 3,5-diméthylphényl-propionate de cellulose DS = 3 (exemple 21 tableau 1) ou la phase p-méthylphényl-propionate de cellulose DS = 3 (exemple 22 tableau 1) qui présentent respectivement des α de 1,25 et 1,27 pour le trans stilbène oxyde (α = 1,17 pour la CHIRALCEL OJ et α = 1,28 pour la CHIRALPAK AS), la phase p-méthylphényl propionate de cellulose DS = 3 (exemple 22 tableau 1) est également bien résolutive pour la base de Tröger avec un α de 1,3 (α = 1,32 pour la CHIRALCEL OD). A titre d'exemple non limitatif, cette dernière phase présente donc une énantiosélectivité vis à vis du trans stilbène oxyde meilleure que la CHIRALCEL OJ avec une inversion d'ordre d'élution par rapport à cette phase commerciale.

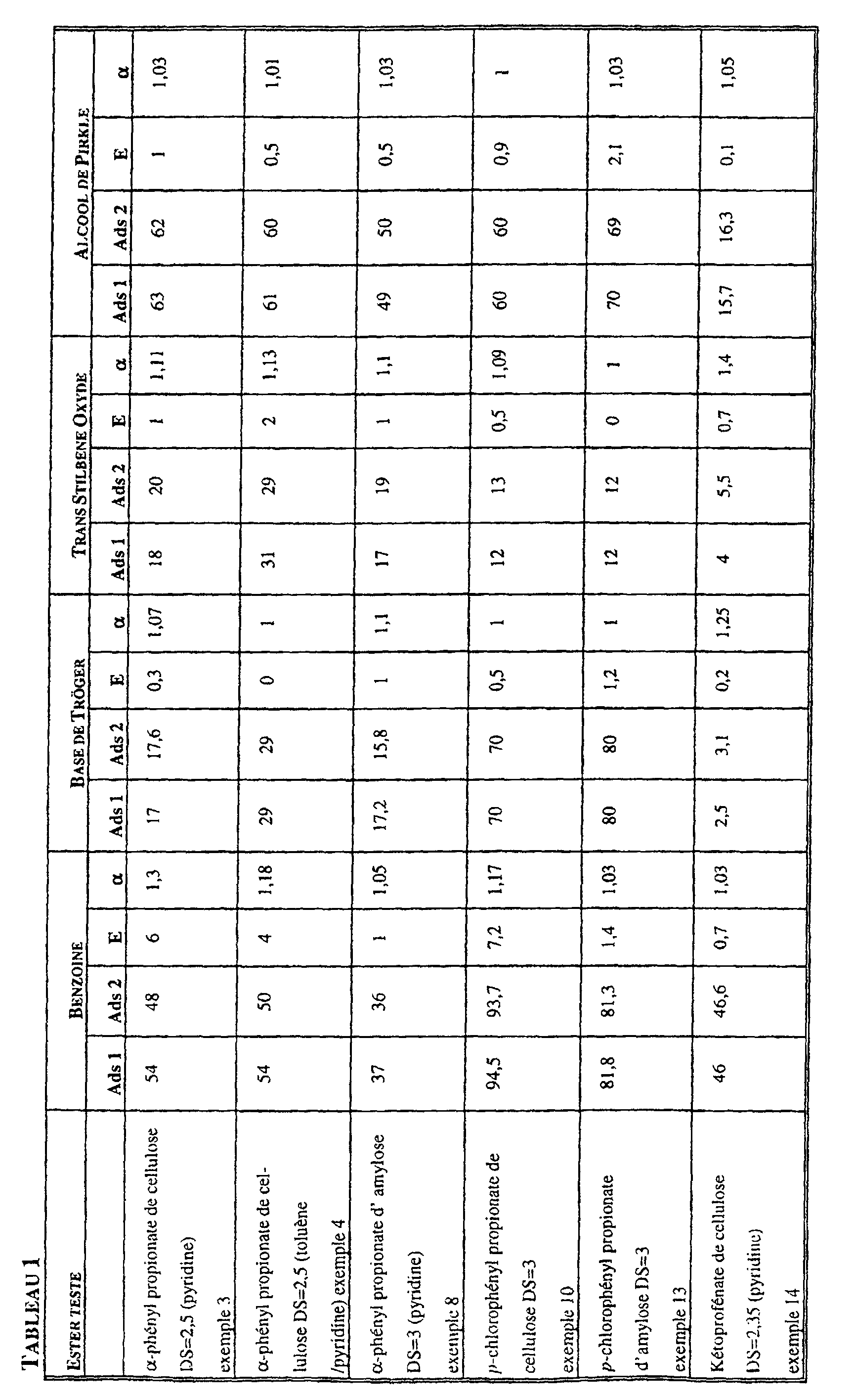

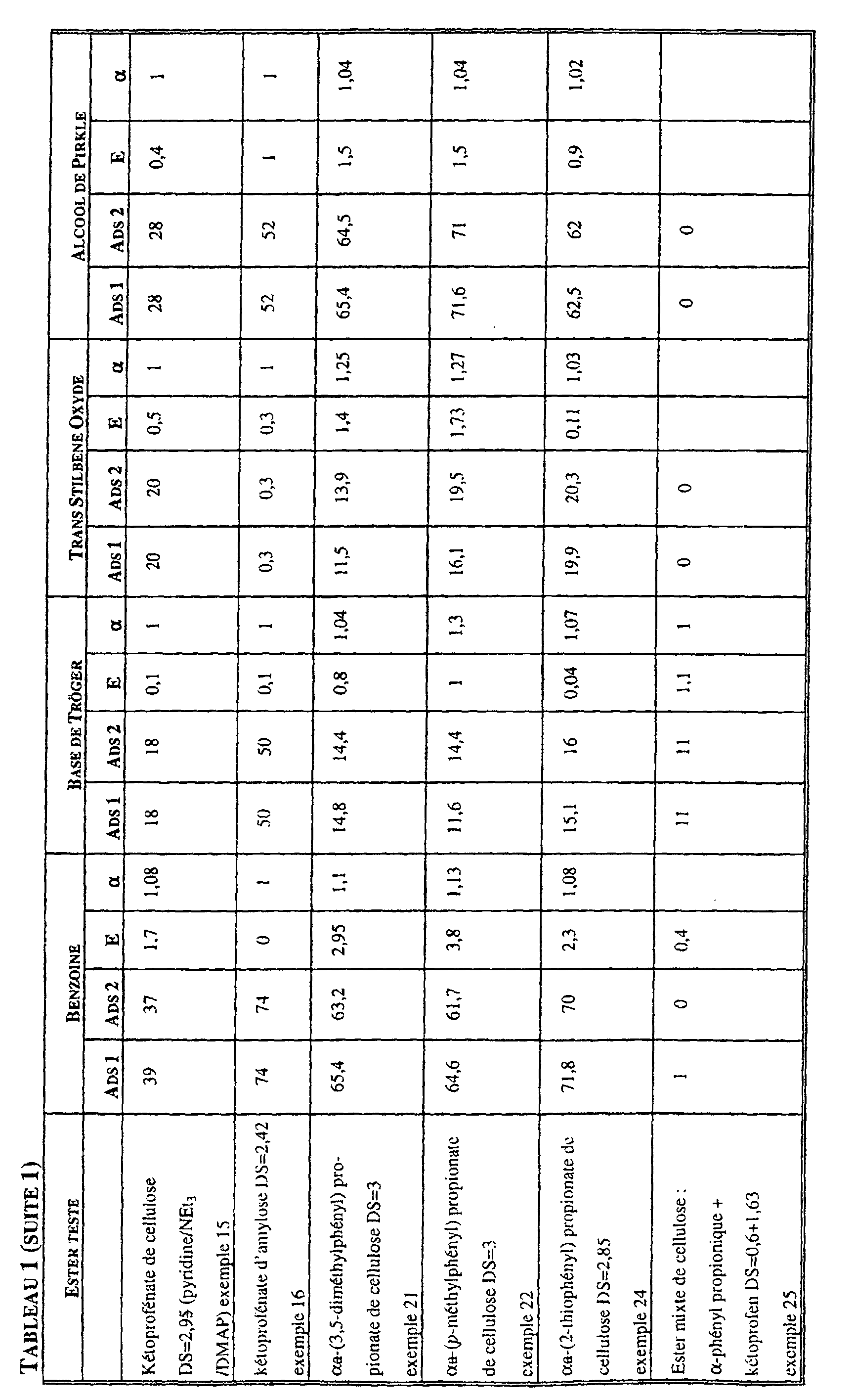

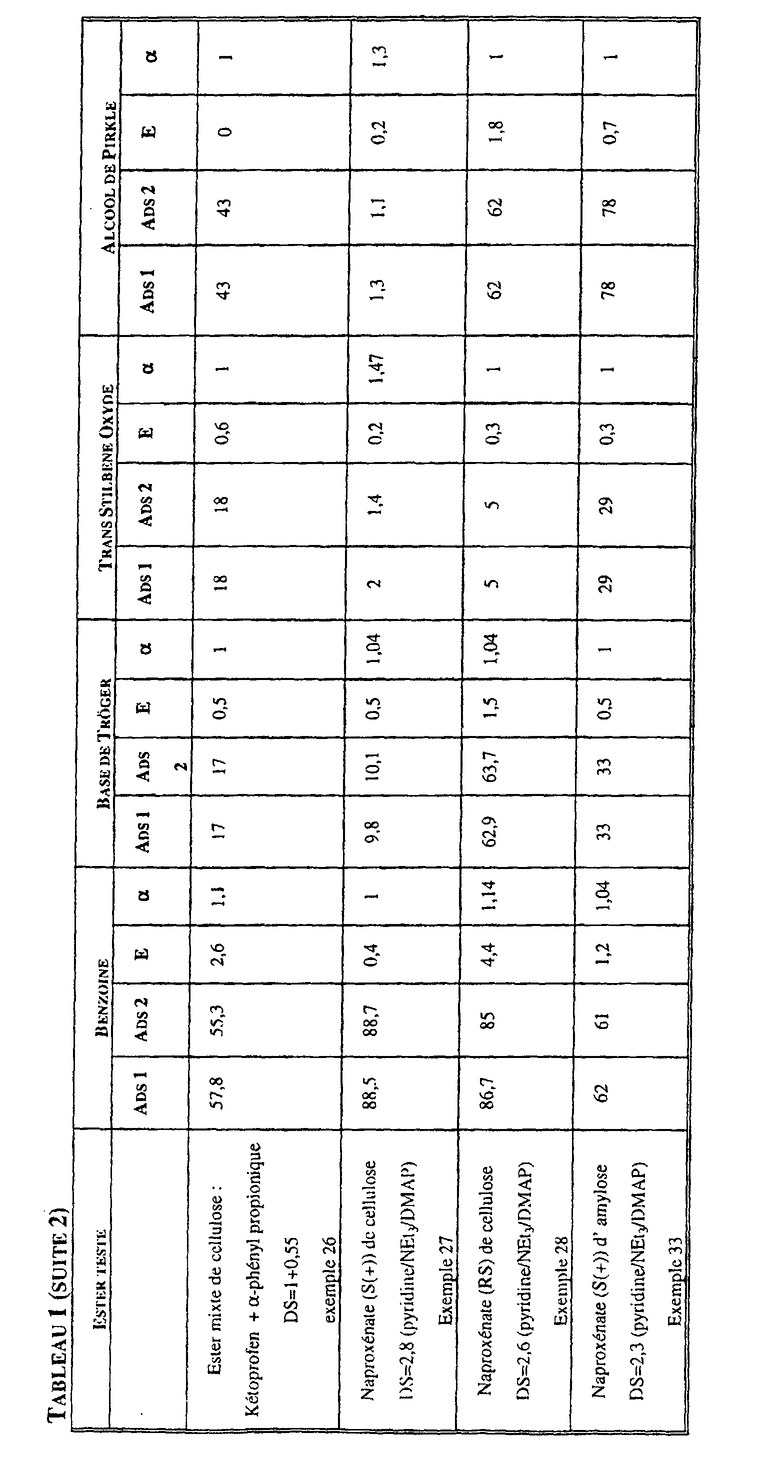

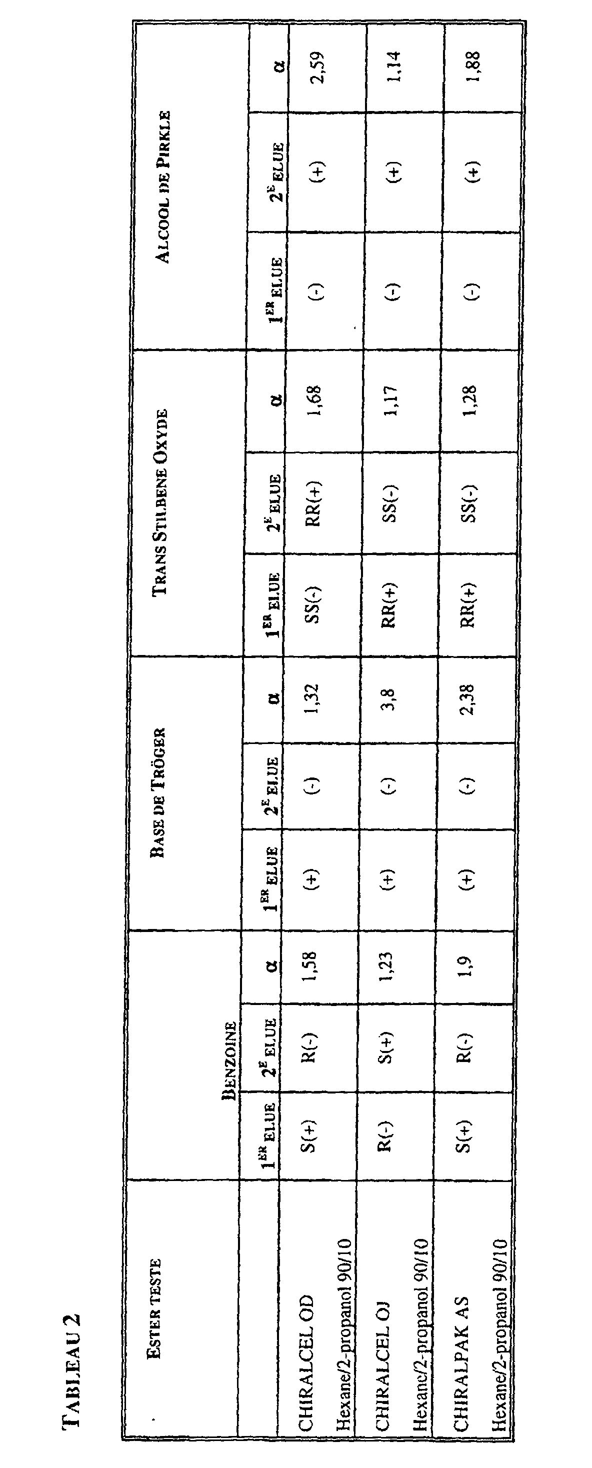

## Revendications

1. Ester de composé polyhydroxylé et éventuellement polyaminé, de préférence de polysaccharide, **caractérisé en ce qu'**il est chiral et **en ce qu'**il est obtenu par réaction en milieu basique d'au moins un composé polyhydroxylé et d'au moins un précurseur-acide ou dérivé de formule (I): 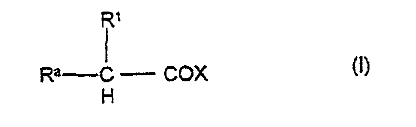 dans laquelle :
• X correspond à un hydroxyle ou à un halogène, de préférence le chlore,
• R^{a} représente un aryle, un radical aralkyle, aralcényle, alkylaryle, alcénylaryle,
• et R¹ représente l'hydrogène , un alkyle linéaire ou ramifié et/ou cyclique en C₁-C₂₀, avantageusement en C₁-C₆, et un radical alcényle, un aryle aralkyle, aralcényle, alkylaryle ou alcénylaryle, et **en ce que**
* il représente au moins un centre stéréogénique introduit par le précurseur-acide ou dérivé (I), et
* les motifs acyle fixés sur ledit composé polyhydroxylé, de préférence le polysaccharide ne sont pas tous de chiralité identique.

2. Ester suivant la revendication 1, **caractérisé en ce que** lesdits motifs acyle fixés sur ledit composé polyhydroxylé sont majoritairement de configuration R.

3. Ester suivant la revendication 1, **caractérisé en ce que** lesdits motifs acyle fixés sur ledit composé polyhydroxylé sont majoritairement de configuration S.

4. Ester suivant la revendication 3, **caractérisé en ce que** la proportion desdits motifs acyle de configuration R est comprise entre 50% et 70%.

5. Ester suivant la revendication 4, **caractérisée en ce que** la proportion desdits motifs acyle de configuration S est comprise entre 50% et 60%.

6. Ester selon l'une quelconque des revendications 1 à 5, **caractérisé en ce que** le degré de substitution (DS) de chaque unité monomérique dudit composé polyhydroxylé est compris entre 0,5 et 3.

7. Ester selon l'une quelconque des revendication 1 à 6, **caractérisé en ce que**, dans la formule (I) du précurseur-acide ou dérivé :
• R^{a} correspond à un substituant comprenant au moins un phényle, au moins un naphtyle, et/ou au moins un thiophényle,
• et R¹ correspond à un méthyle, un éthyle ou un propyle.

8. Ester selon l'une quelconque des revendications 1 à 7, **caractérisé en ce que**, dans la formule (I) du précurseur-acide ou dérivé, X correspond à Cl.

9. Ester selon l'une quelconque des revendications 1 à 8, **caractérisés en ce que** le précurseur (**I**) est obtenu à partir d'un acide originel (**II**) de formule : 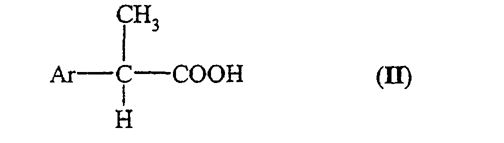 dans laquelle Ar représente un radical aromatique, un acide préféré étant sélectionné dans le groupe comprenant : acide α-phénylpropionique, acide α-(p-chlorophényl)propionique, acide 2-[3-benzoylphényl]propionique (kétoprofen), acide α-(3,5-diméthylphényl)propionique, acide α-(2-thiophényl)-propionique), acide α-(p-méthylphényl)proprionique, un acide anti inflammatoire non stéoridien de préférence l'acide α-méthyl-4-[isobutyl]phénylacétique (ibuprofen), acide 6-méthoxy-α-méthyl-2-naphtalèneacétique (naproxen).

10. Ester selon l'une quelconque des revendications 1 à 9, **caractérisés en ce que** les polysaccharides qui les constituent sont sélectionnés parmi les celluloses et leurs dérivés, et/ou parmi les amidons et leurs dérivés de préférence les amyloses et amulopectines.

11. Ester selon l'une quelconque des revendications 1 à 10, **caractérisé en ce qu'**il est obtenu en faisant réagir au moins un autre précurseur-acide ou dérivé (**I'**) différent du (ou des) précurseurs-acide(s) ou du (ou des) dérivé(s) (**I**), avec le ou les composé(s) polyhydroxylé(s) ;
cet autre réactif (**I'**) étant choisi parmi les composés chiraux de formule (I) ou parmi des composés chiraux ou non dans la liste suivante non limitative : acides acétique, propionique, benzoïque et carbamique.

12. Ester selon l'une quelconque des revendications 1 à 11 **caractérisé en ce qu'**il est choisi parmi les esters suivants comportant majoritairement desdits motifs acyle de configuration R, de préférence comportant entre 50 et 70 % de motifs de configuration R :
- α-phényl-propionate de cellulose
- α-chlorophényl-propionate d'amylose
- α-chlorophényl-propionate de cellulose
- α(3,5-diméthylphényl) propionate de cellulose
- α(paraméthyl-phényl) propionate de cellulose
- α-(2-thiophényl) propionate de cellulose
- un ester mixte de cellulose d'acide α phényl propionique et de kétoprofene
- ibuprofénate de cellulose
- le naproxénate de cellulose
- naproxénate d'amylose
- naproxénate de betacyclodextrine

13. Ester selon l'une quelconque des revendications 1 à 11 **caractérisé en ce qu'**il est choisi parmi les esters suivants comportant majoritairement desdits motifs acyle de configuration S, de préférence comportant entre 50 et 60 % de motifs de configuration 5 :
- α-phényl propionate de cellulose
- α-chlorophényl-propionate d'amylose
- α-chlorophényl-propionate de cellulose
- α(3,5-diméthylphényl) propionate de cellulose
- α(paraméthyl-phényl) propionate de cellulose
- α-(2-thiophényl) propionate de cellulose
- un ester mixte de cellulose d'acide α phényl propionique et de kétoprofene
- kétoprofénate de cellulose
- kétoprofénate d'amylose
- ibuprofénate de cellulose
- naproxénate de cellulose
- naproxénate d'amylose
- naproxénate de betacyclodextrine

14. Procédé de préparation d'un ester selon l'une quelconque des revendications 1 à 13, **caractérisé en ce qu'**il consiste, essentiellement, à faire réagir au moins un composé polyhydroxylé avec au moins un acide ou dérivé de formule (I), de préférence en milieu basique.

15. Procédé de préparation d'un ester selon la revendication 14, **caractérisé, en ce qu'**il consiste, essentiellement, à faire réagir la cellullose avec au moins un acide ou dérivé de formule (I), dans une base choisie parmi la pyridine seule, le mélange pyridine/DMAP, ou la 4-picoline seule, et **en ce qu'**il permet d'obtenir des esters de cellulose comportant desdits motifs acyle majoritairement de configuration R.

16. Procédé de préparation des esters selon la revendication 14, caractérisé d'une part, en ce qu'il consiste, essentiellement, à faire réagir la cellullose avec au moins un acide ou dérivé de formule (I), dans un solvant choisi parmi le mélange pyridine/triéthylamine/DMAP, la 2-picoline seule, et en ce qu'il permet d'obtenir des esters de cellulose comportant desdits motif acyle majoritairement de configuration S.

17. Procédé selon l'une quelconque des revendications 14 à 16, **caractérisé en ce que** l'on met en oeuvre au moins un chlorure d'acide à titre de réactif (**I**) et **en ce que** l'on récupère les esters formés en procédant à au moins une séquence précipitation/dissolution.

18. Procédé d'enrichissement chiral de racémates, **caractérisé en ce qu'**il comprend les étapes qui consistent essentiellement :
1. à utiliser des acides ou dérivés sous forme racémique ou énantiomériquement pure, comme produits de départ pour préparer les esters selon l'une quelconque des revendications 1 à 13 ou les ester obtenus par le procédé selon l'une quelconque des revendications 14 à 17,
2. et à hydrolyser les esters ainsi formés, de préférence à l'aide d'une base et de préférence encore à l'aide d'une base hydroxilée, de préférence encore l'hydroxyle de lithium

19. Procédé selon la revendication 18, **caractérisé en ce que** l'hydrolyse de l'étape **-2-** que l'on met en oeuvre est une hydrolyse totale ou partielle des fonctions esters, de préférence, de préférence à l'aide d'une base forte, plus préférentiellement encore à l'aide d'une base forte sélectionnée parmi les hydroxydes de métaux alcalins, (LiOH étant tout spécialement sélectionné), de façon à libérer majoritairement l'un des deux énantiomères du précurseur-acide ou dérivé (**II**) de départ.

20. Procédé selon la revendication 18 ou 19, **caractérisé en ce que** l'on effectue, avant l'hydrolyse de l'étape **-2-,** une étape **-1bis-** de déracémisation des susdits esters chiraux, de préférence au moyen d'une ou plusieurs bases azotées sélectionnée dans la liste suivante : la triéthylamine, la pyridine, les picolines, la DMAP, le DABCO, la quinoléine.

21. Moyen d'enrichissement chiral pour la mise en oeuvre du procédé selon les revendications 18 à 20, **caractérisé en ce qu'**il comprend un ester selon l'une quelconque des revendications 1 à 13 et/ou un ester obtenu par le procédé selon l'une quelconque des revendications 14 à 17.

22. Support énantiosélectif de chromatographie chirale, **caractérisé en ce qu'**il comprend un ester selon l'une quelconque des revendications 1 à 13 et/ou un ester obtenu par le procédé selon l'une quelconque des revendications 14 à 17.

23. Médicament **caractérisé en ce qu'**il comprend un ester selon l'une quelconque des revendications 1 à 13 et/ou un ester obtenu par le procédé selon l'une quelconque des revendications 14 à 17, le ou les précurseur(s)-acide(s) des esters constituant le ou les principes actifs et **en ce qu'**il est apte à libérer, de manière prolongée et contrôlée, par hydrolyse, le ou les principes actifs.

## Claims

1. Ester of a polyhydroxylated and optionally polyaminated compound, preferably of polysaccharide, **characterized in that** it is chiral and **in that** it is obtained by reacting, in a basic medium, at least one polyhydroxylated compound and at least one acid precursor or derivative of formula (**I**): 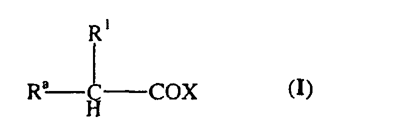 in which
• X corresponds to a hydroxyl or to a halogen, preferably chlorine,
• R^{a} represents an aryl or an aralkyl, aralkenyl, alkylaryl or alkenylaryl radical, and
• R¹ represents hydrogen, a linear or branched and/or cyclic C₁-C₂₀, advantageously C₁-C₆, alkyl, and an alkenyl, aryl, aralkyl, aralkenyl, alkylaryl or alkenylaryl radical, and **in that**
* it represents at least one stereogenic center introduced by the acid precursor or derivative (**I**), and
* the acyl units attached to said polyhydroxylated compound, preferably the polysaccharide, are not all of identical chirality.

2. Ester according to Claim 1, **characterized in that** said acyl units attached to said polyhydroxylated compound are mainly of R configuration.

3. Ester according to Claim 1, **characterized in that** said acyl units attached to said polyhydroxylated compound are mainly of S configuration.

4. Ester according to Claim 3, **characterized in that** the proportion of said acyl units of R configuration is between 50% and 70%.

5. Ester according to Claim 4, **characterized in that** the proportion of said acyl units of S configuration is between 50% and 60%.

6. Ester according to any one of Claims 1 to 5, **characterized in that** the degree of substitution (DS) of each monomer unit of said polyhydroxylated compound is between 0.5 and 3.

7. Ester according to any one of Claims 1 to 6, **characterized in that**, in formula (**I**) of the acid precursor or derivative:
• R^{a} corresponds to a substituent comprising at least one phenyl, at least one naphthyl and/or at least one thiophenyl, and
• R¹ corresponds to a methyl, an ethyl or a propyl.

8. Ester according to any one of Claims 1 to 7, **characterized in that**, in formula (**I**) of the acid precursor or derivative, X corresponds to Cl.

9. Ester according to any one of Claims 1 to 8, **characterized in that** the precursor (**I**) is obtained from an original acid (**II**) of formula: 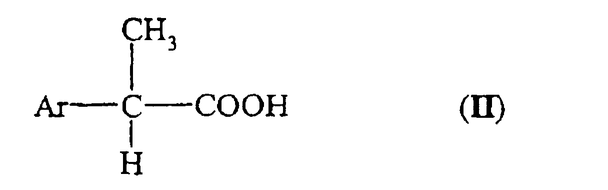 in which Ar represents an aromatic radical, a preferred acid being selected from the group comprising:
α-phenylpropionic acid, α-(p-chlorophenyl)propionic acid, 2-[3-benzoylphenyl]propionic acid (ketoprofen), α-(3,5-dimethylphenyl)propionic acid, α-(2-thiophenyl)-propionic acid, α-(p-methylphenyl)propionic acid, a nonsteroidal anti-inflammatory acid, preferably α-methyl-4-[isobutyl]phenylacetic acid (ibuprofen), 6-methoxy-α-methyl-2-naphthaleneacetic acid (naproxen).

10. Ester according to any one of Claims 1 to 9, **characterized in that** the polysaccharides of which they are constituted are selected from celluloses and derivatives thereof, and/or from starches and derivatives thereof, preferably amyloses and amylopectins.

11. Ester according to any one of Claims 1 to 10, **characterized in that** it is obtained by reacting at least one other acid precursor or derivative (**I'**) which is different from the acid precursor(s) or from the derivative(s) (**I**), with the polyhydroxylated compound(s) ;
this other reagent (**I'**) being chosen from the chiral compounds of formula (**I**) or from chiral or achiral compounds in the following nonlimiting list: acetic acid, propionic acid, benzoic acid and carbamic acid.

12. Ester according to any one of Claims 1 to 11, **characterized in that** it is chosen from the following esters mainly comprising said acyl units of R configuration, preferably comprising between 50% and 70% of units of R configuration:
- cellulose α-phenylpropionate
- amylose α-chlorophenylpropionate
- cellulose α-chlorophenylpropionate
- cellulose α-(3,5-dimethylphenyl)propionate
- cellulose α-(para-methylphenyl)propionate
- cellulose α-(2-thiophenyl)propionate
- a mixed cellulose ester of α-phenylpropionic acid and of ketoprofen
- cellulose ibuprofenate
- cellulose naproxenate
- amylose naproxenate
- beta-cyclodextrin naproxenate.

13. Ester according to any one of Claims 1 to 11, **characterized in that** it chosen from the following esters mainly comprising said acyl units of S configuration, preferably comprising between 50% and 60% of units of S configuration:
- cellulose α-phenylpropionate
- amylose α-chlorophenylpropionate
- cellulose α-chlorophenylpropionate
- cellulose α-(3,5-dimethylphenyl)propionate
- cellulose α- (para-methylphenyl)propionate
- cellulose α-(2-thiophenyl)propionate
- a mixed cellulose ester of α-phenylpropionic acid and of ketoprofen
- cellulose ketoprofenate
- amylose ketoprofenate
- cellulose ibuprofenate
- cellulose naproxenate
- amylose naproxenate
- beta-cyclodextrin naproxenate.

14. Process for preparing an ester according to any one of Claims 1 to 13, **characterized in that** it consists essentially in reacting at least one polyhydroxylated compound with at least one acid or derivative of formula (I), preferably in basic medium.

15. Process for preparing an ester according to Claim 14, **characterized in that** it consists essentially in reacting cellulose with at least one acid or derivative of formula (I), in a base chosen from pyridine alone, a pyridine/DMAP mixture, or 4-picoline alone, and **in that** it gives cellulose esters comprising said acyl units mainly of R configuration.

16. Process for preparing the esters according to Claim 14, **characterized in that**, firstly, it consists essentially in reacting cellulose with at least one acid or derivative of formula (I), in a solvent chosen from a pyridine/triethylamine/DMAP mixture and 2-picoline alone, and **in that** it gives cellulose esters comprising said acyl units mainly of S configuration.

17. Process according to any one of Claims 14 to 16, **characterized in that** at least one acid chloride is used as reagent (I) and **in that** the esters formed are recovered by carrying out at least one precipitation/dissolution sequence.

18. Process for the chiral enrichment of racemates, **characterized in that** it comprises the steps which consist essentially:
1. in using acids or derivatives in racemic or enantiomerically pure form, as starting materials to prepare the esters according to any one of Claims 1 to 13 or the esters obtained by the process according to any one of Claims 14 to 17, and
2. in hydrolyzing the esters thus formed, preferably using a base and more preferably using a hydroxylated base, more preferably lithium hydroxide.

19. Process according to Claim 18, **characterized in that** the hydrolysis in step -2- which is carried out is a total or partial hydrolysis of the ester functions, preferably, preferably using a strong base, even more preferably using a strong base selected from alkali metal hydroxides (LiOH being most especially selected), so as to predominantly release one of the two enantiomers of the starting acid precursor or derivative (II).

20. Process according to Claim 18 or 19, **characterized in that**, before the hydrolysis in step **-2-**, a step **-1a-** of deracemization of abovesaid chiral esters is carried out, preferably using one or more nitrogen bases selected from the following list: triethylamine, pyridine, picolines, DMAP, DABCO, quinoline.

21. Means of chiral enrichment for carrying out the process according to Claims 18 to 20, **characterized in that** it comprises an ester according to any one of Claims 1 to 13 and/or an ester obtained by the process according to any one of Claims 14 to 17.

22. Enantioselective chiral chromatography support, **characterized in that** it comprises an ester according to any one of Claims 1 to 13 and/or an ester obtained by the process according to any one of Claims 14 to 17.

23. Medicinal product, **characterized in that** it comprises an ester according to any one of Claims 1 to 13 and/or an ester obtained by the process according to any one of Claims 14 to 17, the acid precursor(s) of the esters constituting the active principle(s), and **in that** it is capable of releasing the active principle(s) in a sustained and controlled manner, by hydrolysis.

## Patentansprüche

1. Ester einer polyhydroxylierten und gegebenenfalls polyaminierten Verbindung, vorzugsweise eines Polysaccharids, **dadurch gekennzeichnet, daß** er chiral ist und daß er durch Reaktion von mindestens einer polyhydroxylierten Verbindung und mindestens einem Säure-Vorläufer oder Derivat der Formel (I) 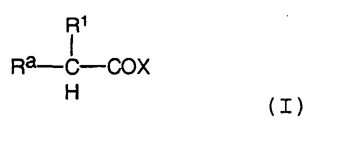 in einem basischen Medium erhalten wird, in der
- X einem Hydroxyl oder einem Halogen, vorzugsweise Chlor, entspricht,
- R^{a} ein Aryl, einen der Reste Aralkyl, Aralkenyl, Alkylaryl, Alkenylaryl darstellt und
- R¹ Wasserstoff, ein verzweigtes oder unverzweigtes und/oder cyclisches Alkyl mit 1 bis 20 Kohlenstoffatomen, vorzugsweise 1 bis 6 Kohlenstoffatomen, und einen der Reste Alkenyl, Arylaralkyl, Aralkenyl, Alkylaryl oder Alkenylaryl darstellt, und daß
* er mindestens ein sterogenes Zentrum aufweist, das durch den Säure-Vorläufer oder das Derivat (I) eingeführt wird, und
* die an die polyhydroxylierte Verbindung, vorzugsweise an das Polysaccharid, fixierten Acyl-Einheiten nicht alle von der gleichen Chiralität sind.

2. Ester nach Anspruch 1, **dadurch gekennzeichnet, daß** die an der polyhydroxylierten Verbindung fixierten Acyl-Einheiten mehrheitlich von R-Konfiguration sind.

3. Ester nach Anspruch 1, **dadurch gekennzeichnet, daß** die an der polyhydroxylierten Verbindung fixierten Acyl-Einheiten mehrheitlich von S-Konfiguration sind.

4. Ester nach Anspruch 3, **dadurch gekennzeichnet, daß** der Anteil der Acyl-Einheiten der R-Konfiguration zwischen 50% und 70% beträgt.

5. Ester nach Anspruch 4, **dadurch gekennzeichnet, daß** der Anteil dieser Acyl-Einheiten der S-Konfiguration zwischen 50% und 60% beträgt.

6. Ester nach einem der Ansprüche 1 bis 5, **dadurch gekennzeichnet, daß** der Grad der Substitution (DS) jeder monomeren Einheit der polyhydroxylierten Verbindung zwischen 0,5 und 3 beträgt.

7. Ester nach einem der Ansprüche 1 bis 6, **dadurch gekennzeichnet, daß** in der Formel (I) des Säure-Vorläufers oder Derivats
- R^{a} einem Substituenten entspricht, der mindestens ein Phenyl, mindestens ein Naphtyl und/oder mindestens ein Thiophenyl umfaßt,
- und R¹ Methyl, Ethyl oder Propyl entspricht.

8. Ester nach einem der Ansprüche 1 bis 7, **dadurch gekennzeichnet, daß** in der Formel (I) des Säure-Vorläufers oder Derivats das X für Cl steht.

9. Ester nach einem der Ansprüche 1 bis 8, **dadurch gekennzeichnet, daß** der Vorläufer (I) aus einer Ausgangssäure (II) der Formel 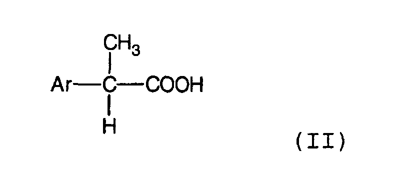 erhalten wird, in der Ar einen aromatischen Rest darstellt, wobei eine bevorzugte Säure aus der Gruppe ausgewählt wird, die
α-Phenylpropionsäure, α-(p-Chlorphenyl)propionsäure, 2-[3-Benzoylphenyl]propionsäure (Ketoprofen), α-(3,5-Dimethylphenyl)propionsäure, α-(2-Thiophenyl)propionsäure, α-(p-Methylphenyl)propionsäure, eine nichtsteoridale entzündungshemmende Säure, vorzugsweise α-Methyl-4-[isobutyl]phenylessigsäure (Ibuprofen), 6-Methoxy-α-methyl-2-naphtalenessigsäure (Naproxen) umfaßt.

10. Ester nach einem der Ansprüche 1 bis 9, **dadurch gekennzeichnet, daß** die sie bildenden Polysaccharide aus den Cellulosen und ihren Derivaten und/oder aus den Stärken und ihren Derivaten, vorzugsweise Amylosen und Amulopectinen, ausgewählt sind.

11. Ester nach einem der Ansprüche 1 bis 10, **dadurch gekennzeichnet, daß** er erhalten wird, indem man mindestens einen anderen Säure-Vorläufer oder ein anderes Derivat (I'), der bzw. das von dem oder den Säure-Vorläufer(n) oder Derivat(en) (I) verschieden ist, mit der oder den polyhydroxylierten Verbindung(en) reagieren läßt;
wobei dieses andere Reagenz (I') aus den chiralen Verbindungen der Formel (I) oder aus chiralen oder nichtchiralen Verbindungen in der folgenden nicht begrenzenden Liste ausgewählt wird: Essigsäure, Propionsäure, Benzoesäure und Carbaminsäure.

12. Ester nach einem der Ansprüche 1 bis 11, **dadurch gekennzeichnet, daß** er aus den folgenden Estern ausgewählt ist, die mehrheitlich diese Acyl-Einheiten der R-Konfiguration aufweisen und vorzugsweise zwischen 50 und 70% Einheiten der R-Konfiguration aufweisen:
- α-Phenylcellulosepropionat
- α-Chlorphenylamylosepropionat
- α-Chlorphenylcellulosepropionat
- α-(3,5-Dimethylphenyl)cellulosepropionat
- α-(Paramethylphenyl)cellulosepropionat
- α-(2-Thiophenyl)cellulosepropionat
- ein gemischter Ester von α-Phenylpropionsäurecellulose und Ketoprofen
- Celluloseibuprofenat
- Cellulosenaproxenat
- Amylosenaproxenat
- Betacyclodextrinnaproxenat.

13. Ester nach einem der Ansprüche 1 bis 11, **dadurch gekennzeichnet, daß** er aus den folgenden Estern ausgewählt ist, die mehrheitlich diese Acyl-Einheiten der S-Konfiguration aufweisen und vorzugsweise zwischen 50 und 60% Einheiten der S-Konfiguration aufweisen:
- α-Phenylcellulosepropionat
- α-Chlorphenylamylosepropionat
- α-Chlorphenylcellulosepropionat
- α-(3,5-Dimethylphenyl)cellulosepropionat
- α-(Paramethylphenyl)cellulosepropionat
- α-(2-Thiophenyl)cellulosepropionat
- ein gemischter Ester von α-Phenylpropionsäurecellulose und Ketoprofen
- Celluloseketoprofenat
- Amyloseketoprofenat
- Celluloseibuprofenat
- Cellulosenaproxenat
- Amylosenaproxenat
- Betacyclodextrinnaproxenat.

14. Verfahren zur Herstellung eines Esters nach einem der Ansprüche 1 bis 13, **dadurch gekennzeichnet, daß** es im wesentlichen darin besteht, daß man mindestens eine polyhydroxylierte Verbindung mit mindestens einer Säure oder einem Derivat der Formel (I) vorzugsweise in einem basischen Medium reagieren läßt.

15. Verfahren zur Herstellung eines Esters nach Anspruch 14, **dadurch gekennzeichnet, daß** es im wesentlichen darin besteht, daß man Cellulose mit mindestens einer Säure oder einem Derivat der Formel (I) in einer Base reagieren läßt, die aus Pyridin allein, einer Mischung Pyridin/DMAP oder 4-Picolin allein ausgewählt ist, und daß es die Herstellung von Celluloseestern gestattet, die diese Acyl-Einheiten mehrheitlich mit R-Konfiguration aufweisen.

16. Verfahren zur Herstellung von Estern nach Anspruch 14, **dadurch gekennzeichnet, daß** es im wesentlichen darin besteht, daß man Cellulose mit mindestens einer Säure oder einem Derivat der Formel (I) in einem Lösungsmittel reagieren läßt, das aus einer Mischung Pyridin/Triethylamin/DMAP, 2-Picolin allein ausgewählt ist, und daß es die Herstellung von Celluloseestern gestattet, die diese Acyl-Einheiten mehrheitlich der S-Konfiguration aufweisen.

17. Verfahren nach einem der Ansprüche 14 bis 16, **dadurch gekennzeichnet, daß** man als Reagenz (I) mindestens ein Säurechlorid einsetzt und daß man die gebildeten Ester gewinnt, indem man mindestens eine Sequenz der Ausfällung und Auflösung durchführt.

18. Verfahren zur chiralen Anreicherung von Racematen, **dadurch gekennzeichnet, daß** es die Schritte umfaßt, die im wesentlichen darin bestehen, daß man
1) Säuren oder Derivate in racemischer oder enantiomer reiner Form als Ausgangsprodukte verwendet, um die Ester nach einem der Ansprüche 1 bis 13 oder die nach dem Verfahren nach einem der Ansprüche 14 bis 17 erhaltenen Ester herzustellen,
2) die auf diese Weise gebildeten Ester vorzugsweise mit Hilfe einer Base oder noch bevorzugter mit Hilfe einer hydroxylierten Base, vorzugsweise Lithiumhydroxyl, hydrolysiert.

19. Verfahren nach Anspruch 18, **dadurch gekennzeichnet, daß** die Hydrolyse des Schritts 2), die man vornimmt, eine vollständige oder partielle Hydrolyse der Esterfunktionen vorzugsweise mit Hilfe einer starken Base, noch bevorzugter mit Hilfe einer starken Base ist, die aus den Hydroxiden von Alkalimetallen ausgewählt wird (wobei ganz besonders LiOH ausgewählt wird), so daß mehrheitlich eines der beiden Enantiomere des Ausgangs-Säurevorläufers oder -Derivats (II) freigesetzt wird.

20. Verfahren nach Anspruch 18 oder 19, **dadurch gekennzeichnet, daß** man vor der Hydrolyse des Schritts 2) einen Schritt 1bis) der Deracemisierung der chiralen Ester vorzugsweise mit Hilfe einer oder mehrerer azotierter Basen ausführt, die aus der folgenden Liste ausgewählt sind: Triethylamin, Pyridin, Picoline, DMAP, DABCO, Chinolin.

21. Mittel zur chiralen Anreicherung für die Durchführung des Verfahrens nach den Ansprüchen 18 bis 20, **dadurch gekennzeichnet, daß** es einen Ester nach einem der Ansprüche 1 bis 13 und/oder einen nach dem Verfahren nach einem der Ansprüche 14 bis 17 erhaltenen Ester aufweist.

22. Enantioselektiver Träger für die chiralen Chromatographie, **dadurch gekennzeichnet, daß** er einen Ester nach einem der Ansprüche 1 bis 13 und/oder einen nach dem Verfahren nach einem der Ansprüche 14 bis 17 erhaltenen Ester aufweist.

23. Medikament, **dadurch gekennzeichnet, daß** es einen Ester nach einem der Ansprüche 1 bis 13 und/oder einen nach dem Verfahren nach einem der Ansprüche 14 bis 17 erhaltenen Ester aufweist, wobei der oder die Säure-Vorläufer der Ester den oder die Wirkstoffe bilden, und daß es für die gesteuerte Langzeitfreisetzung des oder der Wirkstoffe durch Hydrolyse ausgelegt ist.
